# EUROPEAN PATENT SPECIFICATION

(11) **EP 4 213 728 B1**
(45) Date of publication and mention of the grant of the patent: **01.04.2026**
(21) Application number: 21870054.0
(22) Date of filing: 14.09.2021
(51) Int. Cl.: H01Q 1/27, A61B 5/145, A61B 5/00, A61B 5/1468, A61B 5/1473

(54) **SYSTEM, APPARATUS, AND DEVICES FOR ANALYTE MONITORING**
SYSTEM, VORRICHTUNG UND VORRICHTUNGEN ZUR ANALYTÜBERWACHUNG
SYSTÈME, APPAREIL ET DISPOSITIFS DE SURVEILLANCE D'ANALYTE

(30) Priority: 15.09.2020 US 202063078714 P
(43) Date of publication of application: 26.07.2023
(60) Divisional application: 26160363.3
(73) Proprietor: Abbott Diabetes Care, Inc., Alameda, CA 94502 (US)
(72) Inventor: COLE, Jean-Pierre, Tracy, CA 95304 (US); MATIEVICH, William, Jr., Alameda, CA 94501 (US); SAN NICOLAS, Anthony, Joseph, Reno, NV 89502 (US); KUNICH, Theodore, Pleasanton, CA 94588 (US); BETTANI, Fernando, 20128 Milan (IT); STOICA, Constantin, 20834 Nova Milanese (MB) (IT); FORLANI, Christian, Fabio, 20155 Milan (IT)
(74) Representative: Mathys & Squire
(86) International application number: PCT/US2021/050217
(87) International publication number: WO 2022/060706

(56) References cited:
- WO-A1-2008/143943
- US-A1- 2002 140 607
- US-A1- 2011 213 225
- US-A1- 2012 323 098
- US-A1- 2016 344 101
- US-B2- 10 742 269

## Description

### PRIORITY

This application claims priority to and benefit of U.S. Provisional Patent Application No. 63/078,714, filed 15 September 2020.

### FIELD

The subject matter described herein relates generally to systems, devices, and methods for in vivo monitoring of an analyte level.

### BACKGROUND

The detection and/or monitoring of analyte levels, such as glucose, ketones, lactate, oxygen, hemoglobin AIC, or the like, can be vitally important to the health of an individual having diabetes. Patients suffering from diabetes mellitus can experience complications including loss of consciousness, cardiovascular disease, retinopathy, neuropathy, and nephropathy. Diabetics are generally required to monitor their glucose levels to ensure that they are being maintained within a clinically safe range, and can also use this information to determine if and/or when insulin is needed to reduce glucose levels in their bodies, or when additional glucose is needed to raise the level of glucose in their bodies.

Growing clinical data demonstrates a strong correlation between the frequency of glucose monitoring and glycemic control. Despite such correlation, however, many individuals diagnosed with a diabetic condition do not monitor their glucose levels as frequently as they should due to a combination of factors including convenience, testing discretion, pain associated with glucose testing, and cost.

To increase patient adherence to a plan of frequent glucose monitoring, in vivo analyte monitoring systems can be utilized, in which a sensor control device can be worn on the body of an individual who requires analyte monitoring. To increase comfort and convenience for the individual, the sensor control device can have a small form-factor, and can be assembled and applied by the individual with a sensor applicator. The application process includes inserting a sensor, such as a dermal sensor that senses a user's analyte level in a bodily fluid located in the dermal layer of the human body, using an applicator or insertion mechanism, such that the sensor comes into contact with a bodily fluid. The sensor control device can also be configured to transmit analyte data to a receiving device, from which the individual or her health care provider ("HCP") can review the data and make therapy decisions.

The transmission of analyte data from the sensor to the receiving device can be performed using wired or wireless transmission. Prior art systems, however, have placed an increased emphasis on wireless transmission performed using near field communication (NFC) and/or Bluetooth communication. Wireless transmission improves the usability of the analyte monitoring sensor, allowing for manual or automatic transmission of analyte levels to the receiving device monitored by the user. To ensure transmission, a reliable wireless transmission signal should be maintained between the sensor control device and the receiving device.

Thus, a need exists for a system, apparatus, and methods to ensure reliable wireless transmission of analyte levels from the sensor to the receiving device monitored by an individual or HCP.

US10742269B2 discloses embodiments of devices that improve radio frequency (RF) communication between an on body device and a second device. Some of these embodiments pertain to a secondary communication system that captures an RF signal transmitted in a first directional pattern and retransmits it in the second directional pattern. Other embodiments pertain to a secondary communication system that provides an additional antenna positioned in a different location with which a user can communicate.

US2016344101A1 discloses a remote control device includes a first loop antenna portion in the form of a printed metallic trace on a first side of a printed circuit board (PCB) and a second loop antenna portion in the form of a raised metallic structure on a second side of the PCB. The first and second loop antenna portions are connected together through the PCB to form a loop antenna.

### SUMMARY

The purpose and advantages of the disclosed subject matter will be set forth in and apparent from the description that follows, as well as will be learned by practice of the disclosed subject matter. Additional advantages of the disclosed subject matter will be realized and attained by the methods and systems particularly pointed out in the written description and claims hereof, as well as from the appended drawings.

To achieve these and other advantages and in accordance with the purpose of the disclosed subject matter, as embodied and broadly described, the disclosed subject matter is directed to an apparatus for use with an analyte sensor, the apparatus including a printed circuit board configured to monitor an analyte level. The apparatus also includes a battery connected to the printed circuit board and configured to power the printed circuit board. In addition, the apparatus includes a connector connected to the printed circuit board and configured to establish an electrical connection between an analyte sensor and the printed circuit board, and a processor connected to the printed circuit board and configured to process data associated with the monitored analyte level. Further, the apparatus includes a plurality of risers and an antenna for transmitting the monitored analyte level resting on the plurality of risers. The risers extend from a surface of the printed circuit board by a fixed distance.

The analyte level can include a glucose level. The antenna can be a Bluetooth low energy antenna. The plurality of risers can include four risers, with two of the four risers being configured to electrically connect the antenna to the printed circuit board. The one or more of the plurality of risers can include a folded portion of the antenna. The printed circuit board can include FR4 material. At least part of the plurality of risers can be pre-plated tin over nickel. The antenna includes a cross bar located between a first set of the plurality of risers and a second set of the plurality of risers. The cross bar can form a portion of an h-shape. In some non-limiting embodiments, the antenna can include two or more ends forming a y-shape. In certain non-limiting embodiments, the antenna can include a free end that extends from the surface of the printed circuit board by the fixed distance. In other non-limiting embodiments, a first set of the plurality of risers can be located proximate to the connector, while a second set of the plurality of risers can be located proximate to the battery. The second set or the first set of the plurality of risers can be configured to electrically connect the antenna to the printed circuit board. The risers can extend from a surface of the printed circuit board by a fixed distance that can be greater than 1.5 millimeters (mm).

In some non-limiting embodiments, the antenna can be curved around an outer circumference of the battery. The antenna can be configured as an inverted h-shape or a j-shape. The antenna, for example, can have at least one of an unfolded width of about 9.33 mm, an unfolded length of about 12.04 mm, and/or a mass of 0.024 grams. In other non-limiting embodiments, the apparatus can include a separate NFC antenna for transmitting the monitored analyte level. The NFC antenna can be embedded within and/or around a circumference of the printed circuit board. In certain non-limiting embodiments, the connecter can include at least one of silicone rubber or carbon impregnated polymer. In other non-limiting embodiments, the connector can include a connector with metal contacts.

In certain other non-limiting embodiments, a system can include an analyte sensor. A portion of the analyte sensor can be is configured to be positioned in contact with fluid under a skin layer to monitor an analyte level in the fluid. The system can also include a printed circuit board connected to the analyte sensor, and/or a battery connected to the printed circuit board and configured to power the printed circuit board. In addition, the system can include a connector connected to the printed circuit board and configured to establish an electrically connection between the analyte sensor and the printed circuit board, and/or a processor connected to the printed circuit board and configured to process data associated with the monitored analyte level. Further, the system can include an antenna for transmitting the monitored analyte level resting on a plurality of risers. The risers can extend from a surface of the printed circuit board by a predetermined distance. The system can include any of the features described above for the apparatus.

### BRIEF DESCRIPTION OF THE FIGURES

The details of the subject matter set forth herein, both as to its structure and operation, can be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes can be illustrated schematically rather than literally or precisely.
FIG. 1 is a conceptual diagram depicting an example analyte monitoring system that can incorporate one or more embodiments of the present disclosure.
FIGS. 2A and 2B are isometric and side views, respectively, of an example sensor control device according to certain non-limiting embodiments.
FIGS. 3A and 3B are isometric and exploded views, respectively, of the plug assembly of FIGS. 2A and 2B according to certain non-limiting embodiments.
FIGS. 4A and 4B are exploded and bottom isometric views, respectively, of the electronics housing of FIGS. 2A and 2B according to certain non-limiting embodiments.
FIGS. 5A and 5B are side and cross-sectional side views, respectively, of the sensor applicator of FIG. 1 with the cap coupled thereto according to certain non-limiting embodiments.
FIG. 6A is an enlarged cross-sectional side view of the sensor control device mounted within a cap according to certain non-limiting embodiments.
FIG. 6B is an enlarged cross-sectional side view of another embodiment of the sensor control device mounted within the sensor applicator according to certain non-limiting embodiments.
FIG. 7 is an isometric view of an example sensor control device according to certain non-limiting embodiments.
FIG. 8 is a side view of the sensor applicator of FIG. 1 according to certain non-limiting embodiments.
FIG. 9 is a cross-sectional side view of the sensor applicator according to certain non-limiting embodiments.
FIG. 10A and 10B are isometric and side views, respectively, of an example sensor control device according to certain non-limiting embodiments.
FIGS. 11A and 11B are isometric and exploded views, respectively, of the plug assembly according to certain non-limiting embodiments.
FIG. 11C is an exploded isometric bottom view of the plug and the preservation vial according to certain non-limiting embodiments.
FIGS. 12A and 12B are side and cross-sectional side views, respectively, of the sensor applicator of FIG. 1 with the cap according to certain embodiments.
FIG. 13A and 13B are side and cross-sectional side views of the sensor applicator according to certain non-limiting embodiments.
FIG. 14 is a perspective view of an example embodiment of the cap according to certain embodiments.
FIG. 15 is a cross-sectional side view of the sensor control device positioned within the cap according to certain embodiments.
FIGS. 16A and 16B are isometric and side views, respectively, of an example sensor control device according to certain embodiments.
FIGS. 17A and 17B are exploded perspective top and bottom views, respectively, of the sensor control device according to certain embodiments.
FIGS. 18A-18C are isometric, side, and bottom views, respectively, of an example sensor control device according to certain embodiments.
FIGS. 19A and 19B are isometric exploded top and bottom views, respectively, of the sensor control device according to certain embodiments.
FIGS. 20A and 20B illustrate fabrication of the sensor control device according to certain embodiments.
FIG. 21 is a side view of an example sensor, according to certain embodiments.
FIGS. 22A and 22B illustrate isometric and partially exploded isometric views of an example connector assembly, according to certain embodiments.
FIG. 22C illustrates an isometric bottom view of the connector of FIGS. 22A-22B.
FIGS. 22D and 22E illustrate isometric and partially exploded isometric views of another example connector assembly, according to certain embodiments.
FIG. 22F illustrates an isometric bottom view of the connector of FIGS. 22D-22E.
FIGS. 23A and 23B illustrate side and isometric views, respectively, of an example sensor control device, according to certain embodiments.
FIGS. 24A and 24B illustrate exploded, isometric top and bottom views, respectively, of the sensor control device according to certain embodiments.
FIG. 25A is a cross-sectional side view of the sensor control device illustrated in FIGS. 23A-23B and 24A-24B, according to certain embodiments.
FIG. 25B is an exploded isometric view of a portion of another embodiment of the sensor control device illustrated in FIGS. 23A-23B and 24A-24B.
FIG. 26A is an isometric bottom view of the mount illustrated in FIGS. 23A-23B and 24A-24B.
FIG. 26B is an isometric top view of the sensor cap illustrated in FIGS. 23A-23B and 24A-24B.
FIGS. 27A and 27B illustrate side and cross-sectional side views, respectively, of an example sensor applicator, according to certain embodiments.
FIGS. 28A and 28B are perspective and top views, respectively, of the cap post illustrated in FIG. 27B, according to certain embodiments.
FIG. 29 illustrate a cross-sectional side view of the sensor control device positioned within the applicator cap, according to one or more embodiments.
FIG. 30 illustrate a cross-sectional view of a sensor control device showing example interaction between the sensor and the sharp.
FIG. 31A and 31B illustrate a printed circuit board according to certain embodiments.
FIG. 32 illustrates a printed circuit board according to certain embodiments.
FIGS. 33A-33D illustrate an embodiment of an antenna according to certain embodiments.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

The publications discussed herein are provided solely for their disclosure prior to the filing date of the present application.

The present disclosure will now be described more fully hereinafter with reference to the accompanying drawings, which form a part hereof, and which show, by way of illustration, certain example embodiments. Subject matter can, however, be embodied in a variety of different forms and, therefore, covered or claimed subject matter is intended to be construed as not being limited to any example embodiments set forth herein; example embodiments are provided merely to be illustrative. Likewise, a reasonably broad scope for claimed or covered subject matter is intended. Among other things, for example, subject matter can be embodied as methods, devices, components, or systems. Accordingly, embodiments can, for example, take the form of hardware, software, firmware or any combination thereof (other than software per se). The following detailed description is, therefore, not intended to be taken in a limiting sense.

In the detailed description herein, references to "embodiment," "an embodiment," "one non-limiting embodiment," "in various embodiments," etc., indicate that the embodiment(s) described can include a particular feature, structure, or characteristic, but every embodiment might not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

In general, terminology can be understood at least in part from usage in context. For example, terms, such as "and", "or", or "and/or," as used herein can include a variety of meanings that can depend at least in part upon the context in which such terms are used. Typically, "or" if used to associate a list, such as A, B or C, is intended to mean A, B, and C, here used in the inclusive sense, as well as A, B or C, here used in the exclusive sense. In addition, the term "one or more" as used herein, depending at least in part upon context, can be used to describe any feature, structure, or characteristic in a singular sense or can be used to describe combinations of features, structures or characteristics in a plural sense. Similarly, terms, such as "a," "an," or "the," again, can be understood to convey a singular usage or to convey a plural usage, depending at least in part upon context. In addition, the term "based on" can be understood as not necessarily intended to convey an exclusive set of factors and can, instead, allow for existence of additional factors not necessarily expressly described, again, depending at least in part on context.

As used herein, the terms "comprises," "comprising," or any other variation thereof, are intended to cover a non-exclusive inclusion, such that a process, method, article, or apparatus that comprises a list of elements does not include only those elements but can include other elements not expressly listed or inherent to such process, method, article, or apparatus.

There are various types of in vivo analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or "ex vivo") and that typically include a meter device that has a port for receiving an analyte test strip carrying bodily fluid of the user, which can be analyzed to determine the user's blood sugar level.

In vivo monitoring systems can include a sensor that, while positioned in vivo, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers have also been utilized with or incorporated into in vivo and in vitro monitoring systems.

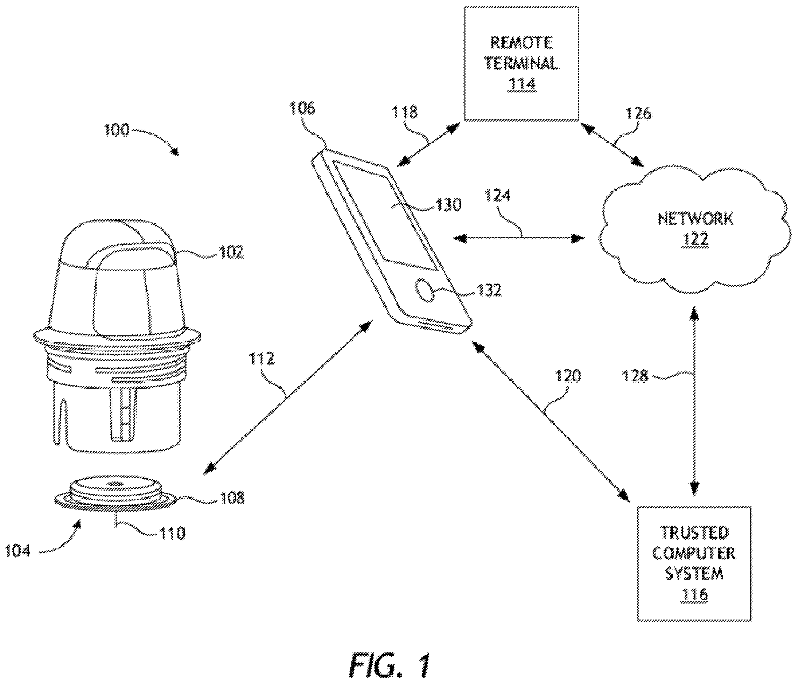

FIG. 1 is a conceptual diagram depicting an example analyte monitoring system 100 that can incorporate one or more embodiments of the present disclosure. A variety of analytes can be detected and quantified using the system 100 (hereafter "the system 100") including, but not limited to, acetyl choline, amylase, bilirubin, cholesterol, chorionic gonadotropin, creatine kinase (e.g., CK-MB), creatine, DNA, fructosamine, glucose, glutamine, growth hormones, hormones, ketones (e.g., ketone bodies), lactate, oxygen, peroxide, prostate-specific antigen, prothrombin, RNA, thyroid stimulating hormone, and troponin. The concentration of drugs, such as, but not limited to, antibiotics (e.g., gentamicin, vancomycin, and the like), digitoxin, digoxin, drugs of abuse, theophylline, and warfarin, can also be determined.

As illustrated, the system 100 includes a sensor applicator 102 (alternately referred to as an "inserter"), a sensor control device 104 (also referred to as an "in vivo analyte sensor control device"), and a reader device 106. The sensor applicator 102 is used to deliver the sensor control device 104 to a target monitoring location on a user's skin (e.g., the arm of the user). Once delivered, the sensor control device 104 is maintained in position on the skin with an adhesive patch 108 coupled to the bottom of the sensor control device 104. A portion of a sensor 110 extends from the sensor control device 104 and is positioned such that it can be transcutaneously positioned and otherwise retained under the surface of the user's skin during the monitoring period.

An introducer can be included to promote introduction of the sensor 110 into tissue. The introducer can comprise, for example, a needle often referred to as a "sharp." Alternatively, the introducer can comprise other types of devices, such as a sheath or a blade. The introducer can transiently reside in proximity to the sensor 110 prior to tissue insertion and then be withdrawn afterward. While present, the introducer can facilitate insertion of the sensor 110 into tissue by opening an access pathway for the sensor 110 to follow. For example, the introducer can penetrate the epidermis to provide an access pathway to the dermis to allow subcutaneous implantation of the sensor 110. After opening the access pathway, the introducer can be withdrawn (retracted) so that it does not represent a hazard while the sensor 110 remains in place.

In illustrative embodiments, the introducer can be solid or hollow, beveled or non-beveled, and/or circular or non-circular in cross-section. In more particular embodiments, suitable introducers can be comparable in cross-sectional diameter and/or tip design to an acupuncture needle, which can have a cross-sectional diameter of about 250 microns. It is to be recognized, however, that suitable introducers can have a larger or smaller cross-sectional diameter if needed for particular applications.

In some embodiments, a tip of the introducer (while present) can be angled over the terminus of the sensor 110, such that the introducer penetrates a tissue first and opens an access pathway for the sensor 110. In other illustrative embodiments, the sensor 110 can reside within a lumen or groove of the introducer, with the introducer similarly opening an access pathway for the sensor 110. In either case, the introducer is subsequently withdrawn after facilitating sensor 110 insertion. Moreover, the introducer (sharp) can be made of a variety of materials, such as various types of metals and plastics.

When the sensor control device 104 is properly assembled, the sensor 110 is placed in communication (e.g., electrical, mechanical, etc.) with one or more electrical components or sensor electronics included within the sensor control device 104. In some applications, for example, the sensor control device 104 can include a printed circuit board (PCB) having a data processor (e.g., an application specific integrated circuit or ASIC) mounted thereto, and the sensor 110 can be operatively coupled to the data processor which, in turn, can be coupled with an antenna and a power source.

The sensor control device 104 and the reader device 106 are configured to communicate with one another over a local communication path or link 112, which can be wired or wireless, uni- or bi-directional, and encrypted or non-encrypted. The reader device 106 can constitute an output medium for viewing analyte concentrations and alerts or notifications determined by the sensor 110 or a processor associated therewith, as well as allowing for one or more user inputs, according to some embodiments. The reader device 106 can be a multi-purpose smartphone or a dedicated electronic reader instrument. While only one reader device 106 is shown, multiple reader devices 106 can be present in certain instances.

The reader device 106 can also be in communication with a remote terminal 114 and/or a trusted computer system 116 via communication path(s)/link(s) 118 and/or 120, respectively, which also can be wired or wireless, uni- or bi-directional, and encrypted or non- encrypted. The reader device 106 can also or alternately be in communication with a network 122 (e.g., a mobile telephone network, the internet, or a cloud server) via communication path/link 124. The network 122 can be further communicatively coupled to remote terminal 114 via communication path/link 126 and/or the trusted computer system 116 via communication path/link 128.

Alternately, the sensor control device 104 can communicate directly with the remote terminal 114 and/or the trusted computer system 116 without an intervening reader device 106 being present. For example, the sensor 110 can communicate with the remote terminal 114 and/or the trusted computer system 116 through a direct communication link to the network 122, according to some embodiments, as described in U.S. Patent No. 10,136,816.

Any suitable electronic communication protocol can be used for each of the communication paths or links, such as NFC, radio frequency identification (RFID), BLUETOOTH^{®} or BLUETOOTH^{®} low energy protocols, Wireless Land Area Network, or the like. The remote terminal 114 and/or the trusted computer system 116 can be accessible, according to some embodiments, by individuals other than a primary user who have an interest in the user's analyte levels. The reader device 106 can include a display 130 and an optional input component 132. The display 130 can comprise a touch-screen interface, according to some embodiments.

In some embodiments, the sensor control device 104 can automatically forward data to the reader device 106. For example, analyte concentration data can be communicated automatically and periodically, such as at a certain frequency as data is obtained or after a certain time period has passed, with the data being stored in a memory until transmittal (e.g., every minute, five minutes, or other predetermined time period). In other embodiments, the sensor control device 104 can communicate with the reader device 106 in a non-automatic manner and not according to a set schedule. For example, data can be communicated from the sensor control device 104 using RFID technology when the sensor electronics are brought into communication range of the reader device 106. Until communicated to the reader device 106, data can remain stored in a memory of the sensor control device 104. Thus, a patient does not have to maintain close proximity to the reader device 106 at all times, and can instead upload data when convenient. In yet other embodiments, a combination of automatic and non-automatic data transfer can be implemented. For example, data transfer can continue on an automatic basis until the reader device 106 is no longer in communication range of the sensor control device 104.

The sensor control device 104 is often included with the sensor applicator 104 in what is known as a "two-piece" architecture that requires final assembly by a user before the sensor 110 can be properly delivered to the target monitoring location. More specifically, the sensor 110 and the associated electrical components included in the sensor control device 104 are provided to the user in multiple (two) packages, and the user must open the packaging and follow instructions to manually assemble the components before delivering the sensor 110 to the target monitoring location with the sensor applicator 102.

More recently, however, advanced designs of sensor control devices and sensor applicators have resulted in a one-piece architecture that allows the system to be shipped to the user in a single, sealed package that does not require any final user assembly steps. Rather, the user need only open one package and subsequently deliver the sensor control device to the target monitoring location. The one-piece system architecture can prove advantageous in eliminating component parts, various fabrication process steps, and user assembly steps. As a result, packaging and waste are reduced, and the potential for user error or contamination to the system is mitigated.

In the illustrated embodiment, the system 100 can comprise what is known as a "two-piece" architecture that requires final assembly by a user before the sensor 110 can be properly delivered to the target monitoring location. More specifically, the sensor 110 and the associated electrical components included in the sensor control device 104 are provided to the user in multiple (two) packages, where each can or cannot be sealed with a sterile barrier but are at least enclosed in packaging. The user must open the packaging and follow instructions to manually assemble the components and subsequently deliver the sensor 110 to the target monitoring location with the sensor applicator 102. In certain other embodiments, however, system 100 can include a "one-piece" architecture.

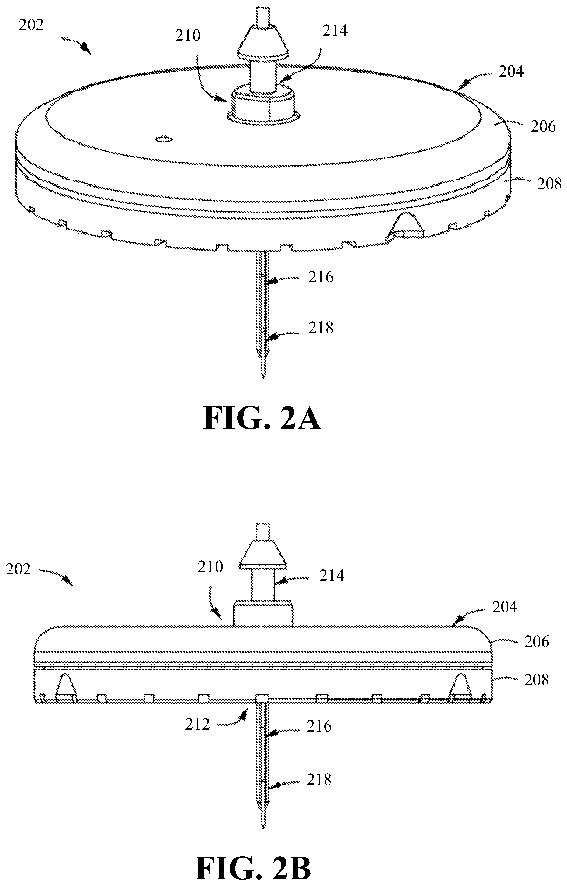

FIGS. 2A and 2B are isometric and side views, respectively, of an example sensor control device 202, according to one or more embodiments of the present disclosure. The sensor control device 202 (alternately referred to as a "puck") can be similar in some respects to the sensor control device 104 of FIG. 1 and therefore can be best understood with reference thereto. The sensor control device 202 can replace the sensor control device 104 of FIG. 1 and, therefore, can be used in conjunction with the sensor applicator 102 (FIG. 1), which delivers the sensor control device 202 to a target monitoring location on a user's skin.

The sensor control device 202, however, can be incorporated into a one-piece system architecture. Unlike the two-piece architecture system, for example, a user is not required to open multiple packages and finally assemble the sensor control device 202. Rather, upon receipt by the user, the sensor control device 202 is already fully assembled and properly positioned within the sensor applicator 102. To use the sensor control device 202, the user need only break one barrier, for example an applicator cap, before promptly delivering the sensor control device 202 to the target monitoring location.

As illustrated, the sensor control device 202 includes an electronics housing 204 that is generally disc-shaped and/or puck shaped with a circular cross-section. In other embodiments, however, the electronics housing 204 can exhibit other cross-sectional shapes, such as ovoid (e.g., pill-shaped), a squircle, or polygonal, without departing from the scope of the disclosure. The electronics housing 204 can be configured to house or otherwise contain various electrical components used to operate the sensor control device 202.

The electronics housing 204 can include a shell 206 and a mount 208 that is matable with the shell 206. The shell 206 can be secured to the mount 208 via a variety of ways, such as a snap fit engagement, an interference fit, sonic welding, or one or more mechanical fasteners (e.g., screws). In some cases, the shell 206 can be secured to the mount 208 such that a sealed interface therebetween is generated. In such embodiments, a gasket or other type of seal material can be positioned at or near the outer diameter (periphery) of the shell 206 and the mount 208, and securing the two components together can compress the gasket and thereby generate a sealed interface. In other embodiments, an adhesive can be applied to the outer diameter (periphery) of one or both of the shell 206 and the mount 208. The adhesive secures the shell 206 to the mount 208 and provides structural integrity, but can also seal the interface between the two components and thereby isolate the interior of the electronics housing 204 from outside contamination. If the sensor control device 202 is assembled in a controlled environment, there can be no need to terminally sterilize the internal electrical components. Rather, the adhesive coupling can provide a sufficient sterile barrier for the assembled electronics housing 204.

The sensor control device 202 can further include a plug assembly 210 that can be coupled to the electronics housing 204. For example, the plug assembly 210 can include a sensor module 212 (partially visible) interconnectable with a sharp module 214 (partially visible). The sensor module 212 can be configured to carry and otherwise include a sensor 216 (partially visible), and the sharp module 214 can be configured to carry and otherwise include a sharp 218 (partially visible) used to help deliver the sensor 216 transcutaneously under a user's skin during application of the sensor control device 202. As illustrated, corresponding portions of the sensor 216 and the sharp 218 extend from the electronics housing 204 and, more particularly, from the bottom of the mount 208. The exposed portion of the sensor 216 can be received within a hollow or recessed portion of the sharp 218. The remaining portion of the sensor 216 is positioned within the interior of the electronics housing 204.

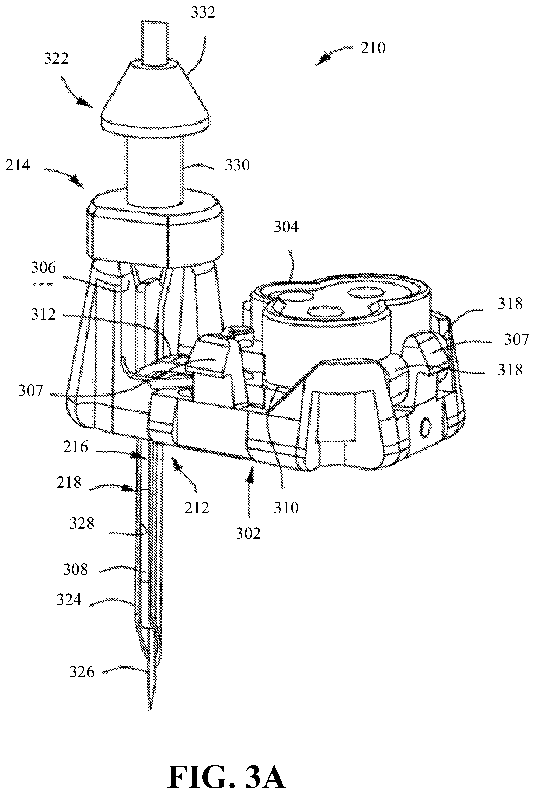

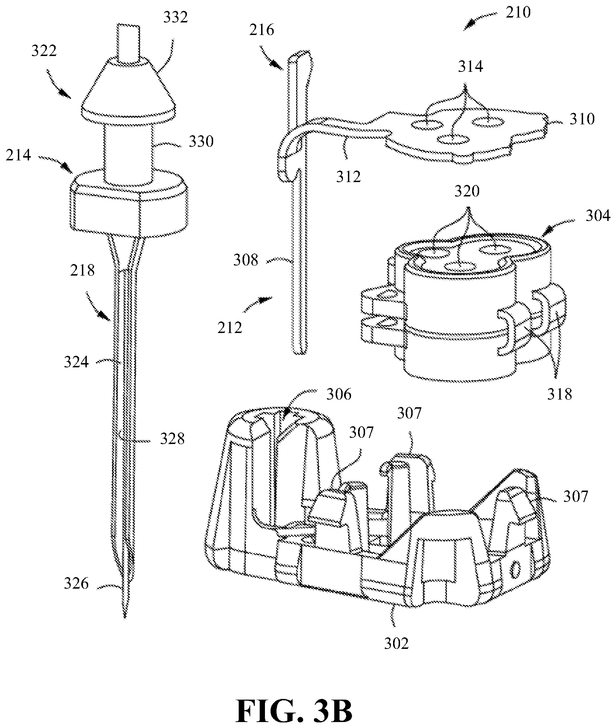

FIGS. 3A and 3B are isometric and exploded views, respectively, of the plug assembly 210, according to one or more embodiments. The sensor module 212 can include the sensor 216, a plug 302, and a connector 304. The plug 302 can be designed to receive and support both the sensor 216 and the connector 304. As illustrated, a channel 306 can be defined through the plug 302 to receive a portion of the sensor 216. Moreover, the plug 302 can provide one or more deflectable arms 307 configured to snap into corresponding features provided on the bottom of the electronics housing 204 (FIGS. 2A and 2B).

The sensor 216 includes a tail 308, a flag 310, and a neck 312 that interconnects the tail 308 and the flag 310. The tail 308 can be configured to extend at least partially through the channel 306 and extend distally from the plug 302. The tail 308 includes an enzyme or other chemistry or biologic and, in some embodiments, a membrane can cover the chemistry. In use, the tail 308 is transcutaneously received beneath a user's skin, and the chemistry included thereon helps facilitate analyte monitoring in the presence of bodily fluids.

The flag 310 can comprise a generally planar surface having one or more sensor contacts 314 (three shown in FIG. 3B) arranged thereon. The sensor contact(s) 314 can be configured to align with a corresponding number of compliant carbon impregnated polymer modules (not shown) encapsulated within the connector 304.

The connector 304 includes one or more hinges 318 that enables the connector 304 to move between open and closed states. The connector 304 is depicted in FIGS. 3A and 3B in the closed state, but can pivot to the open state to receive the flag 310 and the compliant carbon impregnated polymer module(s) therein. The compliant carbon impregnated polymer module(s) provide electrical contacts 320 (three shown) configured to provide conductive communication between the sensor 216 and corresponding circuitry contacts provided within the electronics housing 204 (FIGS. 2A and 2B). The connector 304 can be made of silicone rubber and can serve as a moisture barrier for the sensor 216 when assembled in a compressed state and after application to a user's skin.

The sharp module 214 includes the sharp 218 and a sharp hub 322 that carries the sharp 218. The sharp 218 includes an elongate shaft 324 and a sharp tip 326 at the distal end of the shaft 324. The shaft 324 can be configured to extend through the channel 306 and extend distally from the plug 302. Moreover, the shaft 324 can include a hollow or recessed portion 328 that at least partially circumscribes the tail 308 of the sensor 216. The sharp tip 326 can be configured to penetrate the skin while carrying the tail 308 to put the active chemistry present on the tail 308 into contact with bodily fluids.

The sharp hub 322 can include a hub small cylinder 330 and a hub snap pawl 332, each of which can be configured to help couple the plug assembly 210 (and the entire sensor control device 202) to the sensor applicator 102 (FIG. 1).

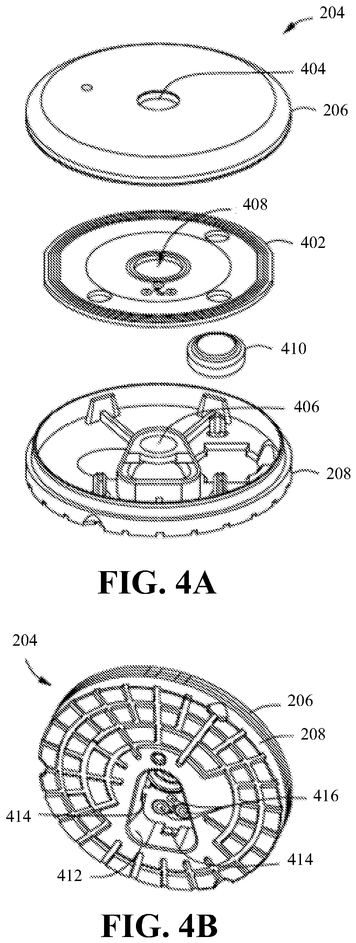

FIGS. 4A and 4B are exploded and bottom isometric views, respectively, of the electronics housing 204, according to one or more embodiments. The shell 206 and the mount 208 operate as opposing clamshell halves that enclose or otherwise substantially encapsulate the various electronic components of the sensor control device 202 (FIGS. 2A and 2B).

A printed circuit board (PCB) 402 can be positioned within the electronics housing 204. A plurality of electronic modules (not shown) can be mounted to the PCB 402 including, but not limited to, a data processing unit, resistors, transistors, capacitors, inductors, diodes, and switches. The data processing unit can comprise, for example, an application specific integrated circuit (ASIC) configured to implement one or more functions or routines associated with operation of the sensor control device 202. More specifically, the data processing unit can be configured to perform data processing functions, where such functions can include but are not limited to, filtering and encoding of data signals, each of which corresponds to a sampled analyte level of the user. The data processing unit can also include or otherwise communicate with an antenna for communicating with the reader device 106 (FIG. 1).

As illustrated, the shell 206, the mount 208, and the PCB 402 each define corresponding central apertures 404, 406, and 408, respectively. When the electronics housing 304 is assembled, the central apertures 404, 406, and 408 coaxially align to receive the plug assembly 210 (FIGS. 3A and 3B) therethrough. A battery 410 can also be housed within the electronics housing 204 and configured to power the sensor control device 202.

In FIG. 4B, a plug receptacle 412 can be defined in the bottom of the mount 208 and provide a location where the plug assembly 210 (FIGS. 3A and 3B) can be received and coupled to the electronics housing 204, and thereby fully assemble the sensor control device 202 (FIG. 2A and 2B). The profile of the plug 302 (FIGS. 3A and 3B) can match or be shaped in complementary fashion to the plug receptacle 412, and the plug receptacle 412 can provide one or more snap ledges 414 (two shown) configured to interface with and receive the deflectable arms 307 (FIGS. 3A and 3B) of the plug 302. The plug assembly 210 is coupled to the electronics housing 204 by advancing the plug 302 into the plug receptacle 412 and allowing the deflectable arms 307 to lock into the corresponding snap ledges 414. When the plug assembly 210 (FIGS. 3A and 3B) is properly coupled to the electronics housing 204, one or more circuitry contacts 416 (three shown) defined on the underside of the PCB 402 can make conductive communication with the electrical contacts 320 (FIGS. 3A and 3B) of the connector 304 (FIGS. 3A and 3B).

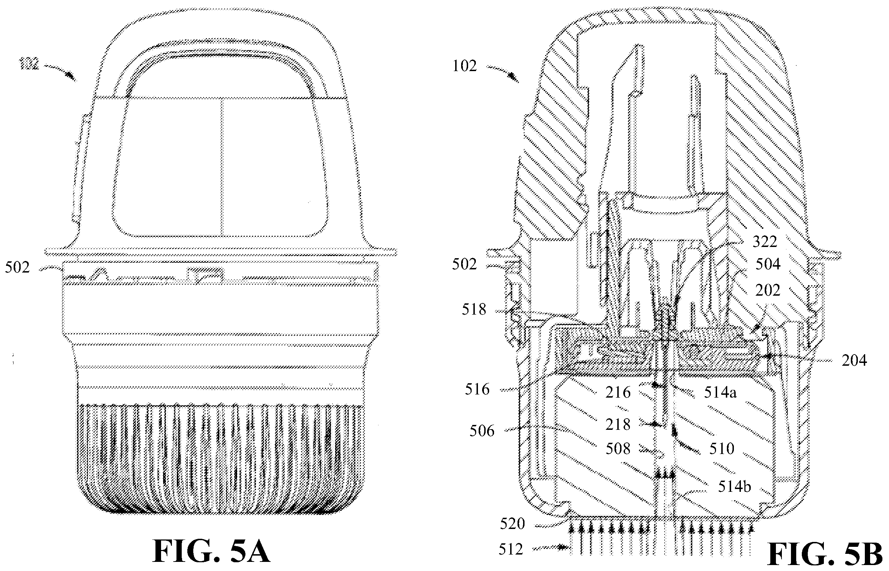

FIGS. 5A and 5B are side and cross-sectional side views, respectively, of the sensor applicator 102 with the applicator cap coupled thereto. More specifically, FIGS. 5A and 5B depict how the sensor applicator 102 might be shipped to and received by a user, according to at least one embodiment. In some embodiments, however, the sensor applicator 102 might further be sealed within a bag (not shown) and delivered to the user within the bag. The bag can be made of a variety of materials that help prevent the ingress of humidity into the sensor applicator 102, which might adversely affect the sensor 216. In at least one embodiment, for example, the sealed back might be made of foil. Any and all of the sensor applicators described or discussed herein can be sealed within and delivered to the user within the bag.

According to the present disclosure, and as seen in FIG. 5B, the sensor control device 202 is already assembled and installed within the sensor applicator 102 prior to being delivered to the user. The applicator cap can be threaded to the housing and include a tamper ring 502. Upon rotating (e.g., unscrewing) the applicator cap relative to the housing, the tamper ring 502 can shear and thereby free the applicator cap from the sensor applicator 102. Following which, the user can deliver the sensor control device 202 to the target monitoring location.

In some embodiments, as mentioned above, the applicator cap can be secured to the housing via a sealed engagement to protect the internal components of the sensor applicator 102. In at least one embodiment, for example, an O-ring or another type of sealing gasket can seal an interface between the housing and the applicator cap. The O-ring or sealing gasket can be a separate component part or alternatively molded onto one of the housing and the applicator cap.

The housing can be made of a variety of rigid materials. In some embodiments, for example, the housing can be made of a thermoplastic polymer, such as polyketone. In other embodiments, the housing can be made of cyclic olefin copolymer (COC), which can help prevent moisture ingress into the interior of the sensor applicator 102. As will be appreciated, any and all of the housings described or discussed herein can be made of polyketone or COC.

With specific reference to FIG. 5B, the sensor control device 202 can be loaded into the sensor applicator 102 by mating the sharp hub 322 with a sensor carrier 504 included within the sensor applicator 102. Once the sensor control device 202 is mated with the sensor carrier 504, the applicator cap can then be secured to the sensor applicator 102.

In the illustrated embodiment, a collimator 506 is positioned within the applicator cap and can generally help support the sensor control device 202 while contained within the sensor applicator 102. In some embodiments, the collimator 606 can form an integral part or extension of the applicator cap, such as being molded with or overmolded onto the applicator cap. In other embodiments, the collimator 506 can comprise a separate structure fitted within or attached to the applicator cap, without departing from the scope of the disclosure. In yet other embodiments, as discussed below, the collimator 506 can be omitted in the package received by the user, but otherwise used while sterilizing and preparing the sensor applicator 102 for delivery.

The collimator 506 can be designed to receive and help protect parts of the sensor control device 202 that need to be sterile, and isolate the sterile components of the sensor applicator 102 from microbial contamination from other locations within the sensor control device 202. To accomplish this, the collimator 506 can define or otherwise provide a sterilization zone 508 (alternately referred to as a "sterile barrier enclosure" or a "sterile sensor path") configured to receive the sensor 216 and the sharp 218 as extending from the bottom of the electronics housing 204. The sterilization zone 508 can generally comprise a hole or passageway extending at least partially through the body of the collimator 506. In the illustrated embodiment, the sterilization zone 508 extends through the entire body of the collimator 506, but can alternatively extend only partially therethrough, without departing from the scope of the disclosure.

When the sensor control device 202 is loaded into the sensor applicator 102 and the applicator cap with the collimator 506 is secured thereto, the sensor 216 and the sharp 218 can be positioned within a sealed region 510 at least partially defined by the sterilization zone 508. The sealed region 510 is configured to isolate the sensor 216 and the sharp 218 from external contamination and can include (encompass) select portions of the interior of the electronics housing 204. Certain embodiments can include a sterilization zone 508 of the collimator 506.

While positioned within the sensor applicator 102, the fully assembled sensor control device 202 in certain embodiments can be subjected to radiation sterilization 512. The radiation sterilization 512 can comprise, for example, e-beam irradiation, but other methods of sterilization can alternatively be used including, but not limited to, low energy X-ray irradiation. In some embodiments, the radiation sterilization 512 can be delivered either through continuous processing irradiation or through pulsed beam irradiation. In pulsed beam irradiation, the beam of radiation sterilization 512 is focused at a target location and the component part or device to be sterilized is moved to the target location at which point the radiation sterilization 512 is activated to provide a directed pulse of radiation. The radiation sterilization 512 is then turned off, and another component part or device to be sterilized is moved to the target location and the process is repeated.

The collimator 506 can be configured to focus the radiation (e.g., beams, waves, energy, etc.) from the radiation sterilization 512 toward the components that are required to be sterile, such as the sensor 216 and the sharp 218. More specifically, the hole or passageway of the sterilization zone 508 allows transmission of the radiation to impinge upon and sterilize the sensor 216 and the sharp 218, while the remaining portions of the collimator 506 prevent (impede) the propagating radiation from disrupting or damaging the electronic components within the electronics housing 204.

The sterilization zone 508 can exhibit any suitable cross-sectional shape necessary to properly focus the radiation on the sensor 216 and the sharp 218 for sterilization. In the illustrated embodiment, for example, the sterilization zone 508 is circular cylindrical, but could alternatively exhibit a polygonal cross-sectional shape, such as cubic or rectangular (e.g., including parallelogram), without departing from the scope of the disclosure.

In the illustrated embodiment, the sterilization zone 508 provides a first aperture 514a at a first end and a second aperture 514b at a second end opposite the first end. The first aperture 514a can be configured to receive the sensor 316 and the sharp 318 into the sterilization zone 508, and the second aperture 514b can allow the radiation (e.g., beams, waves, etc.) from the radiation sterilization 512 to enter the sterilization zone 508 and impinge upon the sensor 216 and the sharp 218. In the illustrated embodiment, the first and second apertures 514a,b exhibit identical diameters.

The body of the collimator 506 reduces or eliminates the radiation sterilization 512 from penetrating through the body material and thereby damaging the electronic components within the electronics housing 204. To accomplish this, in some embodiments, the collimator 506 can be made of a material that has a mass density greater than 0.9 grams per cubic centimeter (g/cc). One example material for the collimator 506 is polyethylene, but could alternatively comprise any material having a mass density similar to or greater than polyethylene. In some embodiments, for example, the material for the collimator 506 can comprise, but is not limited to, a metal (e.g., lead, stainless steel) or a high-density polymer.

In at least one embodiment, the design of the collimator 506 can be altered so that the collimator 506 can be made of a material that has a mass density less than 0.9 grams per cubic centimeter (g/cc) but still operate to reduce or eliminate the radiation sterilization 512 from impinging upon the electronic components within the electronics housing 204. To accomplish this, in some embodiments, the size (e.g., length) of the collimator 506 can be increased such that the propagating electrons from the radiation sterilization 512 are required to pass through a larger amount of material before potentially impinging upon sensitive electronics. The larger amount of material can help absorb or dissipate the dose strength of the radiation sterilization 512 such that it becomes harmless to the sensitive electronics. In other embodiments, however, the converse can equally be true. More specifically, the size (e.g., length) of the collimator 506 can be decreased as long as the material for the collimator 506 exhibits a large enough mass density.

In addition to the radiation blocking characteristics of the body of the collimator 506, in some embodiments, one or more shields 516 (one shown) can be positioned within the sensor housing 304 to protect sensitive electronic components from radiation while the sensor control device 302 is subjected to the radiation sterilization 512. The shield 516, for example, can be positioned to interpose a data processing unit 518 and the radiation source (e.g., an e-beam electron accelerator). In such embodiments, the shield 516 can be positioned adjacent to and otherwise aligned with the data processing unit 518 and the radiation source to block or mitigate radiation exposure (e.g., e-beam radiation or energy) that might otherwise damage the sensitive electronic circuitry of the data processing unit 518.

The shield 516 can be made of any material capable of blocking (or substantially blocking) the transmission of radiation. Suitable materials for the shield 516 include, but are not limited to, lead, tungsten, iron-based metals (e.g., stainless steel), copper, tantalum, tungsten, osmium, or any combination thereof. Suitable metals can be corrosion-resistant, austenitic, and any non-magnetic metal with a density ranging between about 5 grams per cubic centimeter (g/cc) and about 15 g/cc. The shield 516 can be fabricated via a variety of manufacturing techniques including, but not limited to, stamping, casting, injection molding, sintering, two-shot molding, or any combination thereof.

In other embodiments, however, the shield 516 can comprise a metal-filled thermoplastic polymer such as, but not limited to, polyamide, polycarbonate, or polystyrene. In such embodiments, the shield 516 can be fabricated by mixing the shielding material in an adhesive matrix and dispensing the combination onto shaped components or otherwise directly onto the data processing unit 518. Moreover, in such embodiments, the shield 516 can comprise an enclosure that encapsulates (or substantially encapsulates) the data processing unit 518.

In some embodiments, a collimator seal 520 can be applied to the end of the collimator 506 to seal off the sterilization zone 508 and, thus, the sealed region 510. As illustrated, the collimator seal 520 can seal the second aperture 514b. The collimator seal 520 can be applied before or after the radiation sterilization 512. In embodiments where the collimator seal 520 is applied before undertaking the radiation sterilization 512, the collimator seal 520 can be made of a radiation permeable microbial barrier material that allows radiation to propagate therethrough. With the collimator seal 520 in place, the sealed region 510 is able to maintain a sterile environment for the assembled sensor control device 202 until the user removes (unthreads) the applicator cap.

In some embodiments, the collimator seal 520 can comprise two or more layers of different materials. The first layer can be made of a synthetic material (e.g., a flash-spun high- density polyethylene fiber), such as Tyvek^{®} available from DuPont^{®}. Tyvek^{®} is highly durable and puncture resistant and allows the permeation of vapors. The Tyvek^{®} layer can be applied before or after the radiation sterilization 512, and following the radiation sterilization 512, a foil or other vapor and moisture resistant material layer can be sealed (e.g., heat sealed) over the Tyvek^{®} layer to prevent the ingress of contaminants and moisture into the sterilization zone 508 and the sealed region 510. In other embodiments, the collimator seal 520 can comprise only a single protective layer applied to the end of the collimator 506. In such embodiments, the single layer is gas permeable for the sterilization process, but is also capable of protection against moisture and other harmful elements once the sterilization process is complete. Accordingly, the collimator seal 520 can operate as a moisture and contaminant layer, without departing from the scope of the disclosure.

It is noted that, while the sensor 216 and the sharp 218 extend from the bottom of the electronics housing 204 and into the sterilization zone 508 generally concentric with a centerline of the sensor applicator 102 and the applicator cap, it is contemplated herein to have an eccentric arrangement. More specifically, in at least one embodiment, the sensor 216 and the sharp 218 can extend from the bottom of the electronics housing 204 eccentric to the centerline of the sensor applicator 102 and the applicator cap. In such embodiments, the collimator 506 can be re-designed and otherwise configured such that the sterilization zone 508 is also eccentrically positioned to receive the sensor 216 and the sharp 218, without departing from the scope of the disclosure.

In some embodiments, the collimator 506 can comprise a first or "internal" collimator capable of being housed within the applicator cap or otherwise within the sensor applicator 102, as generally described above. A second or "external" collimator (not shown) can also be included or otherwise used in the assembly (manufacturing) process to help sterilize the sensor applicator 102. In such embodiments, the external collimator can be positioned external to the sensor applicator 102 and the applicator cap and used simultaneously with the internal collimator 506 to help focus the radiation sterilization 512 on the sensor 216 and the sharp 218.

In one embodiment, for example, the external collimator can initially receive the radiation sterilization 512. Similar to the internal collimator 506, the external collimator can provide or define a hole or passageway extending through the external collimator. The beams of the radiation sterilization 512 passing through the passageway of the external collimator can be focused and received into the sterilization zone 508 of the internal collimator 506 via the second aperture 514b. Accordingly, the external collimator can operate to pre-focus the radiation energy, and the internal collimator 506 can fully focus the radiation energy on the sensor 216 and the sharp 218.

In some embodiments, the internal collimator 506 can be omitted if the external collimator is capable of properly and fully focusing the radiation sterilization 512 to properly sterilize the sensor 216 and the sharp 218. In such embodiments, the sensor applicator can be positioned adjacent the external collimator and subsequently subjected to the radiation sterilization 512, and the external collimator can prevent radiation energy from damaging the sensitive electronics within the electronics housing 204. Moreover, in such embodiments, the sensor applicator 102 can be delivered to the user without the internal collimator 506 positioned within the applicator cap, thus eliminating complexity in manufacturing and use.

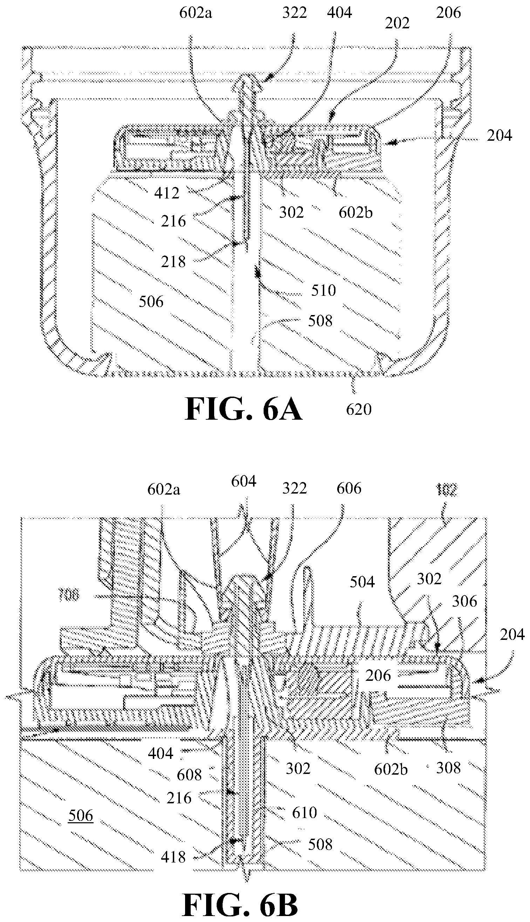

FIG. 6A is an enlarged cross-sectional side view of the sensor control device 202 mounted within the applicator cap, according to one or more embodiments. As indicated above, portions of the sensor 216 and the sharp 218 can be arranged within the sealed region 510 and thereby isolated from external contamination. The sealed region 510 can include (encompass) select portions of the interior of the electronics housing 204 and the sterilization zone 508 of the collimator 506. In one or more embodiments, the sealed region 510 can be defined and otherwise formed by at least a first seal 602a, a second seal 602b, and the collimator seal 520.

The first seal 602a can be arranged to seal the interface between the sharp hub 322 and the top of the electronics housing 204. More particularly, the first seal 602a can seal the interface between the sharp hub 322 and the shell 206. Moreover, the first seal 602a can circumscribe the first central aperture 404 defined in the shell 206 such that contaminants are prevented from migrating into the interior of the electronics housing 204 via the first central aperture 404. In some embodiments, the first seal 602a can form part of the sharp hub 322. For example, the first seal 602a can be overmolded onto the sharp hub 322. In other embodiments, the first seal 602a can be overmolded onto the top surface of the shell 206. In yet other embodiments, the first seal 602a can comprise a separate structure, such as an O-ring or the like, that interposes the sharp hub 322 and the top surface of the shell 206, without departing from the scope of the disclosure.

The second seal 602b can be arranged to seal the interface between the collimator 506 and the bottom of electronics housing 204. More particularly, the second seal 602b can be arranged to seal the interface between the mount 208 and the collimator 506 or, alternatively, between the collimator 506 and the bottom of the plug 302 as received within the bottom of the mount. In applications including the plug 302, as illustrated, the second seal 602b can be configured to seal about and otherwise circumscribe the plug receptacle 412. In embodiments that omit the plug 302, the second seal 602b can alternatively circumscribe the second central aperture 406 (FIG. 4A) defined in the mount 208. Consequently, the second seal 602b can prevent contaminants from migrating into the sterilization zone 508 of the collimator 506 and also from migrating into the interior of the electronics housing 204 via the plug receptacle 412 (or alternatively the second central aperture 406).

In some embodiments, the second seal 602b can form part of the collimator 506. For example, the second seal 602b can be overmolded onto the top of the collimator 506. In other embodiments, the second seal 602b can be overmolded onto the plug 302 or the bottom of the mount 208. In yet other embodiments, the second seal 602b can comprise a separate structure, such as an O-ring or the like, that interposes the collimator 506 and the plug 302 or the bottom of the mount 208, without departing from the scope of the disclosure.

Upon loading the sensor control device 202 into the sensor applicator 102 (FIG. 5B) and securing the applicator cap to the sensor applicator 102, the first and second seals 602a,b become compressed and generate corresponding sealed interfaces. The first and second seals 602a,b can be made of a variety of materials capable of generating a sealed interface between opposing structures. Suitable materials include, but are not limited to, silicone, a thermoplastic elastomer (TPE), polytetrafluoroethylene (PTFE or Teflon^{®}), or any combination thereof.

As discussed above, the collimator seal 520 can be configured to seal off the bottom of the sterilization zone 508 and, thus, the bottom of the sealed region 510. Accordingly, the first and second seals 602a,b and the collimator seal 520 each create corresponding barriers at their respective sealing locations. The combination of these seals 602a,b and 520 allows the sealed region 510 containing the sensor 216 and the sharp 218 to be terminally sterilized.

FIG. 6B is an enlarged cross-sectional side view of another embodiment of the sensor control device 302 mounted within the sensor applicator 102, according to one or more embodiments. More specifically, FIG. 6B depicts alternative embodiments of the first and second seals 602a,b. The first seal 602a is again arranged to seal the interface between the sharp hub 322 and the top of the electronics housing 204 and, more particularly, seal off the first central aperture 404 defined in the shell 206. In the illustrated embodiment, however, the first seal 602a can be configured to seal both axially and radially. More particularly, when the sensor control device 202 is introduced into the sensor applicator 102, the sharp hub 322 is received by the sensor carrier 504. The first seal 602a can be configured to simultaneously bias against one or more axially extending members 604 of the sensor carrier 504 and one or more radially extending members 606 of the sensor carrier 504. Such dual biased engagement compresses the first seal 602a both axially and radially and thereby allows the first seal 602a to seal against the top of the electronics housing 204 in both the radial and axial directions.

The second seal 602b is again arranged to seal the interface between the collimator 506 and the bottom of electronics housing 204 and, more particularly, between the mount 208 and the collimator 506 or, alternatively, between the collimator 506 and the bottom of the plug 302 as received within the bottom of the mount 208. In the illustrated embodiment, however, the second seal 602b can extend into the sterilization zone 508 and define or otherwise provide a cylindrical well 608 sized to receive the sensor 216 and the sharp as extending from the bottom of the mount 208. In some embodiments, a desiccant 610 can be positioned within the cylindrical well to aid maintenance of a low humidity environment for biological components sensitive to moisture.

In some embodiments, the second seal 602b can be omitted and the collimator 506 can be directly coupled to the electronics housing 204. More specifically, in at least one embodiment, the collimator 506 can be threadably coupled to the underside of the mount 208. In such embodiments, the collimator 506 can provide or otherwise define a threaded extension configured to mate with a threaded aperture defined in the bottom of the mount 208. Threadably coupling the collimator 506 to the mount 208 can seal the interface between the collimator 506 and the bottom of electronics housing 204, and thus operate to isolate sealed region 510. Moreover, in such embodiments, the pitch and gauge of the threads defined on the collimator 506 and the mount 208 can match those of the threaded engagement between the applicator cap and the sensor applicator 102. As a result, as the applicator cap is threaded to or unthreaded from the sensor applicator 102, the collimator 506 can correspondingly be threaded to or unthreaded from the electronics housing 304.

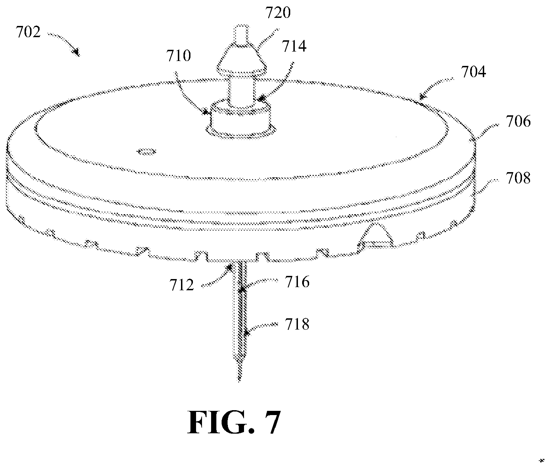

FIG. 7 is an isometric view of an example sensor control device 702, according to one or more additional embodiments of the present disclosure. The sensor control device 702 can be the same as or similar to the sensor control device 104 of FIG. 1 and, therefore, can be used in conjunction with the sensor applicator 102 (FIG. 1), which delivers the sensor control device 702 to a target monitoring location on a user's skin. Moreover, the sensor control device 702 can be alternately characterized as a medical device. Accordingly, the sensor control device 702 can also require proper sterilization prior to being used.

As illustrated, the sensor control device 702 includes an electronics housing 704 that is generally disc-shaped and can have a circular cross-section. In other embodiments, however, the electronics housing 704 can exhibit other cross-sectional shapes, such as ovoid (e.g., pill-shaped), a squircle, or polygonal, without departing from the scope of the disclosure. The electronics housing 704 can be configured to house or otherwise contain various electronic components used to operate the sensor control device 702.

The electronics housing 704 can include a shell 706 and a mount 708 that is matable with the shell 706. The shell 706 can be secured to the mount 708 via a variety of ways, such as a snap fit engagement, an interference fit, sonic welding, one or more mechanical fasteners (e.g., screws), or any combination thereof. In some cases, the shell 706 can be secured to the mount 708 such that a sealed interface therebetween is generated. In such embodiments, a gasket or other type of seal material can be positioned at or near the outer diameter (periphery) of the shell 706 and the mount 708, and securing the two components together can compress the gasket and thereby generate a sealed interface. In other embodiments, an adhesive can be applied to the outer diameter (periphery) of one or both of the shell 706 and the mount 708. The adhesive secures the shell 706 to the mount 708 and provides structural integrity, but can also seal the interface between the two components and thereby isolate the interior of the electronics housing 704 from outside contamination.

In the illustrated embodiment, the sensor control device 702 can further include a plug assembly 710 that can be coupled to the electronics housing 704. The plug assembly 710 can include a sensor module 712 (partially visible) interconnectable with a sharp module 714 (partially visible). The sensor module 712 can be configured to carry and otherwise include a sensor 716 (partially visible), and the sharp module 714 can be configured to carry and otherwise include a sharp 718 (partially visible) used to help deliver the sensor 716 transcutaneously under a user's skin during application of the sensor control device 702. The sharp module 714 can include a sharp hub 720 that carries the sharp 718.

As illustrated, corresponding portions of the sensor 716 and the sharp 718 extend from the electronics housing 704 and, more particularly, from the bottom of the mount 708. The exposed portion of the sensor 716 (alternately referred to as the "tail") can be received within a hollow or recessed portion of the sharp 718. The remaining portions of the sensor 716 are positioned within the interior of the electronics housing 704.

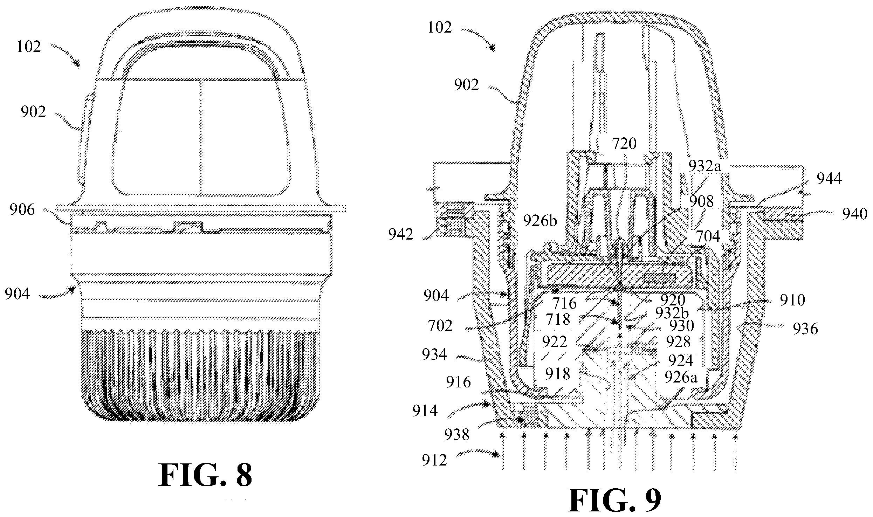

FIG. 8 is a side view of the sensor applicator 102 of FIG. 1. As illustrated, the sensor applicator 102 includes a housing 902 and an applicator cap 904 that can be removably coupled to the housing 902. In some embodiments, the applicator cap 904 can be threaded to the housing 902 and include a tamper ring 906. Upon rotating (e.g., unscrewing) the applicator cap 904 relative to the housing 902, the tamper ring 906 can shear and thereby free the applicator cap 904 from the sensor applicator 102. Once the applicator cap 904 is removed, a user can then use the sensor applicator 102 to position the sensor control device 702 (FIG. 7) at a target monitoring location on the user's body.

In some embodiments, the applicator cap 904 can be secured to the housing 902 via a sealed engagement to protect the internal components of the sensor applicator 102. In at least one embodiment, for example, an O-ring or another type of sealing gasket can seal an interface between the housing 902 and the applicator cap 904. The O-ring or sealing gasket can be a separate component part or alternatively molded onto one of the housing 902 and the applicator cap 904.

FIG. 9 is a cross-sectional side view of the sensor applicator 102. As illustrated, the sensor control device 902 can be received within the sensor applicator 102 and the applicator cap 904 can be coupled to the sensor applicator 102 to secure the sensor control device 702 therein. The sensor control device 702 can include one or more radiation sensitive components 708 arranged within the electronics housing 704. The radiation sensitive component 708 can include an electronic component or module such as, but not limited to, a data processing unit, a resistor, a transistor, a capacitor, an inductor, a diode, a switch, or any combination thereof. The data processing unit can comprise, for example, an application specific integrated circuit (ASIC) configured to implement one or more functions or routines associated with operation of the sensor control device 702. In operation, the data processing unit can perform data processing functions, such as filtering and encoding of data signals corresponding to a sampled analyte level of the user. The data processing unit can also include or otherwise communicate with an antenna for communicating with the reader device 106 (FIG. 1).

In the illustrated embodiment, a cap fill 910 can be positioned within the applicator cap 1404 and can generally help support the sensor control device 702 within the sensor applicator 102. In one or more embodiments, the cap fill 910 can comprise an integral part or extension of the applicator cap 904, such as being molded with or overmolded onto the applicator cap 904. In other embodiments, the cap fill 910 can comprise a separate structure fitted within or otherwise attached to the applicator cap 904, without departing from the scope of the disclosure.

The sensor control device 702 and, more particularly, the distal ends of the sensor 716 and the sharp 718 extending from the bottom of the electronics housing 1304, can be sterilized while positioned within the sensor applicator 102. In certain embodiments, the fully assembled sensor control device 702 can be subjected to radiation sterilization. The radiation sterilization 912 can be delivered either through continuous processing irradiation or through pulsed beam irradiation. In pulsed beam irradiation, the beam of radiation sterilization 912 is focused at a target location and the component part or device to be sterilized is moved to the target location at which point the irradiation is activated to provide a directed pulse of radiation. The radiation sterilization 912 is then turned off, and another component part or device to be sterilized is moved to the target location and the process is repeated.

According to the present disclosure, an external sterilization assembly 914 can be used to help focus the radiation 912 in sterilizing the distal ends of the sensor 716 and the sharp 718, while simultaneously preventing (impeding) propagating radiation 912 from damaging the radiation sensitive component 908. As illustrated, the external sterilization assembly 914 (hereafter the "assembly 914") can include a radiation shield 916 positioned at least partially external to the sensor applicator 102. The radiation shield 916 can provide or define an external collimator 918 configured to help focus the radiation 912 (e.g., beams, waves, energy, etc.) toward the components to be sterilized. More specifically, the external collimator 918 allows transmission of the radiation 912 to impinge upon and sterilize the sensor 716 and the sharp 718, but prevent the radiation 912 from damaging the radiation sensitive component 908 within the electronics housing 704.

In the illustrated embodiment, the external collimator 918 is designed to align with an internal collimator 920 defined by the cap fill 910. Similar to the external collimator 918, the internal collimator 920 can help focus the radiation 912 toward the components to be sterilized. As illustrated, the cap fill 910 can define a radial shoulder 922 sized to receive and otherwise mate with an end of the radiation shield 916, and the external collimator 918 transitions to the internal collimator 920 at the radial shoulder 922. In some embodiments, the transition between the external and internal collimators 918, 920 can be continuous, flush, or smooth. In other embodiments, however, the transition can be discontinuous or stepped, without departing from the scope of the disclosure.

The external and internal collimators 918, 920 can cooperatively define a sterilization zone 924 that focuses the radiation 912 and into which the distal ends of the sensor 916 and the sharp 918 can be positioned. The propagating radiation 912 can traverse the sterilization zone 924 to impinge upon and sterilize the sensor 716 and the sharp 718. However, the cap fill 910 and the radiation shield 916 can each be made of materials that substantially prevent the radiation 912 from penetrating the inner wall(s) of the sterilization zone 924 and thereby damaging the radiation sensitive component 908 within the housing 704. In other words, the cap fill 910 and the radiation shield 916 can each be made of materials having a density sufficient to absorb the dose of the beam energy being delivered. In some embodiments, for example, one or both of the cap fill 910 and the radiation shield 916 can be made of a material that has a mass density greater than 0.9 grams per cubic centimeter (g/cc). In other embodiments, however, the mass density of a suitable material can be less than 0.9 g/cc, without departing from the scope of the disclosure. Suitable materials for the cap fill 910 and the radiation shield 916 include, but are not limited to, a high-density polymer, (e.g., polyethylene, polypropylene, polystyrene, polytetrafluoroethylene, etc.), a metal (e.g., lead, stainless steel, aluminum, etc.), any combination thereof, or any material having a mass density greater than 0.9 g/cc. In at least one embodiment, the cap fill 910 can be made of machined or 3D printed polypropylene and the radiation shield 916 can be made of stainless steel.

In some embodiments, the design of the sterilization zone 924 can be altered so that one or both of the cap fill 910 and the radiation shield 916 can be made of a material that has a mass density less than 0.9 g/cc but can still operate to prevent the radiation sterilization 912 from damaging the radiation sensitive component 908. In such embodiments, the size (e.g., length) of the sterilization zone 924 can be increased such that the propagating electrons from the radiation sterilization 912 are required to pass through a larger amount of material before potentially impinging upon the radiation sensitive component 908. The larger amount of material can help absorb or dissipate the dose strength of the radiation 912 such that it becomes harmless to the sensitive electronics. In other embodiments, however, the converse can equally be true. More specifically, the size (e.g., length) of the sterilization zone 924 can be decreased as long as the material for the cap fill 910 and/or the radiation shield 916 exhibits a large enough mass density.

The sterilization zone 924 defined by the external and internal collimators 918, 920 can exhibit any suitable cross-sectional shape necessary to properly focus the radiation 912 on the sensor 716 and the sharp 718 for sterilization. In the illustrated embodiment, for example, the external and internal collimators 918, 920 each exhibit a circular cross-section with parallel sides. In other embodiments, however, one or both of the external and internal collimators 918, 920 can exhibit a polygonal cross-sectional shape, such as cubic or rectangular (e.g., including parallelogram), without departing from the scope of the disclosure.

In the illustrated embodiment, the sterilization zone 924 provides a first aperture 926a defined by the external collimator 918 and a second aperture 926b defined by the internal collimator 920, where the first and second apertures 926a,b are located at opposing ends of the sterilization zone 924. The first aperture 926a permits the radiation 912 to enter the sterilization zone 924, and the second aperture 926b provides a location where radiation 912 can impact the sensor 716 and the sharp 718. In the illustrated embodiment, the second aperture 926b also provides a location where the sensor 716 and the sharp 718 can be received into the sterilization zone 924. In embodiments where the sterilization zone 924 has a circular cross-section, the diameters of the first and second apertures 926a,b can be substantially the same.

In some embodiments, the sterilization zone 924 defined by the external and internal collimators 918 can be substantially cylindrical and otherwise exhibit a circular or polygonal cross-section. In such embodiments, the first and second apertures 926a,b can exhibit identical diameters and the walls of the sterilization zone 924 can be substantially parallel between the first and second ends of the sterilization zone 924.

In some embodiments, a cap seal 928 (shown in dashed lines) can be arranged at the interface between the cap fill 910 and the radiation shield 916. The cap seal 928 can comprise a radiation permeable microbial barrier. In some embodiments, for example, the cap seal 928 can be made of a synthetic material (e.g., a flash-spun high-density polyethylene fiber), such as TYVEK^{®} available from DuPont^{®}. The cap seal 928 can seal off a portion of the sterilization zone 924 to help form part of a sealed region 930 configured to isolate the sensor 716 and the sharp 718 from external contamination.

The sealed region 930 can include (encompass) select portions of the interior of the electronics housing 704 and the sterilization zone 924. In one or more embodiments, the sealed region 930 can be defined and otherwise formed by at least the cap seal 928, a first or "top" seal 932a, and a second or "bottom" seal 932b. The cap seal 928 and the top and bottom seals 932a,b can each create corresponding barriers at their respective sealing locations, thereby allowing the sterilization zone 924 containing the sensor 716 and the sharp 718 to be terminally sterilized.

The top seal 932a can be arranged to seal the interface between the sharp hub 720 and the top of the electronics housing 704 (i.e., the shell 906 of FIG. 8) and thereby prevent contaminants from migrating into the interior of the electronics housing 704. In some embodiments, the top seal 932a can form part of the sharp hub 720, such as being overmolded onto the sharp hub 720. In other embodiments, however, the top seal 932a can form part of or be overmolded onto the top surface of the shell 706. In yet other embodiments, the top seal 932a can comprise a separate structure, such as an O-ring or the like, that interposes the sharp hub 720 and the top surface of the shell 706, without departing from the scope of the disclosure.

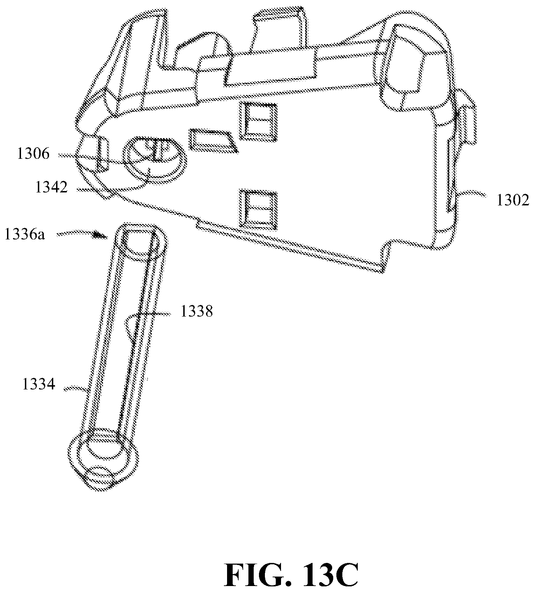

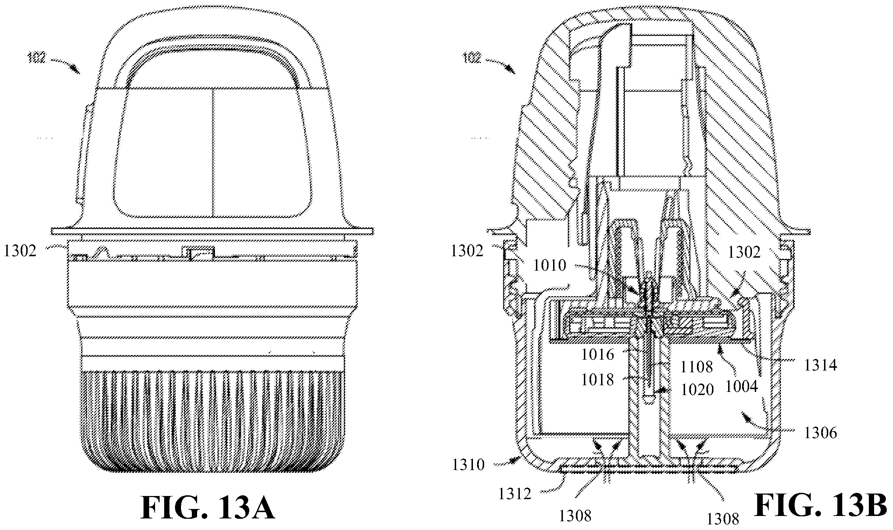

The bottom seal 932b can be arranged to seal the interface between the cap fill 910 and the bottom of electronics housing (i.e., the mount 708 of FIG. 13). The bottom seal 932b can prevent contaminants from migrating into the sterilization zone 924 and from migrating into the interior of the electronics housing 704. In some embodiments, the bottom seal 932b can form part of the cap fill 910, such as being overmolded onto the top of the cap fill 910. In other embodiments, the bottom seal 932b can form part of or be overmolded onto the bottom of the mount 708. In yet other embodiments, the bottom seal 932b can comprise a separate structure, such as an O-ring or the like, that interposes the cap fill 910 and the bottom of the mount 708, without departing from the scope of the disclosure.

Upon loading the sensor control device 702 into the sensor applicator 102 and securing the applicator cap 904 to the sensor applicator 102, the top and bottom seals 932a,b can compress and generate corresponding sealed interfaces. The top and bottom seals 932a,b can be made of a variety of materials capable of generating a sealed interface between opposing structures. Suitable materials include, but are not limited to, silicone, a thermoplastic elastomer (TPE), polytetrafluoroethylene (e.g., TEFLON^{®}), or any combination thereof.

It is noted that, while the sensor 716 and the sharp 718 extend from the bottom of the electronics housing 704 and into the sterilization zone 924 generally concentric with a centerline of the sensor applicator 102 and the applicator cap 904, it is contemplated herein to have an eccentric arrangement. More specifically, in at least one embodiment, the sensor 716 and the sharp 718 can extend from the bottom of the electronics housing 704 eccentric to the centerline of the sensor applicator 102 and the applicator cap 904. In such embodiments, the external and internal collimators 918, 920 can be re-designed and otherwise configured such that the sterilization zone 924 is also eccentrically positioned to receive the sensor 716 and the sharp 718, without departing from the scope of the disclosure.

In some embodiments, the external sterilization assembly 914 can further include a sterilization housing or "pod" 934 coupled to or forming part of the radiation shield 916. The sterilization pod 934 provides and otherwise defines a chamber 936 sized to receive all or a portion of the sensor applicator 102. Once properly seated (received) within the sterilization pod 934, the sensor applicator 102 can be subjected to the radiation sterilization 912 to sterilize the sensor 716 and the sharp 718. The sterilization pod 934 can be made of any of the materials mentioned herein for the radiation shield 916 to help prevent the radiation 912 from propagating through the walls of the sterilization pod 934.

In some embodiments, the radiation shield 916 can be removably coupled to the sterilization pod 934 using one or more mechanical fasteners 938 (one shown), but could alternatively be removably coupled via an interference fit, a snap fit engagement, etc. Removably coupling the radiation shield 916 to the sterilization pod 934 enables the radiation shield 916 to be interchangeable with differently designed (sized) shields to fit particular sterilization applications for varying types and designs of the sensor applicator 102. Accordingly, the sterilization pod 934 can comprise a universal mount that allows the radiation shield 916 to be interchanged with other shield designs having different parameters for the external collimator 918, as needed.

In some embodiments, the external sterilization assembly 914 can further include a mounting tray 940 coupled to or forming part of the sterilization pod 934. The sterilization pod 934 can be removably coupled to the mounting tray 940 using, for example, one or more mechanical fasteners 942 (one shown). The mounting tray 940 can provide or define a central aperture 944 sized to receive the sensor applicator 102 and alignable with the chamber 936 to enable the sensor applicator 102 to enter the chamber 936. As described below, in some embodiments, the mounting tray 940 can define a plurality of central apertures 944 for receiving a corresponding plurality of sensor applicators for sterilization.

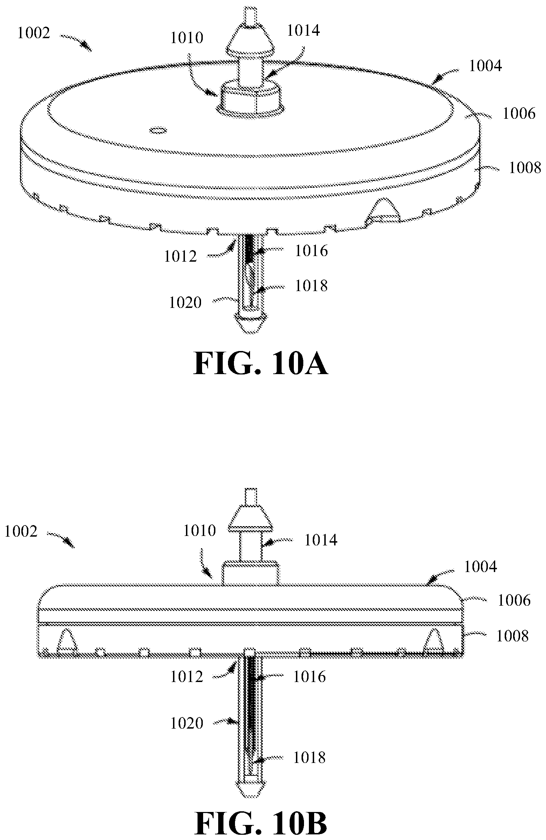

FIGS. 10A and 10B are isometric and side views, respectively, of an example sensor control device 1002, according to one or more embodiments of the present disclosure. The sensor control device 1002 (alternately referred to as a "puck") can be similar in some respects to the sensor control device 104 of FIG. 1 and therefore can be best understood with reference thereto. The sensor control device 1002 can replace the sensor control device 104 of FIG. 1 and, therefore, can be used in conjunction with the sensor applicator 102 (FIG. 1), which delivers the sensor control device 1002 to a target monitoring location on a user's skin.

The sensor control device 1002, however, can be incorporated into a one-piece system architecture in contrast to the sensor control device 104 of FIG. 1. Unlike the two-piece architecture, for example, a user is not required to open multiple packages and finally assemble the sensor control device 1002. Rather, upon receipt by the user, the sensor control device 1002 is already fully assembled and properly positioned within the sensor applicator 102 (FIG. 1). To use the sensor control device 1002, the user need only open one barrier (e.g., the applicator cap) before promptly delivering the sensor control device 1002 to the target monitoring location.

As illustrated, the sensor control device 1002 includes an electronics housing 1004 that is generally disc-shaped and can have a circular cross-section. In other embodiments, however, the electronics housing 1004 can exhibit other cross-sectional shapes, such as ovoid or polygonal, without departing from the scope of the disclosure. The electronics housing 1004 can be configured to house or otherwise contain various electrical components used to operate the sensor control device 1002.

The electronics housing 1004 can include a shell 1006 and a mount 1008 that is matable with the shell 1006. The shell 1006 can be secured to the mount 1008 via a variety of ways, such as a snap fit engagement, an interference fit, sonic welding, or one or more mechanical fasteners (e.g., screws). In some cases, the shell 1006 can be secured to the mount 1008 such that a sealed interface therebetween is generated. In such embodiments, a gasket or other type of seal material can be positioned at or near the outer diameter (periphery) of the shell 1006 and the mount 1008, and securing the two components together can compress the gasket and thereby generate a sealed interface. In other embodiments, an adhesive can be applied to the outer diameter (periphery) of one or both of the shell 1006 and the mount 1008. The adhesive secures the shell 1006 to the mount 1008 and provides structural integrity, but can also seal the interface between the two components and thereby isolate the interior of the electronics housing 1004 from outside contamination. If the sensor control device 1002 is assembled in a controlled environment, there can be no need to terminally sterilize the internal electrical components. Rather, the adhesive coupling can provide a sufficient sterile barrier for the assembled electronics housing 1004.

The sensor control device 1002 can further include a plug assembly 1010 that can be coupled to the electronics housing 1004. The plug assembly 1010 can be similar in some respects to the plug assembly. For example, the plug assembly 1010 can include a sensor module 1012 (partially visible) interconnectable with a sharp module 1014 (partially visible). The sensor module 1012 can be configured to carry and otherwise include a sensor 2616 (partially visible), and the sharp module 1014 can be configured to carry and otherwise include a sharp 1018 (partially visible) used to help deliver the sensor 1016 transcutaneously under a user's skin during application of the sensor control device 1002. As illustrated, corresponding portions of the sensor 1016 and the sharp 1018 extend from the electronics housing 1004 and, more particularly, from the bottom of the mount 1008. The exposed portion of the sensor 1016 can be received within a hollow or recessed portion of the sharp 1018. The remaining portion of the sensor 1016 is positioned within the interior of the electronics housing 1004.

As discussed in more detail below, the sensor control device 1002 can further include a sensor preservation vial 1020 that provides a preservation barrier surrounding and protecting the exposed portions of the sensor 1016 and the sharp 1018 from gaseous chemical sterilization.

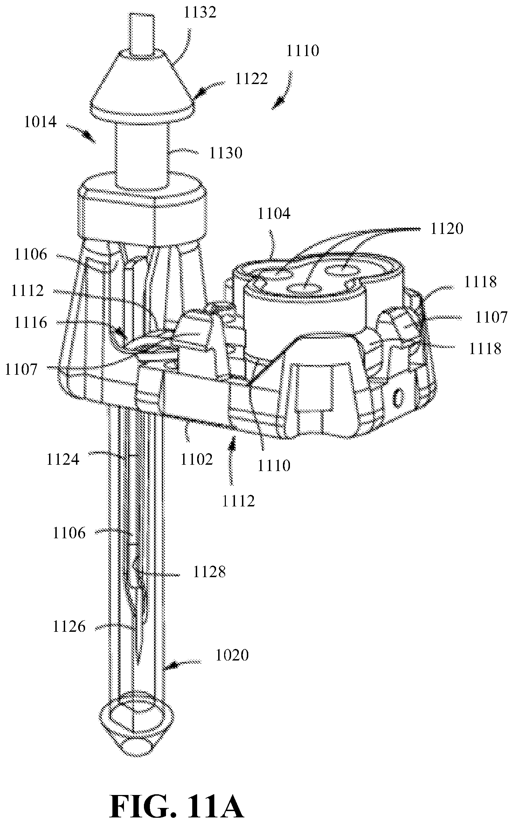

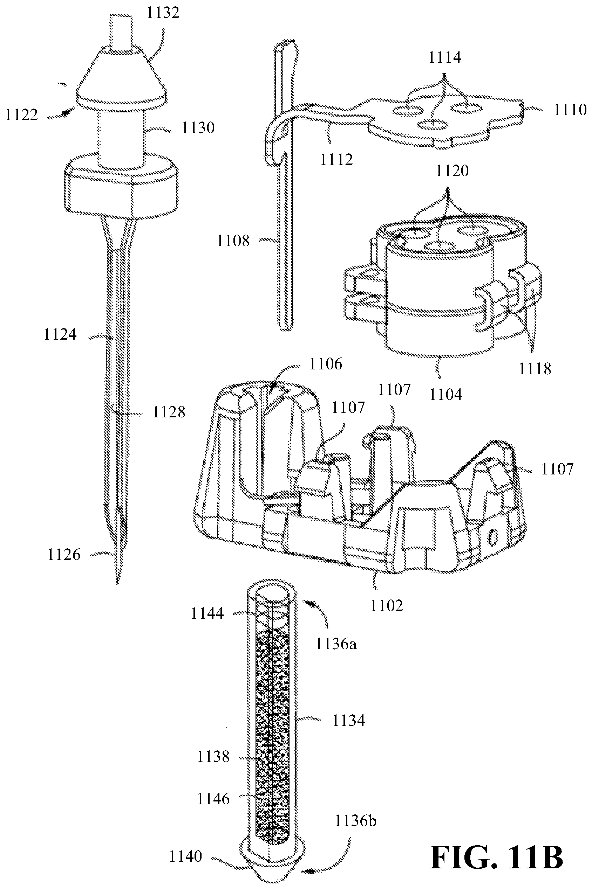

FIGS. 11A and11B are isometric and exploded views, respectively, of the plug assembly 1110, according to one or more embodiments. The sensor module 1012 can include the sensor 1016, a plug, and a connector. The plug can be designed to receive and support both the sensor 1016 and the connector 1104. As illustrated, a channel can be defined through the plug to receive a portion of the sensor 1016. Moreover, the plug can provide one or more deflectable arms configured to snap into corresponding features provided on the bottom of the electronics housing 1004.

The sensor 1016 includes a tail 1108, a flag 1110, and a neck 1112 that interconnects the tail 1108 and the flag 1110. The tail 1108 can be configured to extend at least partially through the channel 1106 and extend distally from the plug 1102. The tail 1108 includes an enzyme or other chemistry or biologic and, in some embodiments, a membrane can cover the chemistry. In use, the tail 1108 is transcutaneously received beneath a user's skin, and the chemistry included thereon helps facilitate analyte monitoring in the presence of bodily fluids.

The flag 1110 can comprise a generally planar surface having one or more sensor contacts 114 (three shown in FIG. 11B) arranged thereon. The sensor contact(s) 114 can be configured to align with a corresponding number of compliant carbon impregnated polymer modules (tops of which shown at 1120) encapsulated within the connector 1104.

The connector 1104 includes one or more hinges 1118 that enables the connector 1104 to move between open and closed states. The connector 1104 is depicted in FIGS. 11A and 11B in the closed state, but can pivot to the open state to receive the flag 1110 and the compliant carbon impregnated polymer module(s) therein. The compliant carbon impregnated polymer module(s) provide electrical contacts 1120 (three shown) configured to provide conductive communication between the sensor 1016 and corresponding circuitry contacts provided within the electrical housing 1004 (FIGS. 10A and 10B). The connector 1104 can be made of silicone rubber and can serve as a moisture barrier for the sensor 1016 when assembled in a compressed state and after application to a user's skin.

The sharp module 1014 includes the sharp 1018 and a sharp hub 1122 that carries the sharp 1018. The sharp 1018 includes an elongate shaft 1124 and a sharp tip 1126 at the distal end of the shaft 1124. The shaft 1124 can be configured to extend through the channel 1106 and extend distally from the plug 1102. Moreover, the shaft 1124 can include a hollow or recessed portion 1128 that at least partially circumscribes the tail 1108 of the sensor 1016. The sharp tip 1126 can be configured to penetrate the skin while carrying the tail 1108 to put the active chemistry present on the tail 1108 into contact with bodily fluids.

The sharp hub 1122 can include a hub small cylinder 2730 and a hub snap pawl 2732, each of which can be configured to help couple the plug assembly 2610 (and the entire sensor control device 2602) to the sensor applicator 102 (FIG. 1).

With specific reference to FIG. 11B, the preservation vial 1020 can comprise a generally cylindrical and elongate body 1134 having a first end 1136a and a second end 1136b opposite the first end 1136a. The first end 1136a can be open to provide access into an inner chamber 1138 defined within the body 1134. In contrast, the second end 1136b can be closed and can provide or otherwise define an enlarged head 1140. The enlarged head 1140 exhibits an outer diameter that is greater than the outer diameter of the remaining portions of the body 1134. In other embodiments, however, the enlarged head 1140 can be positioned at an intermediate location between the first and second ends 1136a,b.

FIG. 11C is an exploded isometric bottom view of the plug 1102 and the preservation vial 1020. As illustrated, the plug 1102 can define an aperture 1142 configured to receive the preservation vial 1020 and, more particularly, the first end 1136a of the body 1134. The channel 1106 can terminate at the aperture 1142 such that components extending out of and distally from the channel 1106 will be received into the inner chamber 1138 when the preservation vial 1020 is coupled to the plug 1102.

The preservation vial 1020 can be removably coupled to the plug 1102 at the aperture 1142. In some embodiments, for example, the preservation vial 1020 can be received into the aperture 1142 via an interference or friction fit. In other embodiments, the preservation vial 1020 can be secured within the aperture 1142 with a frangible member (e.g., a shear ring) or substance that can be broken with minimal separation force. In such embodiments, for example, the preservation vial 1020 can be secured within the aperture 1142 with a tag (spot) of glue, a dab of wax, or the preservation vial 1020 can include an easily peeled off glue. As described below, the preservation vial 1020 can be separated from the plug 1102 prior to delivering the sensor control device 1002 (FIGS. 10A and 10B) to the target monitoring location on the user's skin.

Referring again to FIGS. 11A and 11B, the inner chamber 1138 can be sized and otherwise configured to receive the tail 1108, a distal section of the shaft 1124, and the sharp tip 1126, collectively referred to as the "distal portions of the sensor 1016 and the sharp 1018." The inner chamber 1138 can be sealed or otherwise isolated to prevent substances that might adversely interact with the chemistry of the sensor 1016 from migrating into the inner chamber 1138. More specifically, the inner chamber 1128 can be sealed to protect or isolate the distal portions of the sensor 1016 and the sharp 1018 during a gaseous chemical sterilization process since gases used during gaseous chemical sterilization can adversely affect the enzymes (and other sensor components, such as membrane coatings that regulate analyte influx) provided on the tail 1108.

In some embodiments, a seal 1144 (FIG. 11B) can provide a sealed barrier between the inner chamber 1138 and the exterior environment. In at least one embodiment, the seal 1144 can be arranged within the inner chamber 1138, but could alternatively be positioned external to the body 1134, without departing from the scope of the disclosure. The distal portions of the sensor 1016 and the sharp 1018 can penetrate the seal 1144 and extend into the inner chamber 1138, but the seal 1144 can maintain a sealed interface about the distal portions of the sensor 1016 and the sharp 1018 to prevent migration of contaminants into the inner chamber 1138. The seal 1144 can be made of, for example, a pliable elastomer or a wax.

In other embodiments (or in addition to the seal 1144), a sensor preservation fluid 1146 (FIG. 11B) can be present within the inner chamber 1138 and the distal portions of the sensor 1016 and the sharp 1018 can be immersed in or otherwise encapsulated by the preservation fluid 1146. The preservation fluid 1146 can generate a sealed interface that prevents sterilization gases from interacting with the enzymes provided on the tail 1108.

The plug assembly 1010 can be subjected to radiation sterilization to properly sterilize the sensor 1016 and the sharp 1018. Suitable radiation sterilization processes include, but are not limited to, electron beam (e-beam) irradiation, gamma ray irradiation, X-ray irradiation, or any combination thereof. In some embodiments, the plug assembly 1010 can be subjected to radiation sterilization prior to coupling the preservation vial 1020 to the plug 1102. In other embodiments, however, the plug assembly 1010 can sterilized after coupling the preservation vial 1020 to the plug 1102. In such embodiments, the body 2734 of the preservation vial 1020 and the preservation fluid 1146 can comprise materials and/or substances that permit the propagation of radiation therethrough to facilitate radiation sterilization of the distal portions of the sensor 1016 and the sharp 1018.

Suitable materials for the body 1134 include, but are not limited to, a non-magnetic metal (e.g., aluminum, copper, gold, silver, etc.), a thermoplastic, ceramic, rubber (e.g., ebonite), a composite material (e.g., fiberglass, carbon fiber reinforced polymer, etc.), an epoxy, or any combination thereof. In some embodiments, the material for the body 1134 can be transparent or translucent, but can otherwise be opaque, without departing from the scope of the disclosure.

The preservation fluid 1146 can comprise any inert and biocompatible fluid (i.e., liquid, gas, gel, wax, or any combination thereof) capable of encapsulating the distal portions of the sensor 1016 and the sharp 1018. In some embodiments, the preservation fluid 1146 can also permit the propagation of radiation therethrough. The preservation fluid 1146 can comprise a fluid that is insoluble with the chemicals involved in gaseous chemical sterilization. Suitable examples of the preservation fluid 1146 include, but are not limited to, silicone oil, mineral oil, a gel (e.g., petroleum jelly), a wax, fresh water, salt water, a synthetic fluid, glycerol, sorbitan esters, or any combination thereof. As will be appreciated, gels and fluids that are more viscous can be preferred so that the preservation fluid 1146 does not flow easily.

In some embodiments, the preservation fluid 1146 can include an anti-inflammatory agent, such as nitric oxide or another known anti-inflammatory agent. The anti- inflammatory agent can prove advantageous in minimizing local inflammatory response caused by penetration of the sharp 1018 and the sensor 1016 into the skin of the user. It has been observed that inflammation can affect the accuracy of glucose readings, and by including the anti- inflammatory agent the healing process can be accelerated, which can result in obtaining accurate readings more quickly.

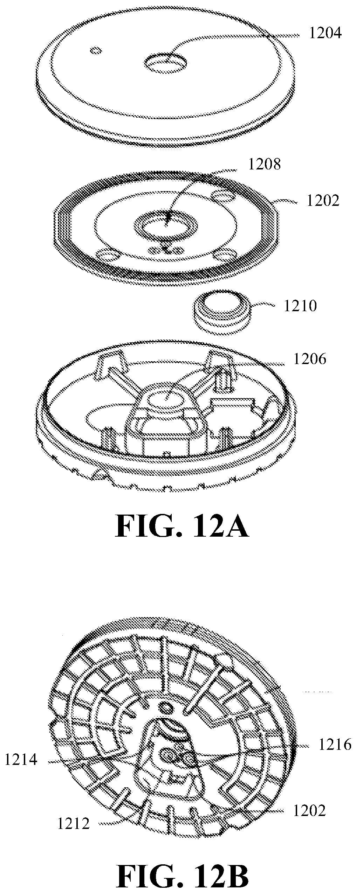

FIGS. 12A and 12B are exploded and bottom isometric views, respectively, of the electronics housing 1004, according to one or more embodiments. The shell 1006 and the mount 1008 operate as opposing clamshell halves that enclose or otherwise substantially encapsulate the various electronic components of the sensor control device 1002 (FIGS. 10A and 10B).

A printed circuit board (PCB) 1202 can be positioned within the electronics housing 1004. A plurality of electronic modules (not shown) can be mounted to the PCB 1202 including, but not limited to, a data processing unit, resistors, transistors, capacitors, inductors, diodes, and switches. The data processing unit can comprise, for example, an application specific integrated circuit (ASIC) configured to implement one or more functions or routines associated with operation of the sensor control device 1002. More specifically, the data processing unit can be configured to perform data processing functions, where such functions can include but are not limited to, filtering and encoding of data signals, each of which corresponds to a sampled analyte level of the user. The data processing unit can also include or otherwise communicate with an antenna for communicating with the reader device 106 (FIG. 1).

As illustrated, the shell 1006, the mount 1008, and the PCB 1202 each define corresponding central apertures 1204, 1206, and 1208, respectively. When the electronics housing 1204 is assembled, the central apertures 1204, 1206, 1208 coaxially align to receive the plug assembly 1010 (FIGS. 11A and 11B) therethrough. A battery 1210 can also be housed within the electronics housing 1004 and configured to power the sensor control device 1002.

In FIG. 12B, a plug receptacle 1212 can be defined in the bottom of the mount 1208 and provide a location where the plug assembly 1010 (FIGS. 10A and 10B) can be received and coupled to the electronics housing 1004, and thereby fully assemble the sensor control device 1002. The profile of the plug 1102 (FIGS. 11A-11C) can match or be shaped in complementary fashion to the plug receptacle 1212, and the plug receptacle 1212 can provide one or more snap ledges 1214 (two shown) configured to interface with and receive the deflectable arms 1107 (FIGS. 11A and 11B) of the plug 1102. The plug assembly 1010 is coupled to the electronics housing 1004 by advancing the plug 1102 into the plug receptacle 1212 and allowing the deflectable arms 1107 to lock into the corresponding snap ledges 1214. When the plug assembly 1010 is properly coupled to the electronics housing 1004, one or more circuitry contacts 1216 (three shown) defined on the underside of the PCB 1202 can make conductive communication with the electrical contacts 1120 (FIGS. 11A and 11B) of the connector 1104 (FIGS. 11A and 11B).

FIGS. 13A and 13B are side and cross-sectional side views, respectively, of an example embodiment of the sensor applicator 102 with the applicator cap coupled thereto. More specifically, FIGS. 13A and 13B depict how the sensor applicator 102 might be shipped to and received by a user. According to the present disclosure, and as seen in FIG. 13B, the sensor control device 1002 is already assembled and installed within the sensor applicator 102 prior to being delivered to the user.

As indicated above, prior to coupling the plug assembly 1010 to the electronics housing 1004, the plug assembly 1010 can be subjected to radiation sterilization to sterilize the distal portions of the sensor 1016 and the sharp 1018. Once properly sterilized, the plug assembly 1010 can then be coupled to the electronics housing 1004, as generally described above, and thereby form the fully assembled sensor control device 1002. The sensor control device 1002 can then be loaded into the sensor applicator 102, and the applicator cap can be coupled to the sensor applicator 102. The applicator cap can be threaded to the housing and include a tamper ring. Upon rotating (e.g., unscrewing) the applicator cap relative to the housing, the tamper ring can shear and thereby free the applicator cap from the sensor applicator 102.

According to the present disclosure, while loaded in the sensor applicator 102, the sensor control device 1002 can be subjected to gaseous chemical sterilization configured to sterilize the electronics housing 1004 and any other exposed portions of the sensor control device 1002. To accomplish this, a chemical can be injected into a sterilization chamber 1306 cooperatively defined by the sensor applicator 102 and the interconnected cap 210. In some applications, the chemical can be injected into the sterilization chamber 1306 via one or more vents 1308 defined in the applicator cap at its proximal end 1310. Example chemicals that can be used for the gaseous chemical sterilization 1304 include, but are not limited to, ethylene oxide, vaporized hydrogen peroxide, and nitrogen oxide (e.g., nitrous oxide, nitrogen dioxide, etc.).

Since the distal portions of the sensor 1016 and the sharp 1018 are sealed within the preservation vial 1020, the chemicals used during the gaseous chemical sterilization process do not interact with the enzymes, chemistry or biologics provided on the tail 1108.

Once a desired sterility assurance level has been achieved within the sterilization chamber 1306, the gaseous solution is removed and the sterilization chamber 1306 is aerated. Aeration can be achieved by a series of vacuums and subsequently circulating nitrogen gas or filtered air through the sterilization chamber 1306. Once the sterilization chamber 1306 is properly aerated, the vents 1308 can be occluded with a seal 1312 (shown in dashed lines).

In some embodiments, the seal 1312 can comprise two or more layers of different materials. The first layer can be made of a synthetic material (e.g., a flash-spun high-density polyethylene fiber), such as Tyvek^{®} available from DuPont^{®}. Tyvek^{®} is highly durable and puncture resistant and allows the permeation of vapors. The Tyvek^{®} layer can be applied before the gaseous chemical sterilization process, and following the gaseous chemical sterilization process, a foil or other vapor and moisture resistant material layer can be sealed (e.g., heat sealed) over the Tyvek^{®} layer to prevent the ingress of contaminants and moisture into the sterilization chamber 1306. In other embodiments, the seal 1312 can comprise only a single protective layer applied to the applicator cap. In such embodiments, the single layer is gas permeable for the sterilization process, but is also capable of protection against moisture and other harmful elements once the sterilization process is complete.

With the seal 1312 in place, the applicator cap provides a barrier against outside contamination, and thereby maintains a sterile environment for the assembled sensor control device 1002 until the user removes (unthreads) the applicator cap. The applicator cap can also create a dust-free environment during shipping and storage that prevents an adhesive patch 1314 used to secure the sensor control device 1002 to the user's skin from becoming dirty.

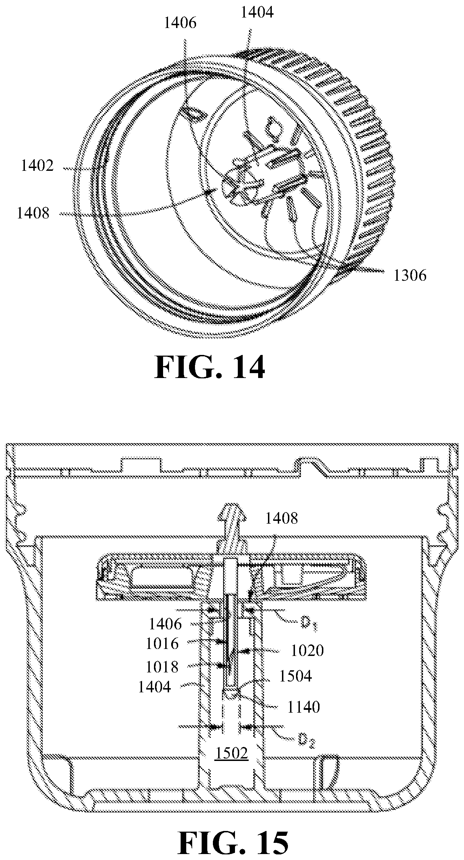

FIG. 14 is a perspective view of an example embodiment of the applicator cap, according to the present disclosure. As illustrated, the applicator cap has a generally circular cross-section and defines a series of threads used to couple the applicator cap to the sensor applicator 102. The vents 1308 are also visible in the bottom of the applicator cap.

The applicator cap can further provide and otherwise define a cap post 1404 centrally located within the interior of the applicator cap and extending proximally from the bottom thereof. The cap post 4104 can be configured to help support the sensor control device 1002 while contained within the sensor applicator 102. Moreover, the cap post 1404 can define an opening 1406 configured to receive the preservation vial 1020 as the applicator cap 210 is coupled to the sensor applicator 102.

In some embodiments, the opening 1406 to the cap post 1404 can include one or more compliant features 1408 that are expandable or flexible to enable the preservation vial 1020 to pass therethrough. In some embodiments, for example, the compliant feature(s) 1408 can comprise a collet-type device that includes a plurality of compliant fingers configured to flex radially outward to receive the preservation vial 1020. In other embodiments, however, the compliant feature(s) 1408 can comprise an elastomer or another type of compliant material configured to expand radially to receive the preservation vial 1020.

FIG. 15 is a cross-sectional side view of the sensor control device 1002 positioned within the applicator cap, according to one or more embodiments. As illustrated, the cap post 1404 defines a post chamber 1502 configured to receive the preservation vial 1020. The opening 3006 to the cap post 3004 provides access into the post chamber 1502 and exhibits a first diameter D1. In contrast, the enlarged head 1140 of the preservation vial 1020 exhibits a second diameter D2 that is larger than the first diameter D1 and greater than the outer diameter of the remaining portions of the preservation vial 1020. Accordingly, as the preservation vial 2620 is extended into the post chamber 1502, the compliant feature(s) 1408 of the opening 1406 can flex (expand) radially outward to receive the enlarged head 1140.

In some embodiments, the enlarged head 1140 can provide or otherwise define an angled outer surface that helps bias the compliant feature(s) 1408 radially outward. The enlarged head 1140, however, can also define an upper shoulder 1504 that prevents the preservation vial 1020 from reversing out of the post chamber 1502. More specifically, the shoulder 1504 can comprise a sharp surface at the second diameter D2 that will engage but not urge the compliant feature(s) 1408 to flex radially outward in the reverse direction.

Once the enlarged head 1140 bypasses the opening 1406, the compliant feature(s) 1408 flex back to (or towards) their natural state. In some embodiments, the compliant feature(s) 1408 can engage the outer surface of the preservation vial 1020, but can nonetheless allow the applicator cap 210 to rotate relative to the preservation vial 1020. Accordingly, when a user removes the applicator cap by rotating the applicator cap relative to the sensor applicator 102, the preservation vial 1020 can remain stationary relative to the cap post 1404.

Upon removing the applicator cap from the sensor applicator 102, and thereby also separating the sensor control device 1002 from the applicator cap, the shoulder 1504 defined on the enlarged head 1140 will engage the compliant feature(s) 1408 at the opening 1406. Because the diameter of the shoulder 1504 is greater than the diameter of the opening 1406, the shoulder 1504 will bind against the compliant feature(s) 1408 and thereby separate the preservation vial 1020 from the sensor control device 1002, which exposes the distal portions of the sensor 1016 and the sharp 1018. Accordingly, the compliant feature(s) 1408 can prevent the enlarged head 1140 from exiting the post chamber 1502 via the opening 1406 upon separating the applicator cap from the sensor applicator 102 and the sensor control device 1002. The separated preservation vial 1020 will fall into and remain within the post chamber 1502.

In some embodiments, instead of the opening 1406 including the compliant feature(s) 1408, as generally described above, the opening 1406 can alternatively be threaded. In such embodiments, a small portion near the distal end of the preservation vial 1020 can also be threaded and configured to threadably engage the threads of the opening 1406. The preservation vial 1020 can be received within the post chamber 1502 via threaded rotation. Upon removing the applicator cap from the sensor applicator 102, however, the opposing threads on the opening 1406 and the preservation vial 1020 bind and the preservation vial 1020 can be separated from the sensor control device 1002.

Accordingly, there are several advantages to incorporating the sensor control device 1002 into an analyte monitoring system (e.g., the analyte monitoring system 100 of FIG. 1). Since the sensor control device 1002 is finally assembled in a controlled environment, tolerances can be reduced or eliminated altogether, which allows the sensor control device 1002 to be thin and small. Moreover, since the sensor control device 1002 is finally assembled in a controlled environment, a thorough pre-test of the sensor control device 1002 can be undertaken at the factory, thus fully testing the sensor unit prior to packaging for final delivery.

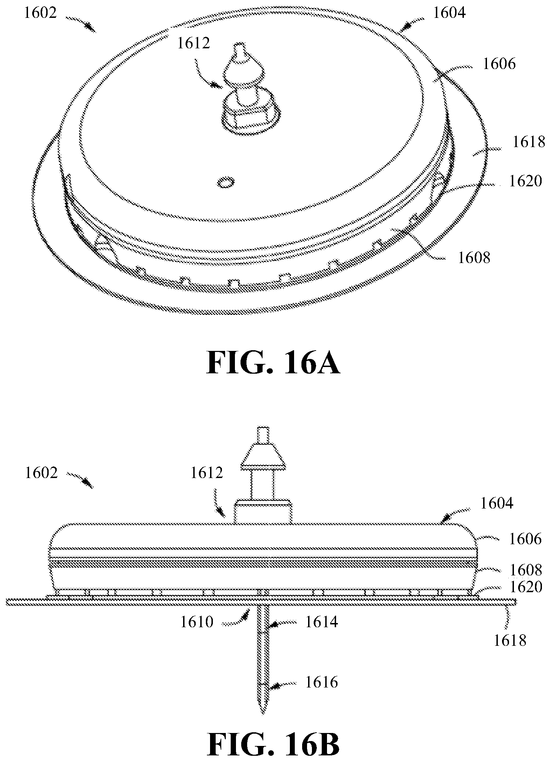

FIGS. 16A and 16B are isometric and side views, respectively, of an example sensor control device 1602, according to one or more embodiments of the present disclosure. The sensor control device 1602 (alternately referred to as a "puck") can be similar in some respects to the sensor control device 104 of FIG. 1 and therefore can be best understood with reference thereto. In some applications, the sensor control device 1602 can replace the sensor control device 104 of FIG. 1 and, therefore, can be used in conjunction with the sensor applicator 102 (FIG. 1), which delivers the sensor control device 1602 to a target monitoring location on a user's skin.

The sensor control device 1602, however, can be incorporated into a one-piece system architecture in contrast to the sensor control device 104 of FIG. 1. Unlike the two-piece architecture, for example, a user is not required to open multiple packages and finally assemble the sensor control device 1602 before use. Rather, upon receipt by the user, the sensor control device 1602 is already fully assembled and properly positioned within the sensor applicator 102 (FIG. 1). To use the sensor control device 1602, the user need only open one barrier (e.g., removing the applicator cap) before promptly delivering the sensor control device 1602 to the target monitoring location.

As illustrated, the sensor control device 1602 includes an electronics housing 1604 that is generally disc-shaped and can have a circular cross-section. In other embodiments, however, the electronics housing 1604 can exhibit other cross-sectional shapes, such as ovoid or polygonal, without departing from the scope of the disclosure. The electronics housing 1604 can be configured to house or otherwise contain various electrical components used to operate the sensor control device 1602.

The electronics housing 1604 can include a shell 1606 and a mount 1608 that is matable with the shell 1606. The shell 1606 can be secured to the mount 1608 via a variety of ways, such as a snap fit engagement, an interference fit, sonic (or ultrasonic) welding, using one or more mechanical fasteners (e.g., screws), or any combination thereof. In some embodiments, the interface between the shell 1606 and the mount 1608 can be sealed. In such embodiments, a gasket or other type of seal material can be positioned or applied at or near the outer diameter (periphery) of the shell 1606 and the mount 1608. Securing the shell 1606 to the mount 1608 can compress the seal material and thereby generate a sealed interface. In at least one embodiment, an adhesive can be applied to the outer diameter (periphery) of one or both of the shell 606 and the mount 1608, and the adhesive can not only secure the shell 1606 to the mount 1608 but can also seal the interface.

In embodiments where a sealed interface is created between the shell 1606 and the mount 1608, the interior of the electronics housing 1604 can be effectively isolated from outside contamination between the two components. In such embodiments, if the sensor control device 1602 is assembled in a controlled and sterile environment, there can be no need to sterilize the internal electrical components (e.g., via gaseous chemical sterilization). Rather, the sealed engagement can provide a sufficient sterile barrier for the assembled electronics housing 1604.

The sensor control device 1602 can further include a sensor module 1610 (partially visible in FIG. 16B) and a sharp module 1612 (partially visible). The sensor and sharp modules 1610, 1612 can be interconnectable and coupled to the electronics housing 1604. The sensor module 1610 can be configured to carry and otherwise include a sensor 1614 (FIG. 16B), and the sharp module 1612 can be configured to carry and otherwise include a sharp 1616 (FIG. 16B) used to help deliver the sensor 1614 transcutaneously under a user's skin during application of the sensor control device 1602.

As illustrated in FIG. 16B, corresponding portions of the sensor 1614 and the sharp 1616 extend from the electronics housing 1604 and, more particularly, from the bottom of the mount 1608. The exposed portion of the sensor 1614 can be received within a hollow or recessed portion of the sharp 1616. The remaining portion(s) of the sensor 1614 is/are positioned within the interior of the electronics housing 1604.

An adhesive patch 1618 can be positioned on and otherwise attached to the underside of the mount 1608. Similar to the adhesive patch 108 of FIG. 1, the adhesive patch 1618 can be configured to secure and maintain the sensor control device 1602 in position on the user's skin during operation. In some embodiments, a transfer adhesive 1620 can interpose the adhesive patch 1618 and the bottom of the mount 1608. The transfer adhesive 1620 can help facilitate the assembly process of the sensor control device 1602.

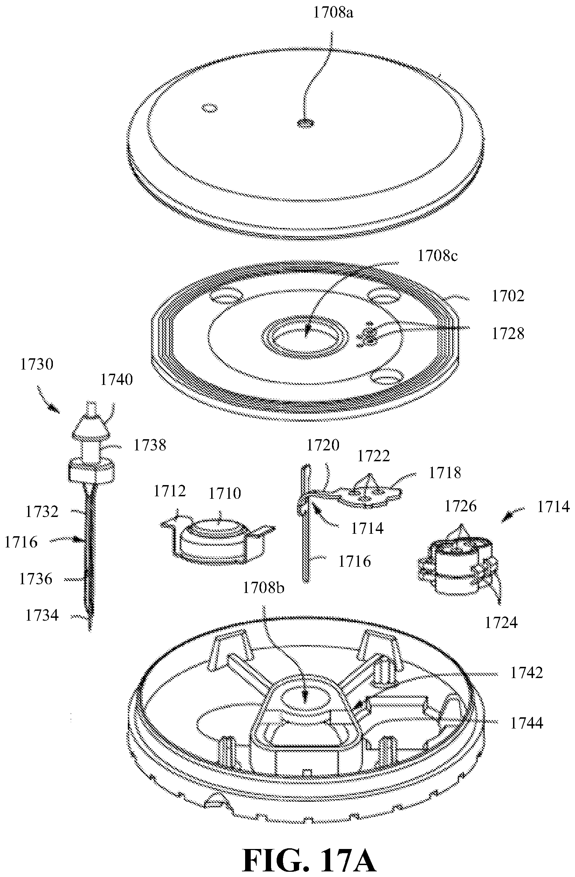

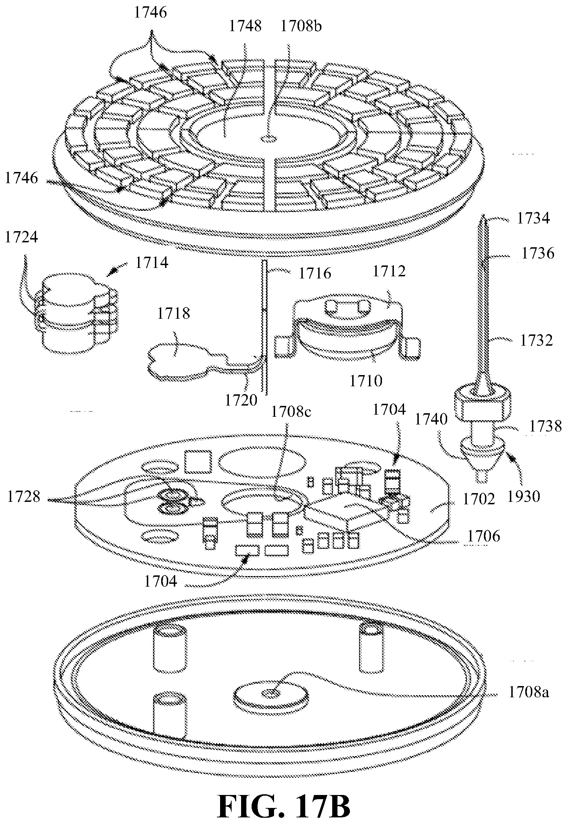

FIGS. 17A and 17B are exploded perspective top and bottom views, respectively, of the sensor control device 1602, according to one or more embodiments. As illustrated, the shell 1606 and the mount 1608 of the electronics housing 1604 operate as opposing clamshell halves that enclose or otherwise substantially encapsulate the various electronic components of the sensor control device 1602.

A printed circuit board (PCB) 1702 can be positioned within the electronics housing 1604. As shown in FIG. 17B, a plurality of electronic modules 1704 can be mounted to the underside of the PCB 1702. Example electronic modules 1704 include, but are not limited to, resistors, transistors, capacitors, inductors, diodes, and switches. A data processing unit 1706 (FIG. 17B) can also be mounted to the PCB 1702 and can comprise, for example, an application specific integrated circuit (ASIC) configured to implement one or more functions or routines associated with operation of the sensor control device 1602. More specifically, the data processing unit 1706 can be configured to perform data processing functions, such as filtering and encoding of data signals, each of which corresponds to a sampled analyte level of the user. The data processing unit 1706 can also include or otherwise communicate with an antenna for communicating with the reader device 106 (FIG. 1).

As illustrated, the shell 1606, the mount 1608, and the PCB 1702 each define corresponding central apertures 1708a, 1708b, 1708c, respectively. When the sensor control device 1602 is assembled, the central apertures 1708a-c coaxially align to receive portions of the sensor and sharp modules 1610, 1612 therethrough.

A battery 1710 and a corresponding battery mount 1712 can also be housed within the electronics housing 1604. The battery 1710 can be configured to power the sensor control device 1602.

The sensor module 1610 can include the sensor 1614 and a connector 1714. The sensor 1614 includes a tail 1716, a flag 1718, and a neck 1720 that interconnects the tail 1716 and the flag 1718. The tail 1716 can be configured to extend through the central aperture 1708b defined in the mount 1608 and extend distally from the underside thereof. The tail 1716 includes an enzyme or other chemistry or biologic and, in some embodiments, a membrane can cover the chemistry. In use, the tail 1716 is transcutaneously received beneath a user's skin, and the chemistry included thereon helps facilitate analyte monitoring in the presence of bodily fluids.

The flag 1718 can comprise a generally planar surface having one or more sensor contacts 1722 (three shown in FIG. 17A) disposed thereon. The flag 1718 can be configured to be received within the connector 1714 where the sensor contact(s) 1722 align with a corresponding number of compliant carbon impregnated polymer modules (not shown) encapsulated within the connector 1714.

The connector 1714 includes one or more hinges 1724 that enables the connector 1714 to pivot between open and closed states. The connector 1714 is depicted in FIGS. 17A and 17B in the closed state, but can transition to the open state to receive the flag 1718 and the compliant carbon impregnated polymer module(s) therein. The compliant carbon impregnated polymer module(s) provide electrical contacts 1726 (three shown in FIG. 17A) configured to provide conductive communication between the sensor 1614 and corresponding circuitry contacts 1728 provided on the PCB 1702. When the sensor module 1610 is properly coupled to the electronics housing 1604, the circuitry contacts 1728 make conductive communication with the electrical contacts 1726 of the connector 1714. The connector 1714 can be made of silicone rubber and can serve as a moisture barrier for the sensor 1614.

The sharp module 1612 includes the sharp 1616 and a sharp hub 1730 that carries the sharp 1616. The sharp 1616 includes an elongate shaft 1732 and a sharp tip 1734 at the distal end of the shaft 1732. The shaft 1732 can be configured to extend through each of the coaxially aligned central apertures 1708a-c and extend distally from the bottom of the mount 1608.

Moreover, the shaft 1732 can include a hollow or recessed portion 1736 that at least partially circumscribes the tail 1716 of the sensor 1614. The sharp tip 1734 can be configured to penetrate the skin while carrying the tail 1716 to put the active chemistry of the tail 1716 into contact with bodily fluids.

The sharp hub 1730 can include a hub small cylinder 1738 and a hub snap pawl 1740, each of which can be configured to help couple the sensor control device 1602 to the sensor applicator 102 (FIG. 1).

Referring specifically to FIG. 17A, in some embodiments the sensor module 1610 can be at least partially received within a sensor mount pocket 1742 included within the electronics housing 1604. In some embodiments, the sensor mount pocket 1742 can comprise a separate structure, but can alternatively form an integral part or extension of the mount 1608. The sensor mount pocket 1742 can be shaped and otherwise configured to receive and seat the sensor 1614 and the connector 1714. As illustrated, the sensor mount pocket 1742 defines an outer periphery 1744 that generally circumscribes the region where the sensor 1614 and the connector 1714 are to be received. In at least one embodiment, the outer periphery 1744 can be sealed to the underside of the PCB 1702 when the electronics housing 1604 is fully assembled. In such embodiments, a gasket (e.g., an O-ring or the like), an adhesive, or another type of seal material can be applied (arranged) at the outer periphery 1744 and can operate to seal the interface between the sensor mount pocket and the PCB 1702.

Sealing the interface between the sensor mount pocket 1742 and the underside of the PCB 1702 can help create or define a sealed zone or region within the electronics housing 1604. The sealed region can prove advantageous in helping to isolate (protect) the tail 1716 of the sensor 1614 from potentially harmful sterilization gases used during gaseous chemical sterilization.

Referring specifically to FIG. 17B, a plurality of channels or grooves 1746 can be provided or otherwise defined on the bottom of the mount 1608. As illustrated, the grooves 1746 can form a plurality of concentric rings in combination with a plurality of radially extending channels. The adhesive patch 1618 (FIGS. 16A and 16B) can be attached to the underside of the mount 1608, and, in some embodiments, the transfer adhesive 1620 (FIGS. 16A and 16B) can interpose the adhesive patch 1618 and the bottom of the mount 1608. The grooves 1746 can prove advantageous in promoting the egress of moisture away from the center of the electronics housing 1604 beneath the adhesive patch 1618.

In some embodiments, a cap post seal interface 1748 can be defined on the bottom of the mount 1608 at the center of the mount 1608. As illustrated, the cap post seal interface 1748 can comprise a substantially flat portion of the bottom of the mount 1608. The second central aperture 1708b is defined at the center of the cap post seal interface 1748 and the grooves 1746 can circumscribe the cap post seal interface 1748. The cap post seal interface 1748 can provide a sealing surface that can help isolate (protect) the tail 1716 of the sensor 1614 from potentially harmful sterilization gases used during gaseous chemical sterilization.

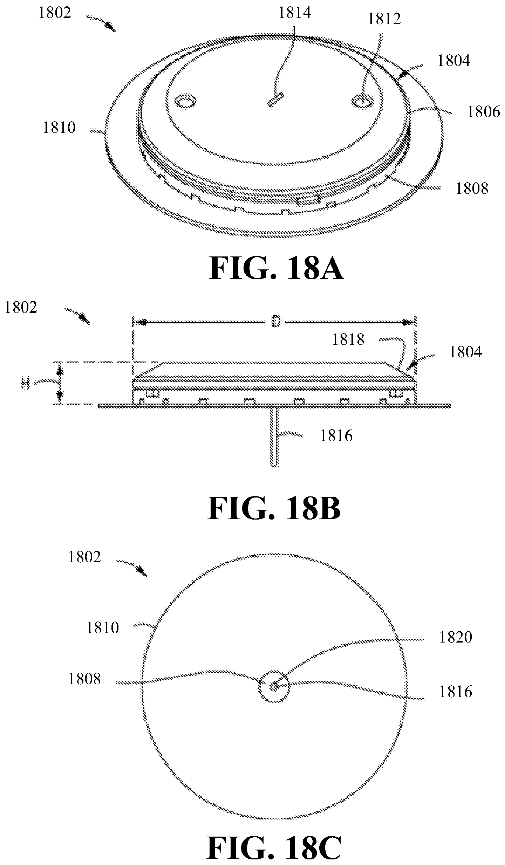

FIGS. 18A-18C are isometric, side, and bottom views, respectively, of an example sensor control device 1802, according to one or more embodiments of the present disclosure. The sensor control device 1802 (alternately referred to as an on-body patch or unit) can be similar in some respects to the sensor control device 104 of FIG. 1 and therefore can be best understood with reference thereto. The sensor control device 1802 can replace the sensor control device 104 of FIG. 1 and, therefore, can be used in conjunction with the sensor applicator 102 (FIG. 1), which delivers the sensor control device 1802 to a target monitoring location on a user's skin. However, in contrast to the sensor control device 104 of FIG. 1, various structural advantages and improvements allow the sensor control device 1802 to be incorporated into a one- piece system architecture.

Unlike the sensor control device 104 of FIG. 1, for example, a user is not required to open multiple packages and finally assemble the sensor control device 1802 prior to delivery to the target monitoring location. Rather, upon receipt by the user, the sensor control device 1802 can already be assembled and properly positioned within the sensor applicator 102. To use the sensor control device 1802, the user need only break one barrier (e.g., the applicator cap) before promptly delivering the sensor control device 1802 to the target monitoring location.

Referring first to FIG. 18A, the sensor control device 1802 comprises an electronics housing 1804 that is generally disc-shaped and can have a generally circular cross- section. In other embodiments, however, the electronics housing 1804 can exhibit other cross- sectional shapes, such as ovoid or polygonal, without departing from the scope of the disclosure. The electronics housing 1804 can include a shell 1806 and a mount 1808 that is matable with the shell 1806. An adhesive patch 1810 can be positioned on and otherwise attached to the underside of the mount 1808. Similar to the adhesive patch 108 of FIG. 1, the adhesive patch 1810 can be configured to secure and maintain the sensor control device 1802 in position on the user's skin during operation.

In some embodiments, the shell 1806 can define a reference feature 1812. As illustrated, the reference feature 1812 can comprise a depression or blind pocket defined in the shell 1806 and extending a short distance into the interior of the electronics housing 3704. The reference feature 1812 can operate as a "datum c" feature configured to help facilitate control of the sensor control device 1802 in at least one degree of freedom during factory assembly. In contrast, prior sensor control devices (e.g., the sensor control device 104 of FIG. 1) typically include a tab extending radially from the side of the shell. The tab is used as an in-process clocking datum, but must be removed at the end of fabrication, and followed by an inspection of the shell where the tab once existed, which adds complexity to the prior fabrication process.

The shell 1806 can also define a central aperture 1814 sized to receive a sharp (not shown) that is extendable through the center of the electronics housing 1804.

FIG. 18B depicts a portion of a sensor 1816 extending from the electronics housing 1804. The remaining portion(s) of the sensor 1816 is/are positioned within the interior of the electronics housing 1804. Similar to the sensor 110 of FIG. 1, the exposed portion of the sensor 1816 is configured to be transcutaneously positioned under the user's skin during use. The exposed portion of the sensor 1816 can include an enzyme or other chemistry or biologic and, in some embodiments, a membrane can cover the chemistry.

The sensor control device 1802 provides structural improvements that result in a height H and a diameter D that can be less than prior sensor control devices (e.g., the sensor control device 104 of FIG. 1). In at least one embodiment, for example, the height H can be, which is about 1 mm or more less than the height of prior sensor control devices, and the diameter D can be, which is about 2 mm or more less than the diameter of prior sensor control devices. In certain other embodiments the height H and diameter D can be any other suitable value, such as between 1 mm - 5 mm or between .1 mm - 10 mm less than the height or diameter of the prior sensor device.

Moreover, the structural improvements of the sensor control device 1802 allows the shell 1806 to provide or otherwise define a chamfered or angled outer periphery 1818. In contrast, prior sensor control devices commonly require a rounded or outwardly arcuate outer periphery to accommodate internal components. The reduced height H, the reduced diameter D, and the angled outer periphery 1818 can each prove advantageous in providing a sensor control device 1802 that is thinner, smaller, and less prone to being prematurely detached by catching on sharp corners or the like while attached to the user's skin.

FIG. 18C depicts a central aperture 1820 defined in the underside of the mount 1808. The central aperture 1820 can be sized to receive a combination sharp (not shown) and sensor 1816, where the sensor 1816 is received within a hollow or recessed portion of the sharp. When the electronics housing 1804 is assembled, the central aperture 1820 coaxially aligns with the central aperture 1814 (FIG. 18A) of the shell 1806 (FIG. 18A) and the sharp penetrates the electronics housing by extending simultaneously through each central aperture 1814, 1820.

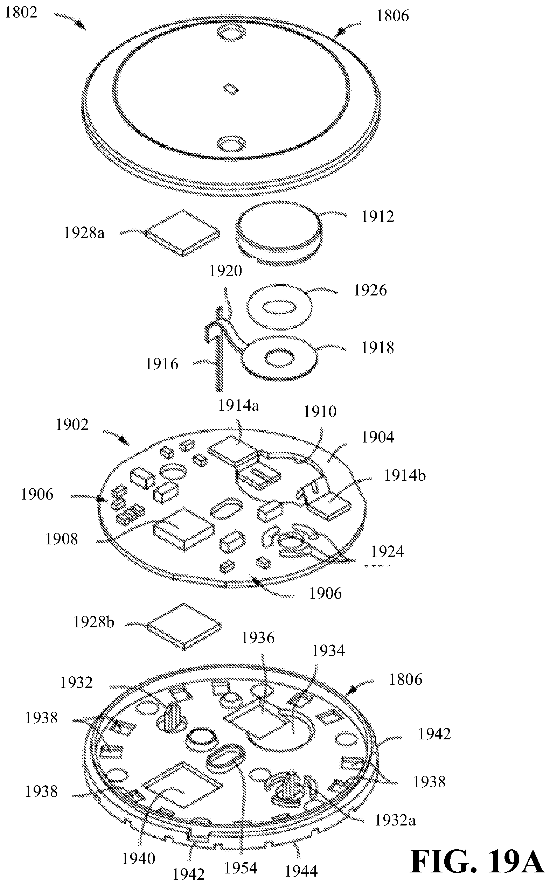

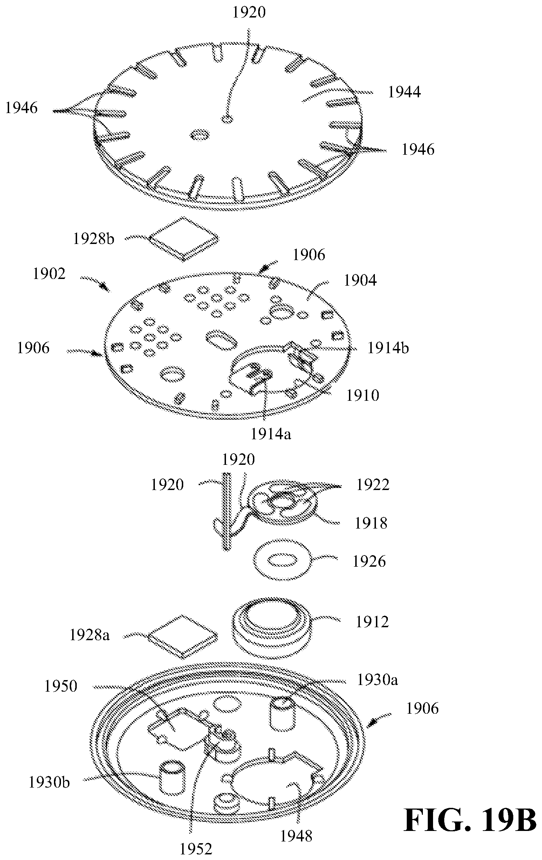

FIGS. 19A and 19B are exploded top and bottom views, respectively, of the sensor control device 1802, according to one or more embodiments. The shell 1806 and the mount 1808 operate as opposing clamshell halves that enclose or otherwise substantially encapsulate the various electronic components of the sensor control device 1802. As illustrated, the sensor control device 1802 can include a printed circuit board assembly (PCBA) 1902 that includes a printed circuit board (PCB) 1904 having a plurality of electronic modules 1906 coupled thereto. Example electronic modules 1906 include, but are not limited to, resistors, transistors, capacitors, inductors, diodes, and switches. Prior sensor control devices commonly stack PCB components on only one side of the PCB. In contrast, the PCB components 1906 in the sensor control device 1802 can be dispersed about the surface area of both sides (i.e., top and bottom surfaces) of the PCB 1904.

Besides the electronic modules 1906, the PCBA 1902 can also include a data processing unit 1908 mounted to the PCB 1904. The data processing unit 1908 can comprise, for example, an application specific integrated circuit (ASIC) configured to implement one or more functions or routines associated with operation of the sensor control device 1802. More specifically, the data processing unit 1908 can be configured to perform data processing functions, where such functions can include but are not limited to, filtering and encoding of data signals, each of which corresponds to a sampled analyte level of the user. The data processing unit 1908 can also include or otherwise communicate with an antenna for communicating with the reader device 106 (FIG. 1).

A battery aperture 1910 can be defined in the PCB 1904 and sized to receive and seat a battery 1912 configured to power the sensor control device 1802. An axial battery contact 1914a and a radial battery contact 1914b can be coupled to the PCB 1904 and extend into the battery aperture 1910 to facilitate transmission of electrical power from the battery 1912 to the PCB 1904. As their names suggest, the axial battery contact 1914a can be configured to provide an axial contact for the battery 1912, while the radial battery contact 1914b can provide a radial contact for the battery 1912. Locating the battery 1912 within the battery aperture 1910 with the battery contacts 1914a,b helps reduce the height H (FIG. 18B) of the sensor control device 1802, which allows the PCB 1904 to be located centrally and its components to be dispersed on both sides (i.e., top and bottom surfaces). This also helps facilitate the chamfer 1818 (FIG. 18B) provided on the electronics housing 1804.

The sensor 1916 can be centrally located relative to the PCB 1904 and include a tail 1916, a flag 1918, and a neck 1920 that interconnects the tail 1916 and the flag 1918. The tail 1916 can be configured to extend through the central aperture 1820 of the mount 1808 to be transcutaneously received beneath a user's skin. Moreover, the tail 1916 can have an enzyme or other chemistry included thereon to help facilitate analyte monitoring.

The flag 1918 can include a generally planar surface having one or more sensor contacts 1922 (three shown in FIG. 19B) arranged thereon. The sensor contact(s) 1922 can be configured to align with and engage a corresponding one or more circuitry contacts 1924 (three shown in FIG. 19A) provided on the PCB 1904. In some embodiments, the sensor contact(s) 1922 can comprise a carbon impregnated polymer printed or otherwise digitally applied to the flag 1918. Prior sensor control devices typically include a connector made of silicone rubber that encapsulates one or more compliant carbon impregnated polymer modules that serve as electrical conductive contacts between the sensor and the PCB. In contrast, the presently disclosed sensor contacts(s) 1922 provide a direct connection between the sensor 1816 and the PCB 1904 connection, which eliminates the need for the prior art connector and advantageously reduces the height H (FIG. 18B). Moreover, eliminating the compliant carbon impregnated polymer modules eliminates a significant circuit resistance and therefor improves circuit conductivity.

The sensor control device 1802 can further include a compliant member 1926, which can be arranged to interpose the flag 1918 and the inner surface of the shell 1806. More specifically, when the shell 1806 and the mount 1808 are assembled to one another, the compliant member 1926 can be configured to provide a passive biasing load against the flag 1918 that forces the sensor contact(s) 1922 into continuous engagement with the corresponding circuitry contact(s) 1924. In the illustrated embodiment, the compliant member 1926 is an elastomeric O-ring, but could alternatively comprise any other type of biasing device or mechanism, such as a compression spring or the like, without departing from the scope of the disclosure.

The sensor control device 1802 can further include one or more electromagnetic shields, shown as a first shield 1928a and a second shield 1928b. The shields 1928a,b can be arranged between the shell 1806 and the mount 1808; i.e., within the electronics housing 1804. In the illustrated embodiment, the first shield 1928a is arranged above the PCB 1904 such that it faces the top surface of the PCB 1904, and the second shield 1928b is arranged below the PCB 1904 such that it faces the bottom surface of the PCB 1904.

The shields 1928a,b can be configured to protect sensitive electronic components from radiation while the sensor control device 1802 is subjected to radiation sterilization. More specifically, at least one of the shields 1928a,b can be positioned to interpose the data processing unit 1908 and a radiation source, such as an e-beam electron accelerator. In some embodiments, for example, at least one of the shields 1928a,b can be positioned adjacent to and otherwise aligned with the data processing unit 1908 and the radiation source to block or mitigate radiation absorbed dose that might otherwise damage the sensitive electronic circuitry of the data processing unit 1908.

In the illustrated embodiment, the data processing unit 1908 interposes the first and second shields 1928a,b such that the first and second shields 1928a,b essentially bookend the data processing unit 1908 in the axial direction. In at least one embodiment, however, only one of the shields 1928a,b can be necessary to properly protect the data processing unit 1908 during radiation sterilization. For example, if the sensor control device 1802 is subjected to radiation sterilization directed toward the bottom of the mount 1808, only the second shield 1928b can be needed to interpose the data processing unit 1908 and the radiation source, and the first shield 1928a can be omitted. Alternatively, if the sensor control device 1802 is subjected to radiation sterilization directed toward the top of the shell 1806, only the first shield 1928a can be needed to interpose the data processing unit 1908 and the radiation source, and the second shield 1928b can be omitted. In other embodiments, however, both shields 1928a,b can be employed, without departing from the scope of the disclosure.

The shields 1928a,b can be made of any material capable of attenuating (or substantially attenuating) the transmission of radiation. Suitable materials for the shields 1928a,b include, but are not limited to, lead, tungsten, iron-based metals (e.g., stainless steel), copper, tantalum, tungsten, osmium, aluminum, carbon, or any combination thereof. Suitable metals for the shields 1928a,b can be corrosion-resistant, austenitic, and any non-magnetic metal with a density ranging between about 2 grams per cubic centimeter (g/cc) and about 23 g/cc. The shields 1928a,b can be fabricated via a variety of manufacturing techniques including, but not limited to, stamping, casting, injection molding, sintering, two-shot molding, or any combination thereof.

In other embodiments, however, the shields 1928a,b can comprise a metal-filled thermoplastic polymer such as, but not limited to, polyamide, polycarbonate, or polystyrene. In such embodiments, the shields 1928a,b can be fabricated by mixing the shielding material in an adhesive matrix and dispensing the combination onto shaped components or otherwise directly onto the data processing unit 1908. Moreover, in such embodiments, the shields 1928a,b can comprise an enclosure that encapsulates (or substantially encapsulates) the data processing unit 1908. In such embodiments, the shields 1928a,b can comprise a metal-filled thermoplastic polymer, as mentioned above, or can alternatively be made of any of the materials mentioned herein that are capable of attenuating (or substantially attenuating) the transmission of radiation.

The shell 1806 can provide or otherwise define a first clocking receptacle 1930a (FIG. 19B) and a second clocking receptacle 1930b (FIG. 19B), and the mount 1808 can provide or otherwise define a first clocking post 1932a (FIG. 19A) and a second clocking post 1932b (FIG. 19A). Mating the first and second clocking receptacles 1930a,b with the first and second clocking posts 1932a,b, respectively, will properly align the shell 1806 to the mount 1808.

Referring specifically to FIG. 18A, the inner surface of the mount 1808 can provide or otherwise define a plurality of pockets or depressions configured to accommodate various component parts of the sensor control device 1802 when the shell 1806 is mated to the mount 1808. For example, the inner surface of the mount 1808 can define a battery locator 1934 configured to accommodate a portion of the battery 1912 when the sensor control device 1802 is assembled. An adjacent contact pocket 1936 can be configured to accommodate a portion of the axial contact 1914a.

Moreover, a plurality of module pockets 1938 can be defined in the inner surface of the mount 1808 to accommodate the various electronic modules 1906 arranged on the bottom of the PCB 1904. Furthermore, a shield locator 1940 can be defined in the inner surface of the mount 1808 to accommodate at least a portion of the second shield 1928b when the sensor control device 1802 is assembled. The battery locator 1934, the contact pocket 1936, the module pockets 1938, and the shield locator 1940 all extend a short distance into the inner surface of the mount 1808 and, as a result, the overall height H (FIG. 18B) of the sensor control device 1802 can be reduced as compared to prior sensor control devices. The module pockets 1938 can also help minimize the diameter of the PCB 1904 by allowing PCB components to be arranged on both sides (i.e., top and bottom surfaces).

Still referring to FIG. 19A, the mount 1808 can further include a plurality of carrier grip features 1942 (two shown) defined about the outer periphery of the mount 1808. The carrier grip features 1942 are axially offset from the bottom 1944 of the mount 1808, where a transfer adhesive (not shown) can be applied during assembly. In contrast to prior sensor control devices, which commonly include conical carrier grip features that intersect with the bottom of the mount, the presently disclosed carrier grip features 1942 are offset from the plane (i.e., the bottom 1944) where the transfer adhesive is applied. This can prove advantageous in helping ensure that the delivery system does not inadvertently stick to the transfer adhesive during assembly. Moreover, the presently disclosed carrier grip features 1942 eliminate the need for a scalloped transfer adhesive, which simplifies the manufacture of the transfer adhesive and eliminates the need to accurately clock the transfer adhesive relative to the mount 1808. This also increases the bond area and, therefore, the bond strength.

Referring to FIG. 19B, the bottom 1944 of the mount 1808 can provide or otherwise define a plurality of grooves 1946, which can be defined at or near the outer periphery of the mount 1808 and equidistantly spaced from each other. A transfer adhesive (not shown) can be coupled to the bottom 1944 and the grooves 1946 can be configured to help convey (transfer) moisture away from the sensor control device 1802 and toward the periphery of the mount 1808 during use. In some embodiments, the spacing of the grooves 1946 can interpose the module pockets 1938 (FIG. 19A) defined on the opposing side (inner surface) of the mount 1808. As will be appreciated, alternating the position of the grooves 1946 and the module pockets 1938 ensures that the opposing features on either side of the mount 1808 do not extend into each other. This can help maximize usage of the material for the mount 1808 and thereby help maintain a minimal height H (FIG. 18B) of the sensor control device 1802. The module pockets 1938 can also significantly reduce mold sink, and improve the flatness of the bottom 1944 that the transfer adhesive bonds to.

Still referring to FIG. 19B, the inner surface of the shell 1806 can also provide or otherwise define a plurality of pockets or depressions configured to accommodate various component parts of the sensor control device 1802 when the shell 1806 is mated to the mount 1808. For example, the inner surface of the shell 1806 can define an opposing battery locator 1948 arrangeable opposite the battery locator 1934 (FIG. 19A) of the mount 1808 and configured to accommodate a portion of the battery 1912 when the sensor control device 1802 is assembled. Moreover, a shield locator 1950 can be defined in the inner surface of the shell 1806 to accommodate at least a portion of the first shield 1928a when the sensor control device 1802 is assembled. The opposing battery locator 1948 and the shield locator 1950 extend a short distance into the inner surface of the shell 1806, which helps reduce the overall height H (FIG. 18B) of the sensor control device 1802.

A sharp and sensor locator 1952 can also be provided by or otherwise defined on the inner surface of the shell 1806. The sharp and sensor locator 1952 can be configured to receive both the sharp (not shown) and a portion of the sensor 1816. Moreover, the sharp and sensor locator 1952 can be configured to align and/or mate with a corresponding sharp and sensor locator provided on the inner surface of the mount 1808.

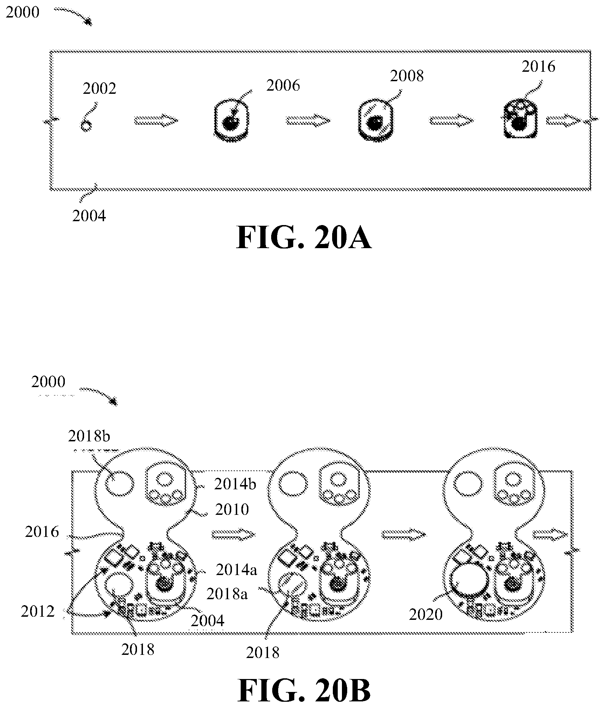

FIGS. 20A and 20B depict fabrication of the sensor control device according to certain embodiments. In a first step of the process 2000, a hole 2002 can be punched or otherwise formed in the base substrate 2004, which can comprise a sheet of material that can eventually form the base or lower cover 2008 of the sensor control device. The base substrate 2004 can comprise a belt or thin film made of a variety of different materials including, but not limited to, a plastic, a metal, a composite material, or any combination thereof. In at least one embodiment, the base substrate 2004 can comprise a laminated aluminum foil having a polyester film on one side (e.g., the bottom side), and a polyolefin heat seal layer on the opposing side (e.g., the top side).

In a second step of the process 2000, a sensor holder can be coupled to the base substrate 2004. The sensor holder can be the same as or similar to either of the sensor holders. Accordingly, the sensor holder can define a channel 2006 sized to receive the tail of the sensor. In some embodiments, the sensor holder can be ultrasonically welded or heat-sealed to the base substrate, thus resulting in a sealed and watertight engagement. In at least one embodiment, however, the base substrate can comprise or otherwise include an adhesive substrate on the top side to secure and seal the sensor holder in place.

In a third step of the process 2000, a first adhesive substrate 2008 can be attached to the top of the sensor holder. The first adhesive substrate 2008 can be similar to any known adhesive substrates, and can thus comprise a pressure-adhesive tape that forms a bond when pressure is applied. In at least one embodiment, the first adhesive substrate 2008 can comprise double- sided polyolefin foam tape and can be pressure sensitive on both sides.

In a fourth step of the process 2000, the sensor 2016 can be secured to the sensor holder using the first adhesive substrate 2008. More specifically, the tail can be extended through the channel 2006 and the flag can be bent generally orthogonal to the tail 10314 and coupled to the underlying first adhesive substrate 2008.

Referring now to FIG. 20B, in a fifth step of the process 2000, a printed circuit board (PCB) 2010 can be positioned on the base substrate 2004 and about the sensor holder. The PCB 2010 can include a plurality of electronic modules 2012 mounted thereto. The electronic modules 2012 can include at least one of a Bluetooth antenna and a near field communication (NFC) antenna. As illustrated, the PCB 2010 can define two opposing lobes 2014a and 2014b interconnected by a neck portion 2016. Opposing battery contacts 2018a and 2018b can be provided on the opposing lobes 2014a,b to facilitate electrical communication with a battery 2020.

In a sixth step of the process 2000, a second adhesive substrate 2008b can be applied to the first battery contact 2018a in preparation for receiving the battery 2020 in an adjacent seventh step of the process 2000. The second adhesive substrate can comprise a pressure-adhesive tape used to couple the battery 2020 to the first battery contact 2018a. The second adhesive substrate, however, can also comprise a Z-axis anisotropic (or conductive) pressure-adhesive tape that also facilitates electrical communication (i.e., transfer of electrical power) between the battery 2020 and the first battery contact 2018a.

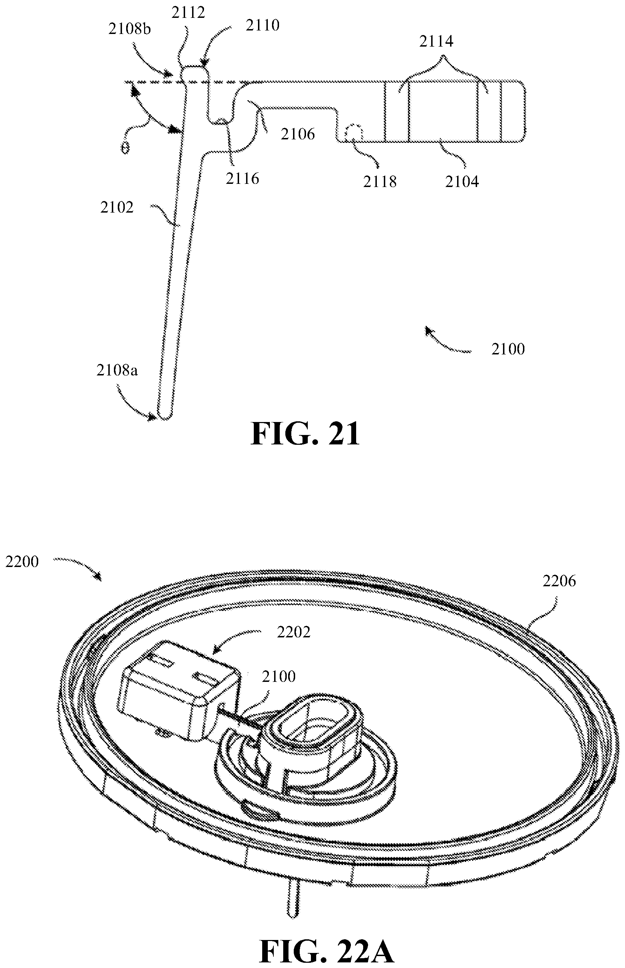

FIG. 21 illustrates a side view of an example sensor 2100, according to one or more embodiments of the disclosure. The sensor 2100 can be similar in some respects to any of the sensors described herein and, therefore, can be used in an analyte monitoring system to detect specific analyte concentrations. As illustrated, the sensor 2100 can include a tail 2102, a flag 2104, and a neck 2106 that interconnects the tail 2102 and the flag 2104. The tail 2102 includes an enzyme or other chemistry or biologic and, in some embodiments, a membrane can cover the chemistry. In use, the tail 2102 can be transcutaneously received beneath a user's skin, and the chemistry included thereon helps facilitate analyte monitoring in the presence of bodily fluids.

The tail 2102 can be received within a hollow or recessed portion of a sharp (not shown) to at least partially circumscribe the tail 2102 of the sensor 2100. As illustrated, the tail 2102 can extend at an angle Q offset from horizontal. In some embodiments, the angle Q can be about 85°. Accordingly, in contrast to other sensor tails, the tail 2102 cannot extend perpendicularly from the flag 2104, but instead offset at an angle from perpendicular. This can prove advantageous in helping maintain the tail 2102 within the keep the recessed portion of the sharp.

The tail 2102 can include a first or bottom end 2108a and a second or top end 2108b opposite the top end 2108a. A tower 2110 can be provided at or near the top end 2108b and can extend vertically upward from the location where the neck 2106 interconnects the tail 2102 to the flag 2104. During operation, if the sharp moves laterally, the tower 2110 will help picot the tail 2102 toward the sharp and otherwise stay within the recessed portion of the sharp. Moreover, in some embodiments, the tower 2110 can provide or otherwise define a protrusion 2112 that extends laterally therefrom. When the sensor 2100 is mated with the sharp, and the tail 2102 extends within the recessed portion of the sharp, the protrusion 2112 can engage the inner surface of the recessed portion. In operation, the protrusion 2112 can help keep the tail 2102 within the recessed portion.

The flag 2104 can include a generally planar surface having one or more sensor contacts 2114 arranged thereon. The sensor contact(s) 2114 can be configured to align with a corresponding number of compliant carbon impregnated polymer modules encapsulated within a connector.

In some embodiments, as illustrated, the neck 2106 can provide or otherwise define a dip or bend 2116 extending between the flag 2104 and the tail 2102. The bend 2116 can prove advantageous in adding flexibility to the sensor 2100 and helping prevent bending of the neck 2106.

In some embodiments, a notch 2118 (shown in dashed lines) can optionally be defined in the flag near the neck 2106. The notch 2118 can add flexibility and tolerance to the sensor 2100 as the sensor 2100 is mounted to the mount. More specifically, the notch 2118 can help take up interference forces that can occur as the sensor 2100 is mounted within the mount.

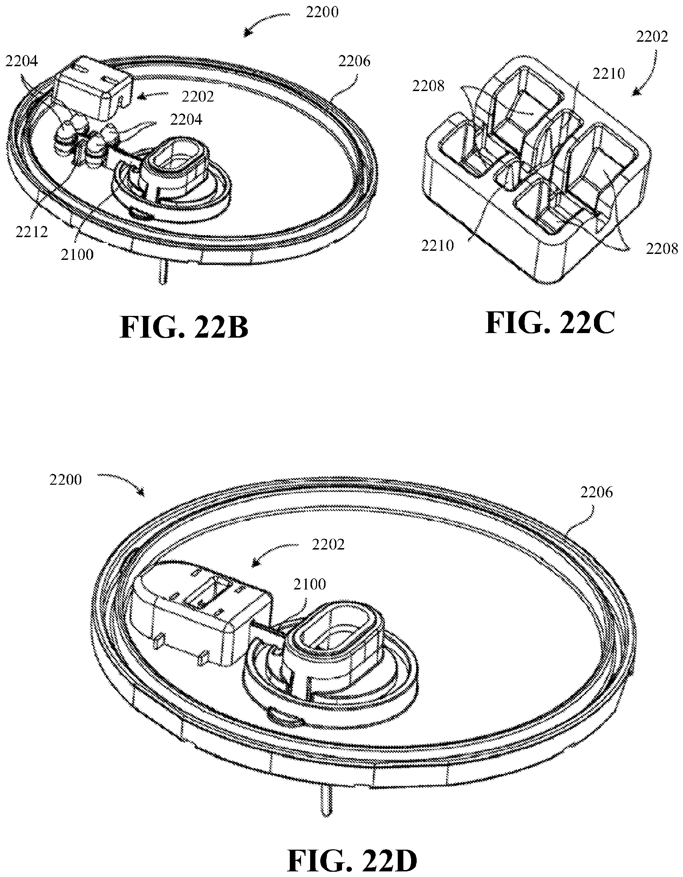

FIGS. 22A and 22B are isometric and partially exploded isometric views of an example connector assembly 2200, according to one or more embodiments. As illustrated, the connector assembly 2200 can include a connector 2202. The connector 2202 can include an injection molded part used to help secure one or more compliant carbon impregnated polymer modules 2204 (four shown in FIG. 22B) to a mount 2206. More specifically, the connector 2202 can help secure the modules 2204 in place adjacent the sensor 2100 and in contact with the sensor contacts 2114 (FIG. 21) provided on the flag 2104 (FIG. 21). The modules 2204 can be made of a conductive material to provide conductive communication between the sensor 2100 and corresponding circuitry contacts (not shown) provided within the mount 2206.

As seen in FIG. 22C, connector 2202 can define pockets 2208 sized to receive the modules 2204. Moreover, in some non-limiting embodiments, connector 2202 can further define one or more depressions 2210 configured to mate with one or more corresponding flanges 2212 (FIG. 22B) on mount 2206. Mating the depressions 2210 with the flanges 2212 can secure connector 2202 to mount 2206 via an interference fit or the like. In other embodiments, connector 2202 can be secured to mount 2206 using an adhesive or via sonic welding.

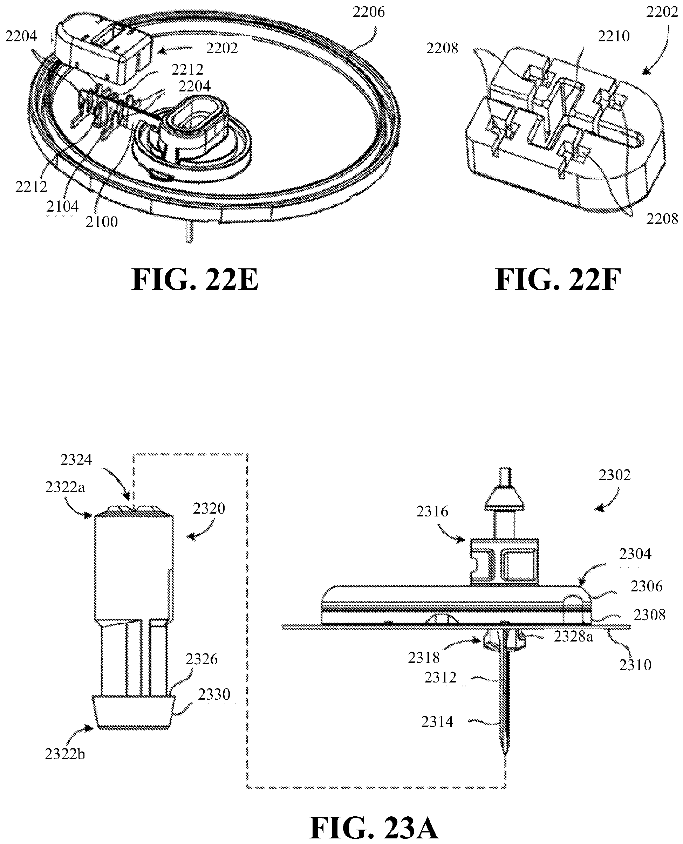

FIGS. 22D and 22E illustrate isometric and partially exploded isometric views of another example connector assembly 2200, according to one or more embodiments. As illustrated, the connector assembly 2200 can include a connector 2202, and FIG. 22F is an isometric bottom view of the connector 2202. The connector 2202 can comprise an injection molded part, a compliant carbon impregnated polymer, a silicon or doped silicon, or a Molex connector, used to help keep one or more compliant metal contacts 2204 (four shown in FIG. 22E) secured against sensor 2100 on a mount 2206. More specifically, connector 2202 can help secure the contacts 2204 in place adjacent sensor 2100 and in contact with the sensor contacts 2114 (FIG. 21) provided on the flag 2104. In other non-limiting embodiments, connector 2202 can comprise any other material known in the art. Contacts 2204 can be made of a stamped conductive material that provides conductive communication between sensor 2100 and corresponding circuitry contacts (not shown) provided within mount 2206. In some embodiments, for example, contacts 2204 can be soldered to a PCB (not shown) arranged within the mount 2206.

As best seen in FIG. 22F, connector 2202 can define pockets 2208 sized to receive contacts 2204. Moreover, in some embodiments, the connector 2202 can further define one or more depressions 2210 configured to mate with one or more corresponding flanges 2212 on the mount 2206. Mating the depressions 2210 with flanges 2212 can help secure connector 2202 to mount 2206 via an interference fit or the like. In other embodiments, connector 2202 can be secured to mount 2206 using an adhesive or via sonic welding.

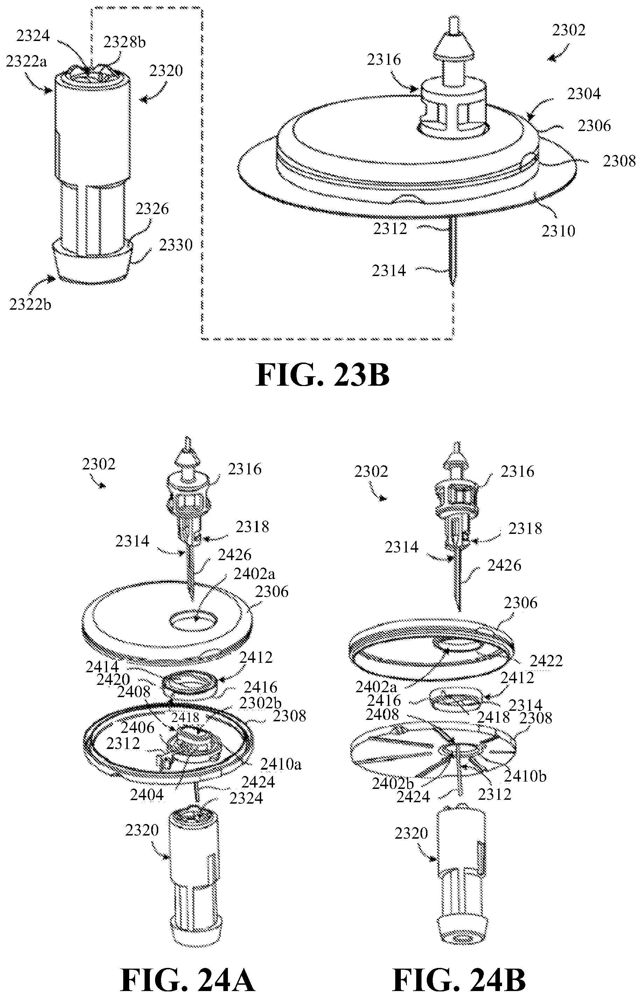

FIGS. 23A and 23B illustrate side and isometric views, respectively, of an example sensor control device 2302, according to one or more embodiments of the present disclosure. The sensor control device 2302 can be similar in some respects to the sensor control device 102 of FIG. 1 and therefore can be best understood with reference thereto. Moreover, the sensor control device 2302 can replace the sensor control device 104 of FIG. 1 and, therefore, can be used in conjunction with the sensor applicator 102 of FIG. 1, which can deliver the sensor control device 2302 to a target monitoring location on a user's skin.

As illustrated, the sensor control device 2302 includes an electronics housing 2304, which can be generally disc-shaped and have a circular cross-section. In other embodiments, however, the electronics housing 2304 can exhibit other cross-sectional shapes, such as ovoid, oval, or polygonal, without departing from the scope of the disclosure. The electronics housing 2304 includes a shell 2306 and a mount 2308 that is matable with the shell 2306. The shell 2306 can be secured to the mount 2308 via a variety of ways, such as a snap fit engagement, an interference fit, sonic welding, laser welding, one or more mechanical fasteners (e.g., screws), a gasket, an adhesive, or any combination thereof. In some non-limiting embodiments, shell 2306 can be secured to the mount 2308 such that a sealed interface is generated therebetween. An adhesive patch 2310 can be positioned on and otherwise attached to the underside of the mount 2308. Similar to the adhesive patch 108 of FIG. 1, the adhesive patch 2310 can be configured to secure and maintain the sensor control device 2302 in position on the user's skin during operation.

The sensor control device 2302 can further include a sensor 2312 and a sharp 2314 used to help deliver the sensor 2312 transcutaneously under a user's skin during application of the sensor control device 2302. Corresponding portions of the sensor 2312 and the sharp 2314 extend distally from the bottom of the electronics housing 2304 (e.g., the mount 2308). A sharp hub 2316 can be overmolded onto the sharp 2314 and configured to secure and carry the sharp 2314. As shown in FIG. 23A, the sharp hub 2316 can include or otherwise define a mating member 2318. In assembling sharp 2314 to sensor control device 2302, sharp 2314 can be advanced axially through the electronics housing 2304 until the sharp hub 2316 engages an upper surface of electronics housing 2304 or an internal component thereof and the mating member 2318 extends distally from the bottom of the mount 2308. As described herein below, in at least one embodiment, the sharp hub 2316 can sealingly engage an upper portion of a seal overmolded onto the mount 2308. As the sharp 2314 penetrates the electronics housing 2304, the exposed portion of the sensor 2312 can be received within a hollow or recessed (arcuate) portion of the sharp 2314. The remaining portion of the sensor 2312 is arranged within the interior of the electronics housing 2304.

Sensor control device 2302 can further include a sensor cap 2320, shown detached from the electronics housing 2304 in FIGS. 23A-23B. Sensor cap 2320 can help provide a sealed barrier that surrounds and protects exposed portions of the sensor 2312 and the sharp 2314. As illustrated, the sensor cap 2320 can comprise a generally cylindrical body having a first end 2322a and a second end 2322b opposite the first end 2322a. The first end 2322a can be open to provide access into an inner chamber 2324 defined within the body. In contrast, the second end 2322b can be closed and can provide or otherwise define an engagement feature 2326. As described in more detail below, the engagement feature 2326 can help mate the sensor cap 2320 to an applicator cap of a sensor applicator (e.g., the sensor applicator 102 of FIG. 1), and can help remove the sensor cap 2320 from the sensor control device 2302 upon removing the sensor cap from the sensor applicator.

The sensor cap 2320 can be removably coupled to the electronics housing 2304 at or near the bottom of the mount 2308. More specifically, the sensor cap 2320 can be removably coupled to the mating member 2318, which extends distally from the bottom of the mount 2308. In at least one embodiment, for example, the mating member 2318 can define a set of external threads 2328a (FIG. 23A) matable with a set of internal threads 2328b (FIG. 23B) defined within the inner chamber 2324 of the sensor cap 2320. In some embodiments, the external and internal threads 2328a,b can comprise a flat thread design (e.g., lack of helical curvature), but can alternatively comprise a helical threaded engagement. Accordingly, in at least one embodiment, the sensor cap 2320 can be threadably coupled to the sensor control device 2302 at the mating member 2318 of the sharp hub 2316. In other embodiments, the sensor cap 2320 can be removably coupled to the mating member 2318 via other types of engagements including, but not limited to, an interference or friction fit, or a frangible member or substance (e.g., wax, an adhesive, etc.) that can be broken with minimal separation force (e.g., axial or rotational force).

In some embodiments, the sensor cap 2320 can comprise a monolithic (singular) structure extending between the first and second ends 2322a,b. In other embodiments, however, the sensor cap 2320 can comprise two or more component parts. In the illustrated embodiment, for example, the body of the sensor cap 2320 can include a desiccant cap 2330 arranged at the second end 9l22b. The desiccant cap 2330 can house or comprise a desiccant to help maintain preferred humidity levels within the inner chamber 2324. Moreover, the desiccant cap 2330 can also define or otherwise provide the engagement feature 2326 of the sensor cap 2320. In at least one non-limiting embodiment, the desiccant cap 2330 can comprise an elastomeric plug inserted into the bottom end of the sensor cap 2320.

FIGS. 24A and 24B illustrate exploded, isometric top and bottom views, respectively, of the sensor control device 2302, according to certain embodiments. Shell 2306 and mount 2308 operate as opposing clamshell halves that enclose or otherwise substantially encapsulate various electronic components (not shown) of the sensor control device 2302. Example electronic components that can be arranged between shell 2306 and mount 2308 include, but are not limited to, a battery, resistors, transistors, capacitors, inductors, diodes, and switches.

The shell 2306 can define a first aperture 2402a and the mount 2308 can define a second aperture 2402b, and the apertures 2402a, b can align when the shell 2306 is properly mounted to the mount 2308. As best seen in FIG. 24A, the mount 2308 can provide or otherwise define a pedestal 2404 that protrudes from the inner surface of the mount 2308 at the second aperture 2402b. The pedestal 2404 can define at least a portion of the second aperture 2402b. Moreover, a channel 2406 can be defined on the inner surface of the mount 2308 and can circumscribe the pedestal 2402. In the illustrated embodiment, the channel 2406 is circular in shape, but could alternatively be another shape, such as oval, ovoid, or polygonal.

The mount 2308 can comprise a molded part made of a rigid material, such as plastic or metal. In some embodiments, a seal 2408 can be overmolded onto the mount 2308 and can be made of an elastomer, rubber, a polymer, or another pliable material suitable for facilitating a sealed interface. In embodiments where the mount 2308 is made of a plastic, the mount 2308 can be molded in a first "shot" of injection molding, and the seal 2408 can be overmolded onto the mount 2308 in a second "shot" of injection molding. Accordingly, the mount 2308 can be referred to or otherwise characterized as a "two-shot mount."

In the illustrated embodiment, the seal 2408 can be overmolded onto the mount 2308 at the pedestal 2404 and also on the bottom of the mount 2308. More specifically, the seal 2408 can define or otherwise provide a first seal element 24l0a overmolded onto the pedestal 2404, and a second seal element 2410b (FIG. 24B) interconnected to or with first seal element 24l0a and overmolded onto mount 2308 at the bottom of mount 2308. In some embodiments, one or both of seal elements 24l0a,b can help form corresponding portions (sections) of the second aperture 2402b. While seal 2408 is described herein as being overmolded onto mount 2308, it is also contemplated herein that one or both of seal elements 24l0a,b can comprise an elastomeric component part independent of mount 2408, such as an O-ring or a gasket.

The sensor control device 2302 can further include a collar 2412, which can be a generally annular structure that defines a central aperture 2414. The central aperture 2414 can be sized to receive the first seal element 24l0a and can align with both the first and second apertures 2402a, b when the sensor control device 2302 is properly assembled. The shape of the central aperture 2414 can generally match the shape of the second aperture 2402b and the first seal element 24l0a.

In some embodiments, the collar 2412 can define or otherwise provide an annular lip 2416 on its bottom surface. The annular lip 2416 can be sized and otherwise configured to mate with or be received into the channel 2406 defined on the inner surface of the mount 2308. In some embodiments, a groove 2418 can be defined on the annular lip 2416 and can be configured to accommodate or otherwise receive a portion of the sensor 2312 extending laterally within the mount 2308. In some embodiments, the collar 2412 can further define or otherwise provide a collar channel 2420 (FIG. 24A) on its upper surface sized to receive and otherwise mate with an annular ridge 2422 (FIG. 24B) defined on the inner surface of the shell 2306 when the sensor control device 2302 is properly assembled.

The sensor 2312 can include a tail 2424 that extends through the second aperture 2402b defined in the mount 2308 to be transcutaneously received beneath a user's skin. The tail 2424 can have an enzyme or other chemistry included thereon to help facilitate analyte monitoring. The sharp 2314 can include a sharp tip 2426 extendable through the first aperture 2402a defined by the shell 2306. As the sharp tip 2426 penetrates the electronics housing 2304, the tail 2424 of the sensor 2312 can be received within a hollow or recessed portion of the sharp tip 2426. The sharp tip 2426 can be configured to penetrate the skin while carrying the tail 2424 to put the active chemistry of the tail 2424 into contact with bodily fluids.

The sensor control device 2302 can provide a sealed subassembly that includes, among other component parts, portions of the shell 2306, the sensor 2312, the sharp 2314, the seal 2408, the collar 2412, and the sensor cap 2320. The sealed subassembly can help isolate the sensor 2312 and the sharp 2314 within the inner chamber 2324 (FIG. 24A) of the sensor cap 2320. In assembling the sealed subassembly, the sharp tip 2426 is advanced through the electronics housing 2304 until the sharp hub 2316 engages the seal 2408 and, more particularly, the first seal element 24l0a. The mating member 2318 provided at the bottom of the sharp hub 2316 can extend out the second aperture 2402b in the bottom of the mount 2308, and the sensor cap 2320 can be coupled to the sharp hub 2316 at the mating member 2318. Coupling the sensor cap 2320 to the sharp hub 2316 at the mating member 2318 can urge the first end 2322a of the sensor cap 2320 into sealed engagement with the seal 2408 and, more particularly, into sealed engagement with the second seal element 2410b on the bottom of the mount 2308. In some embodiments, as the sensor cap 2320 is coupled to the sharp hub 2316, a portion of the first end 9l22a of the sensor cap 2320 can bottom out (engage) against the bottom of the mount 2308, and the sealed engagement between the sensor hub 2316 and the first seal element 24l0a can be able to assume any tolerance variation between features.

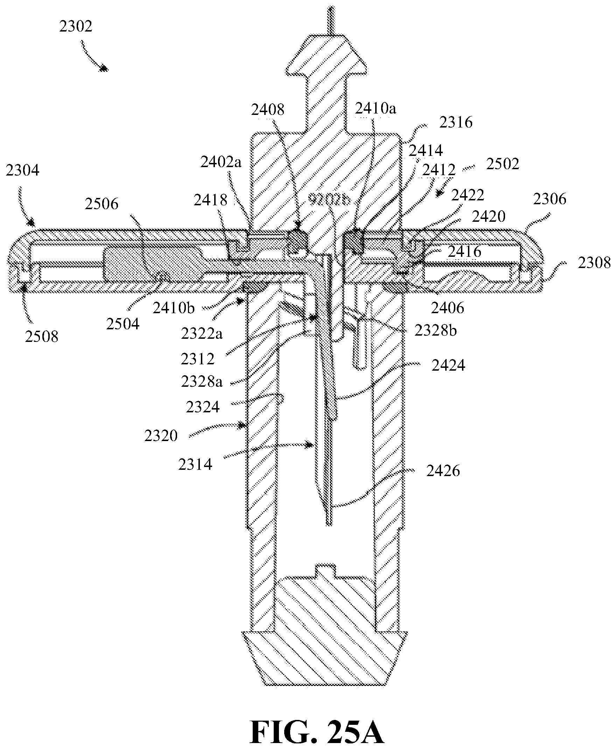

FIG. 25A illustrate a cross-sectional side view of the sensor control device 2302, according to certain embodiments. As indicated above, the sensor control device 2302 can include or otherwise incorporate a sealed subassembly 2502, which can be useful in isolating the sensor 2312 and the sharp 2314 within the inner chamber 2324 of the sensor cap 2320. To assemble the sealed subassembly 2502, the sensor 2312 can be located within the mount 2308 such that the tail 2424 extends through the second aperture 2402b at the bottom of the mount 2308. In at least one embodiment, a locating feature 2504 can be defined on the inner surface of the mount 2308, and the sensor 2312 can define a groove 2506 that is matable with the locating feature 2504 to properly locate the sensor 2312 within the mount 2308.

Once the sensor 2312 is properly located, the collar 2412 can be installed on the mount 2308. More specifically, the collar 2412 can be positioned such that the first seal element 24l0a of the seal 2408 is received within the central aperture 2414 defined by the collar 2412 and the first seal element 24l0a generates a radial seal against the collar 2412 at the central aperture 2414. Moreover, the annular lip 2416 defined on the collar 2412 can be received within the channel 2406 defined on the mount 2308, and the groove 2418 defined through the annular lip 2416 can be aligned to receive the portion of the sensor 2312 that traverses the channel 2406 laterally within the mount 2308. In some non-limiting embodiments, an adhesive can be injected into the channel 2406 to secure the collar 2412 to the mount 2308. The adhesive can also facilitate a sealed interface between the two components and generate a seal around the sensor 2312 at the groove 2418, which can isolate the tail 2424 from the interior of the electronics housing 2304.

The shell 2306 can then be mated with or otherwise coupled to the mount 2308. In some embodiments, as illustrated, the shell 2306 can mate with the mount 2308 via a tongue- and-groove engagement 2508 at the outer periphery of the electronics housing 2304. An adhesive can be injected (applied) into the groove portion of the engagement 2508 to secure the shell 2306 to the mount 2308, and also to create a sealed engagement interface. Mating the shell 2306 to the mount 2308 can also cause the annular ridge 2422 defined on the inner surface of the shell 2306 to be received within the collar channel 2420 defined on the upper surface of the collar 2412. In some embodiments, an adhesive can be injected into the collar channel 2420 to secure the shell 2306 to the collar 2412, and also to facilitate a sealed interface between the two components at that location. When the shell 2306 mates with the mount 2308, the first seal element 24l0a can extend at least partially through (into) the first aperture 2402a defined in the shell 2306.

The sharp 2314 can then be coupled to the sensor control device 2302 by extending the sharp tip 2426 through the aligned first and second apertures 2402a, b defined in the shell 2306 and the mount 2308, respectively. The sharp 2314 can be advanced until the sharp hub 2316 engages the seal 2408 and, more particularly, engages the first seal element 24l0a. The mating member 2318 can extend (protrude) out the second aperture 2402b at the bottom of the mount 2308 when the sharp hub 2316 engages the first seal element 24l0a.

The sensor cap 2320 can then be removably coupled to the sensor control device 2302 by threadably mating the internal threads 2328b of the sensor cap 2320 with the external threads 2328a of the mating member 2318. The inner chamber 2324 can be sized and otherwise configured to receive the tail 2424 and the sharp tip 2426 extending from the bottom of the mount 2308. Moreover, the inner chamber 2324 can be sealed to isolate the tail 2424 and the sharp tip 2426 from substances that might adversely interact with the chemistry of the tail 2424. In some embodiments, a desiccant (not shown) can be present within the inner chamber 2324 to maintain proper humidity levels.

Tightening (rotating) the mated engagement between the sensor cap 2320 and the mating member 2318 can urge the first end 2322a of the sensor cap 2320 into sealed engagement with the second seal element 2410b in an axial direction (e.g., along the centerline of the apertures 2402a, b), and can further enhance the sealed interface between the sharp hub 2316 and the first seal element 24l0a in the axial direction. Moreover, tightening the mated engagement between the sensor cap 2320 and the mating member 2318 can compress the first seal element 24l0a, which can result in an enhanced radial sealed engagement between the first seal element 24l0a and the collar 2412 at the central aperture 2414. Accordingly, in at least one embodiment, the first seal element 24l0a can help facilitate axial and radial sealed engagements.

As mentioned above, the first and second seal elements 24l0a,b can be overmolded onto the mount 2308 and can be physically linked or otherwise interconnected. Consequently, a single injection molding shot can flow through the second aperture 2402b of the mount 2308 to create both ends of the seal 2408. This can prove advantageous in being able to generate multiple sealed interfaces with only a single injection molded shot. An additional advantage of a two-shot molded design, as opposed to using separate elastomeric components (e.g., O-rings, gaskets, etc.), is that the interface between the first and second shots is a reliable bond rather than a mechanical seal. Hence, the effective number of mechanical sealing barriers is effectively cut in half. Moreover, a two-shot component with a single elastomeric shot also has implications to minimizing the number of two-shot components needed to achieve all the necessary sterile barriers.

Once properly assembled, the sealed subassembly 2502 can be subjected to a radiation sterilization process to sterilize the sensor 2312 and the sharp 2314. The sealed subassembly 2502 can be subjected to the radiation sterilization prior to or after coupling the sensor cap 2320 to the sharp hub 2316. When sterilized after coupling the sensor cap 2320 to the sharp hub 2316, the sensor cap 2320 can be made of a material that permits the propagation of radiation therethrough. In some embodiments, the sensor cap 2320 can be transparent or translucent, but can otherwise be opaque, without departing from the scope of the disclosure.

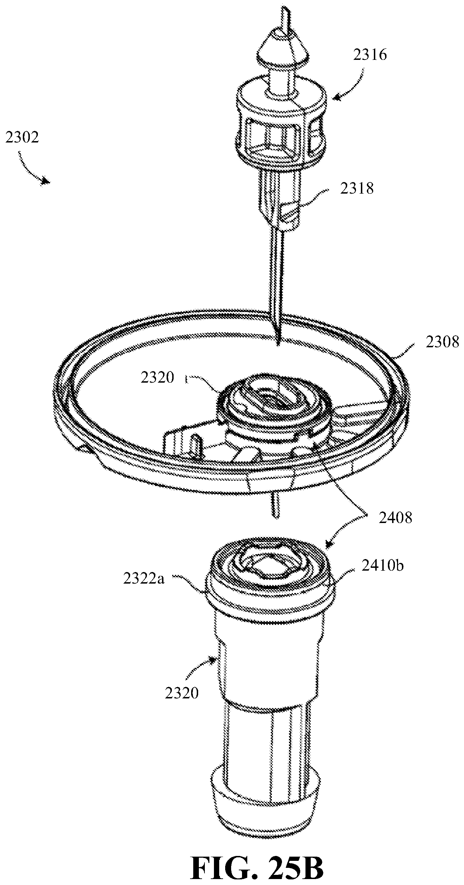

FIG. 25B illustrates an exploded isometric view of a portion of another embodiment of the sensor control device 2302 of FIGS. 23A-23B and 24A-24B. Embodiments included above describe the mount 2308 and the seal 2408 being manufactured via a two-shot injection molding process. In other embodiments, however, as briefly mentioned above, one or both of the seal elements 24l0a,b of the seal 2408 can comprise an elastomeric component part independent of the mount 2408. In the illustrated embodiment, for example, the first seal element 24l0a can be overmolded onto the collar 2412 and the second seal element 24l0b can be overmolded onto the sensor cap 2320. Alternatively, the first and second seal elements 24l0a,b can comprise a separate component part, such as a gasket or O-ring positioned on the collar 2412 and the sensor cap 2320, respectively. Tightening (rotating) the mated engagement between the sensor cap 2320 and the mating member 2318 can urge the second seal element 2410b into sealed engagement with the bottom of the mount 2308 in an axial direction, and can enhance a sealed interface between the sharp hub 2316 and the first seal element 24l0a in the axial direction.

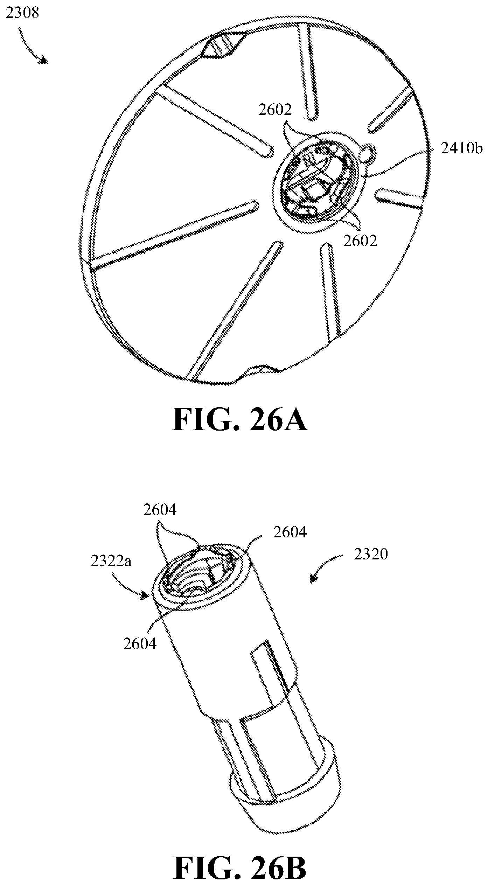

FIG. 26A illustrates an isometric bottom view of the mount 2308, and FIG. 26B illustrates an isometric top view of the sensor cap 2320 according to certain embodiments. As shown in FIG. 26A, mount 2308 can provide or otherwise define one or more indentations or pockets 2602 at or near the opening to the second aperture 2402b. As shown in FIG. 26B, the sensor cap 2320 can provide or otherwise define one or more projections 2604 at or near the first end 9l22a of the sensor cap 2320. The projections 2604 can be received within the pockets 2602 when the sensor cap 2320 is coupled to the sharp hub 2316. More specifically, as described above, as the sensor cap 2320 is coupled to the mating member 2318 of the sensor hub 2316, the first end 9l22a of the sensor cap 2320 is brought into sealed engagement with the second seal element 24l0b. In this process, the projections 2604 can also be received within the pockets 2602, which can help prevent premature unthreading of the sensor cap 2320 from the sharp hub 2316.

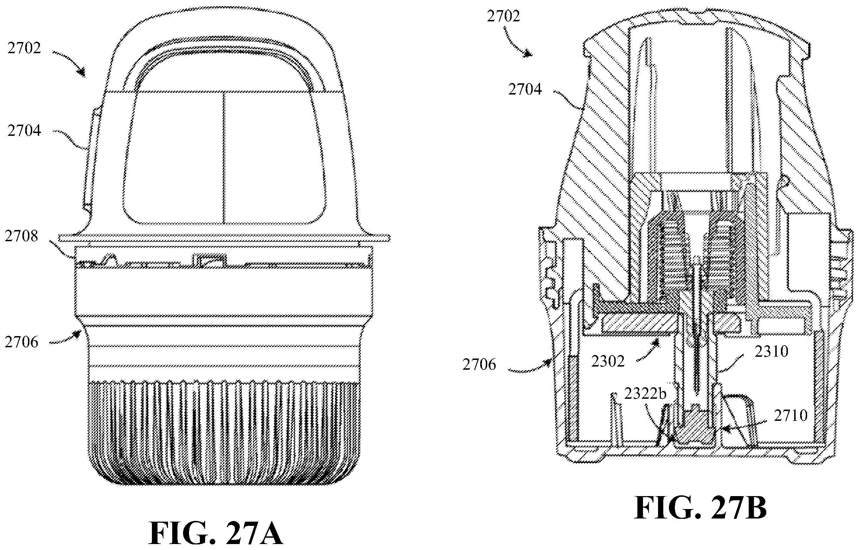

FIGS. 27A and 27B illustrate side and cross-sectional side views, respectively, of an example sensor applicator 2702 according to certain embodiments. The sensor applicator 2702 can be similar in some respects to the sensor applicator 102 of FIG. 1 and, therefore, can be designed to deliver (fire) a sensor control device, such as the sensor control device 2302. FIG. 27A depicts how the sensor applicator 2702 might be shipped to and received by a user, and FIG. 27B depicts the sensor control device 2302 arranged within the interior of the sensor applicator 2702.

As shown in FIG. 27A, the sensor applicator 2702 includes a housing 2704 and an applicator cap 2706 removably coupled to the housing 2704. In some embodiments, the applicator cap 2706 can be threaded to the housing 2704 and include a tamper ring 2708. Upon rotating (e.g., unscrewing) the applicator cap 2706 relative to the housing 2704, the tamper ring 2708 can shear and thereby free the applicator cap 2706 from the sensor applicator 2702.

In FIG. 27B, the sensor control device 2302 is positioned within the sensor applicator 2702. Once the sensor control device 2302 is fully assembled, it can then be loaded into the sensor applicator 2702 and the applicator cap 2706 can be coupled to the sensor applicator 2702. In some embodiments, the applicator cap 2706 and the housing 2704 can have opposing, matable sets of threads that enable the applicator cap 2706 to be screwed onto the housing 2704 in a clockwise (or counter-clockwise) direction and thereby secure the applicator cap 2706 to the sensor applicator 2702.

Securing the applicator cap 2706 to the housing 2704 can also cause the second end 9l22b of the sensor cap 2320 to be received within a cap post 2710 located within the interior of the applicator cap 2706 and extending proximally from the bottom thereof. The cap post 2710 can be configured to receive at least a portion of the sensor cap 2320 as the applicator cap 2706 is coupled to the housing 2704.

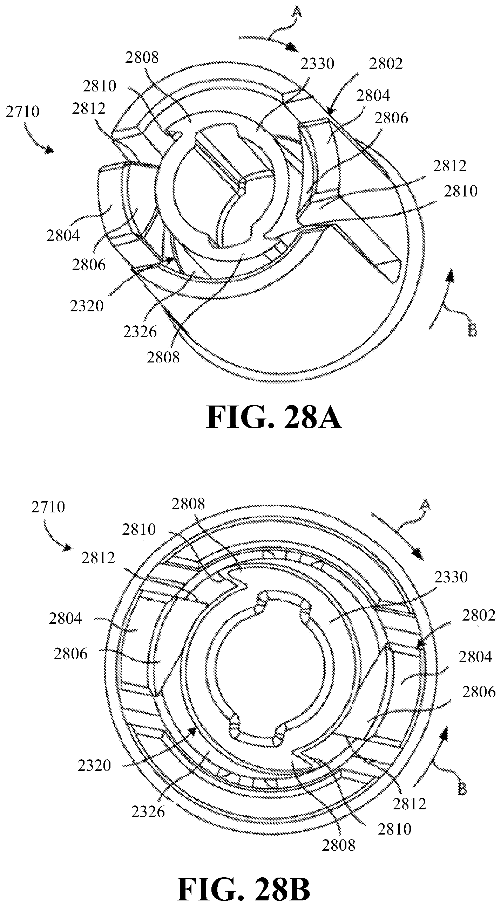

FIGS. 28A and 28B are perspective and top views, respectively, of the cap post 2710, according to one or more additional embodiments. In the illustrated depiction, a portion of the sensor cap 2320 is received within the cap post 2710 and, more specifically, the desiccant cap 2330 of the sensor cap 2320 is arranged within cap post 2710. The cap post 2710 can define a receiver feature 2802 configured to receive the engagement feature 2326 of the sensor cap 2320 upon coupling (e.g., threading) the applicator cap 2706 (FIG. 27B) to the sensor applicator 2702 (FIGS. 27A-27B). Upon removing the applicator cap 2706 from the sensor applicator 2702, however, the receiver feature 2802 can prevent the engagement feature 2326 from reversing direction and thus prevent the sensor cap 2320 from separating from the cap post 2710. Instead, removing the applicator cap 2706 from the sensor applicator 2702 will simultaneously detach the sensor cap 2320 from the sensor control device 2302 (FIGS. 23A-24B and 24A-24B), and thereby expose the distal portions of the sensor 2312 (FIGS. 24A-24B) and the sharp 2314 (FIGS. 24A-24B).

Many design variations of the receiver feature 2802 can be employed, without departing from the scope of the disclosure. In the illustrated embodiment, the receiver feature 2802 includes one or more compliant members 2804 (two shown) that are expandable or flexible to receive the engagement feature 2326. The engagement feature 2326 can comprise, for example, an enlarged head and the compliant member(s) 2804 can comprise a collet-type device that includes a plurality of compliant fingers configured to flex radially outward to receive the enlarged head.

The compliant member(s) 2804 can further provide or otherwise define corresponding ramped surfaces 2806 configured to interact with one or more opposing camming surfaces 2808 provided on the outer wall of the engagement feature 2326. The configuration and alignment of the ramped surface(s) 2806 and the opposing camming surface(s) 2808 is such that the applicator cap 2706 is able to rotate relative to the sensor cap 2320 in a first direction A (e.g., clockwise), but the cap post 2710 binds against the sensor cap 2320 when the applicator cap 2706 is rotated in a second direction B (e.g., counter clockwise). More particularly, as the applicator cap 2706 (and thus the cap post 2710) rotates in the first direction A, the camming surfaces 2808 engage the ramped surfaces 2806, which urge the compliant members 2804 to flex or otherwise deflect radially outward and results in a ratcheting effect. Rotating the applicator cap 2706 (and thus the cap post 2710) in the second direction B, however, will drive angled surfaces 2810 of the camming surfaces 2808 into opposing angled surfaces 2812 of the ramped surfaces 2806, which results in the sensor cap 2320 binding against the compliant member(s) 2804.

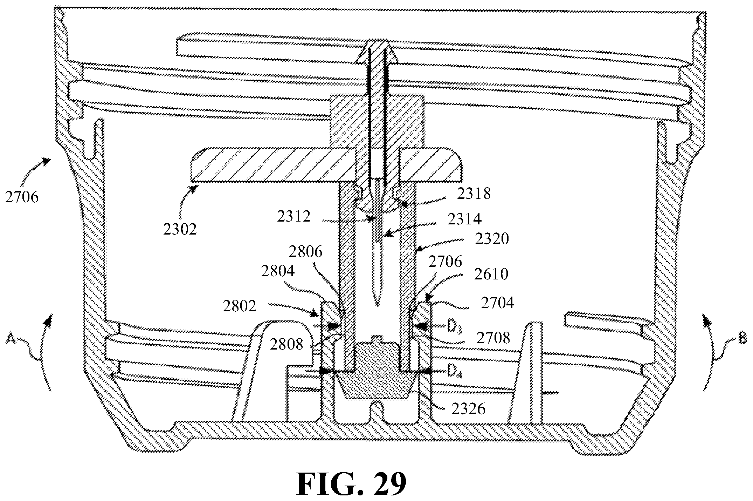

FIG. 29 is a cross-sectional side view of the sensor control device 2302 positioned within the applicator cap 2706, according to one or more embodiments. As illustrated, the opening to the receiver feature 2802 exhibits a first diameter D3, while the engagement feature 2326 of the sensor cap 2320 exhibits a second diameter D4 that is larger than the first diameter D3 and greater than the outer diameter of the remaining portions of the sensor cap 2320. As the sensor cap 2320 is extended into the cap post 2710, the compliant member(s) 2804 of the receiver feature 2802 can flex (expand) radially outward to receive the engagement feature 2326. In some embodiments, as illustrated, the engagement feature 2326 can provide or otherwise define an angled outer surface that helps bias the compliant member(s) 2804 radially outward. Once the engagement feature 2326 bypasses the receiver feature 2802, the compliant member(s) 2804 are able to flex back to (or towards) their natural state and thus lock the sensor cap 2320 within the cap post 2710.

As the applicator cap 2706 is threaded to (screwed onto) the housing 2704 (FIGS. 5A-35B) in the first direction A, the cap post 2710 correspondingly rotates in the same direction and the sensor cap 2320 is progressively introduced into the cap post 2710. As the cap post 2710 rotates, the ramped surfaces 2806 of the compliant members 2804 ratchet against the opposing camming surfaces 2808 of the sensor cap 2320. This continues until the applicator cap 2706 is fully threaded onto (screwed onto) the housing 2704. In some embodiments, the ratcheting action can occur over two full revolutions of the applicator cap 2706 before the applicator cap 2706 reaches its final position.

To remove the applicator cap 2706, the applicator cap 2706 is rotated in the second direction B, which correspondingly rotates the cap post 2710 in the same direction and causes the camming surfaces 2808 (i.e., the angled surfaces 2810 of FIGS. 28A-28B) to bind against the ramped surfaces 2806 (i.e., the angled surfaces 2812 of FIGS. 28A-28B). Consequently, continued rotation of the applicator cap 2706 in the second direction B causes the sensor cap 2320 to correspondingly rotate in the same direction and thereby unthread from the mating member 2318 to allow the sensor cap 2320 to detach from the sensor control device 2302. Detaching the sensor cap 2320 from the sensor control device 2302 exposes the distal portions of the sensor 2312 and the sharp 2314, and thus places the sensor control device 2302 in position for firing (use).

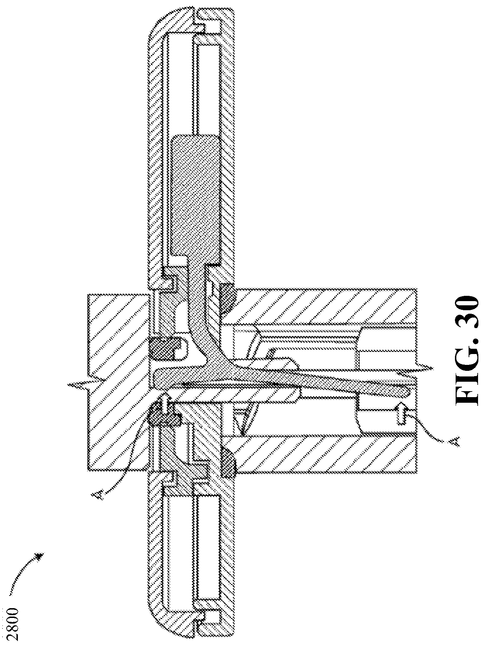

FIG. 30 is a cross-sectional view of a sensor control device 2800 showing example interaction between the sensor and the sharp. After assembly of the sharp, the sensor should sit in a channel defined by the sharp. In certain non-limiting embodiments, the sensor can deflect inwards and otherwise aligned fully with the sharp. In some other non-limiting embodiments, as shown in FIG. 30, the slight bias forces can be assumed by the sensor at the locations indicated by the two arrows A. Biasing the sensor against the sharp can be advantageous so that any relative motion between the sensor and the sharp during subcutaneous insertion does not expose the sensor tip (i.e., the tail) outside the sharp channel, which could potentially cause an insertion failure.

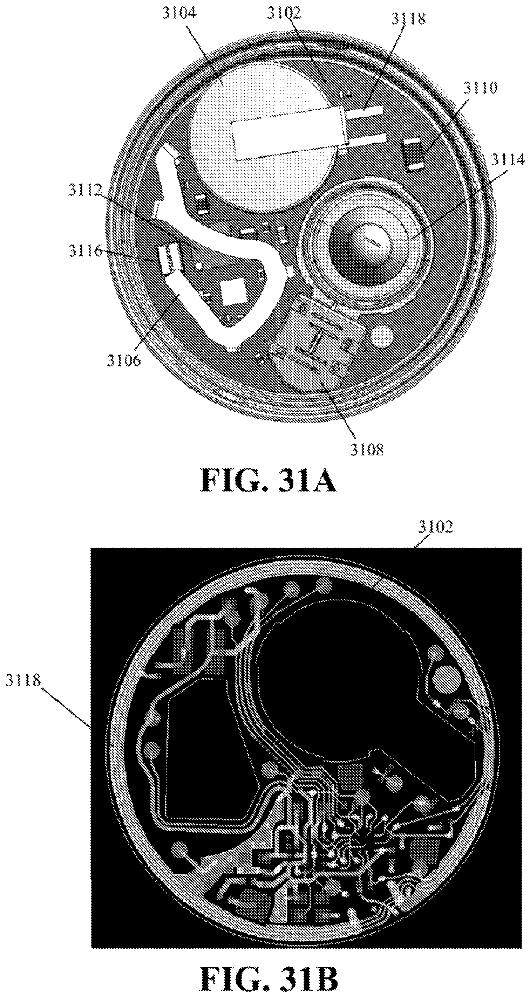

FIGS. 31A and 31B illustrate a printed circuit board according to certain embodiments. The printed circuit board (PCB) 3102 can be included in an apparatus, such as a sensor control device. The PCB, for example, can be made of FR4 or FR-4 composite material, which can include woven fiberglass cloth with an epoxy resin binder. In other non-limiting embodiments, the PCB can comprise any other material known in the art. In some embodiments, a button cell or cylinder cell battery 3104 can be attached to PCB 3102. For example, battery 3104 can be attached to PCB 3102 using spot soldering and/or battery tabs 3118. Using battery tabs can help to reduce battery size, while eliminating battery contact. Battery 3104 can be configured to power the PCB and/or one or more components attached to the PCB. PCB 3102 can also include one or more modules 3110, such as resistors, transistors, capacitors, inductors, diodes, and/or switches. The one or more modules 3110 can be attached, connected, or mounted to PCB 3102.

An analyte sensor, as shown in FIGS. 1-3B, 5B, 6A, 6B, 7, 9-11B, 13B, 15, 16B-17B, 18B, 19A, 19B, 21, 22A, 22B, 22D, 22E, 23A, 23B, 24A, 24B, 25A, 25B, 27B, 29, and/or 30 can be attached or connected to PCB 3102. A portion of the analyte sensor can be configured to be positioned in contact with fluid under a skin layer to monitor an analyte level in the fluid. In certain non-limiting embodiments, the sensor can include a tail, a flag, and a neck that interconnects the tail and the flag. An assembly 3108, for example a plug assembly as shown for example in FIGS. 3A, 3B, 22B, and 22E, can be included as part of the sensor assembly to receive and support both the sensor and a connector. When the assembly 3108 is properly coupled to the electronics housing, one or more circuitry contacts defined, for example, on the underside of PCB 3110 can make conductive communication with the electrical contacts of the connector. In some non-limiting embodiments, therefore, the connector can be connected to the PCB and can be configured to establish an electrical connection between an analyte sensor and the PCB.

While in certain embodiments the connector can take the form shown in FIGS. 3A, 3B, 11A, 11B, 17A, and/or 17B, in other embodiments the connector can be any other shape, such as collar shaped. At least part of the connector can comprise at least one of silicone rubber and/or carbon impregnated polymer. Although some the embodiments included herein describe the use of a plug to connect the connector to the PCB, in certain other embodiments the connector can be directly connected to the PCB. For example, assembly 3108 can comprise a Molex connector and a flag of the sensor, as shown in FIGS. 22B and 22E. In some embodiments, one or more parts of the sensor, such as the tail, flag, and neck, can be shaped to help secure and keep the sensor in the sharp channel. For example, the neck can include a biasing tower and/or the flag can include one or more apertures to lay on help secure or keep the sensor properly aligned within the sharp channel. In some non-limiting embodiments, a sharp hub 3114 can be used to help hold or secure the sensor to the PBC.

In some non-limiting embodiments, a processor 3112 can be connected to the PCB 3102. Processor 3112 can be embodied by any computational or data processing device, such as a general data processing unit, a central processing unit (CPU), a digital signal processor (DSP), an application specific integrated circuit (ASIC), programmable logic devices (PLDs), field programmable gate arrays (FPGAs), input/output (I/O) circuitry, digitally enhanced circuits, or comparable device, or any combination thereof. PCB 3102 can include a single processor or controller, or a plurality of controllers or processors. For example, PCB 3102 can include a general data processing unit 3112 or an ASIC 3116, or both a general data processing unit 3112 and an ASIC 3116. In certain non-limiting embodiments, a processor, whether general data processing unit 3112 or ASIC 3116, can be configured to process data associated with the monitored analyte level.

In certain non-limiting embodiments, one or more antennas can be attached to the PCB. The one or more antennas, for examples, can be a Bluetooth low energy antenna, an NFC antenna, or any other antenna used for wireless communication that can be used to transmit the monitored analyte level. The monitored analyte level, for example, can be glucose level, ketone level, lactate level, oxygen level, hemoglobin AIC level, or the like, can be vitally important to the health of an individual having diabetes In addition to, or alternatively, the one or more antennas can be used to transmit any other information obtained by the sensor or stored at the sensor control device. The monitored analyte level or other information can be transmitted from the sensor control device to a reader device, such as reader device 106 shown in FIG. 1. The reader device, for example, can be any user equipment or mobile device used by an individual or a health care provider.

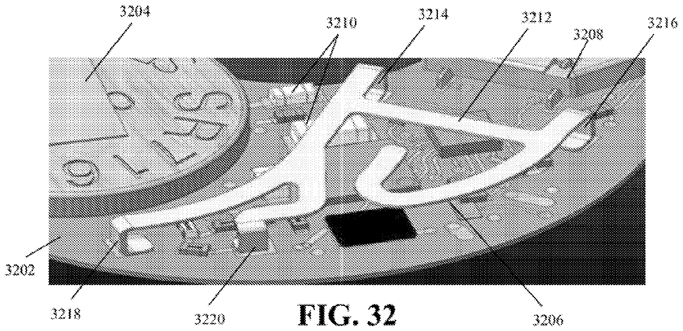

The antenna, for example, can be a Bluetooth low energy antenna. The antenna can be configured as an inverted h-shape, a j-shape, an inverted f-shape, or can take any other form. For example, antenna 3106 in FIG. 31A is j-shaped, while antenna 3218 in FIG. 32 is h-shaped. Antenna 3106 can be shaped so as to curve around an outer circumference of battery 3104. In other non-limiting embodiments, instead of being curved around the outer circumference of battery 3104, antenna 3106 can simply be located at a different location on the PCB so as to not overlap with battery 3104.

As shown in FIG. 31A, antenna 3106 can rest on a plurality of risers extending from a surface of the PCB by a fixed distance. The risers can help to raise the antenna above the PCB and/or one or more other components attached to the PCB. In some embodiments, raising the antenna above the PCB and/or one or more other components attached to the PCB can help to reduce interference and/or improve the quality of the signal transmitted or received from antenna 3106. The plurality of risers can range from 2 to 10 risers, from 1 to 15 risers, or any other number of risers. In other embodiments only a single riser can be provided. The fixed distance of the plurality of risers extending from the surface of the PCB can be greater than 1.5 millimeters. In particular, the fixed distance of the plurality of risers can be between 0.1 mm - 5 mm, 1.2 mm - 1.8 mm, or 1.525 - 1.675 millimeters. One or more of the plurality of risers can be configured to electrically connect the antenna to the PCB, while another of the one or more of the plurality of rises can simply be configured to structurally support the antenna. In some non-limiting embodiments, the one or more risers can be a portion of the antenna that is folded over to extend from the surface of the PCB by a fixed distance. As such, one or more of the plurality of risers can include a folded portion of the antenna. In other non-limiting embodiments, the one or more risers can be a separate component on which the antenna rests or to which the antenna is connected.

In certain non-limiting embodiments, antenna 3106 can be a dual Bluetooth low energy/NFC antenna. In other embodiments, however, two different antennas can be provided, antenna 3106 for Bluetooth low energy communications and a separate antenna for NFC communications. As shown in FIGS. 31A and 31B, either antenna 3106 or the separate NFC antenna 3118 can be used to transmit the monitored analyte levels. The separate NFC antenna 3118 can be provided as a module attached to the PCB. In some non-limiting embodiments, as shown in FIG. 31B, an NFC antenna 3118 can be embedded within and/or around a circumference of the PCB. For example, NFC antenna 3118 can be embedded in the material, e.g., FR4 of PCB 3102, as shown in FIG. 31B. In another embodiment, NFC antenna 3118 can be embedded in a lobe during fabrication of PCB 3102.

FIG. 32 illustrates a printed circuit board according to certain embodiments. The PCB 3102 can be included in an apparatus, such as a sensor control device, and can include battery 3204, antenna 3206, assembly 3108, and modules 3210. Antenna 3206 can be a Bluetooth low energy antenna. As shown in FIG. 32, antenna 3206 can be h-shaped and/or can rest on a plurality of risers. For example, antenna 3206 can rest on four risers 3214, 3216, 3218, and 3220. In some embodiments, two of the four risers configured to electrically connect the antenna to the PCB and the other two risers are for support. In other embodiments, all four risers can be configured to electrically connect the antenna to the PCB. A first set of the plurality of risers (e.g., two of the four risers 3214, 3216) can be located proximate to the connector, while a second set of the plurality of risers (e.g., two of the four risers 3218, 3220) can be located proximate to the battery. Antenna 3206, for example, can have a cross-bar 3212 located between the first and second sets of risers. In some embodiments, one or more of the plurality of risers are at least in part pre-plated tin over nickel. In other embodiments, the plurality of risers can be composed of any other one or more materials known in the art.

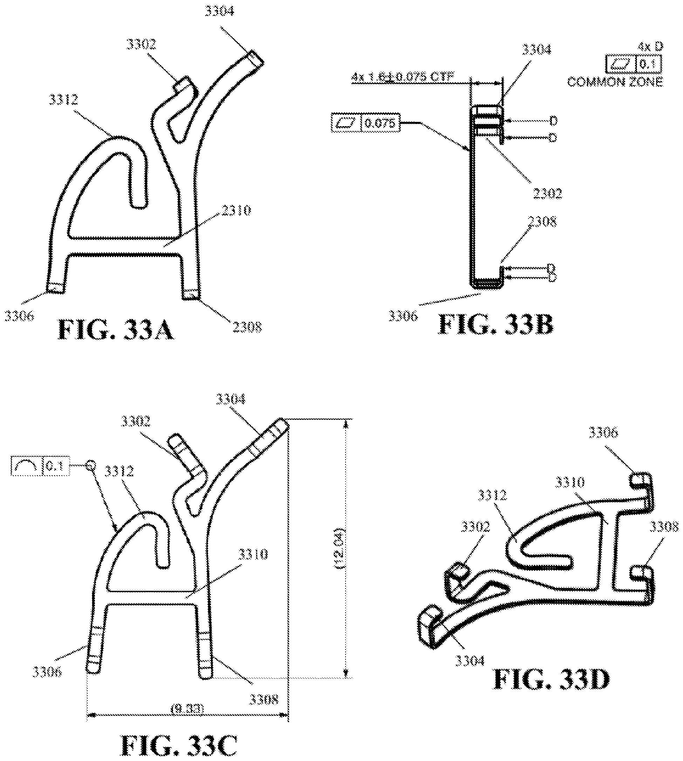

FIGS. 33A-33D illustrate an embodiment of an antenna according to certain embodiments. In particular, FIG. 33A illustrates a front view of h-shaped antenna 3206. As shown in FIG. 33A, antenna 3206 includes five ends, four of which are risers 3302, 3304, 3306, and 3308. Risers 3306 and 3308 are located at the base of the h-shaped antenna 3306 and are separated from one another by a distance about the length of cross-bar 3310. In addition to the four risers, antenna 3206 includes portion with a free, e.g., rounded, end 3312 that is not directly connected to the PCB surface. In certain non-limiting embodiments, therefore, the antenna can include a free end 3312 that extends from the surface of the printed circuit board by the fixed distance. The free end can form a hook, as shown in Fig. 33A with the edge of free end 3312 facing cross-bar 3310, but in other non-limiting embodiments the edge of rounded end 3312 can be facing away from cross-bar 3310. Risers 3302 and 3304 are located closer to one another than risers 3306 and 3308. As shown in FIG. 33A, the portion of the antenna that includes risers 3302 and 3304 can be branched or y-shaped, with the antenna portion leading to 3304 having a curved shape. As such, the antenna can include two or more ends forming a y-shape. The antenna portion leading to 3302, on the other hand, has two straight line segments that intersect approximately at a right angle. For example, the two straight line segments intersect at between 75 to 100 degree angles. In other embodiments, the two straight line segments can intersect at between 45 to 130, between 55 to 120, or between 65 to 110 degree angles .

FIG. 33B illustrates a side view of h-shaped antenna 3206, including risers 3302, 3304, 3306, and 3308. The risers have a length or fixed distance ranging between 1.525 - 1.675 millimeters. The bottom plane of one or more of the risers can be attached or mounted to the PCB. For example, the bottom plane, which can be rectangular in shape, can be soldered or welded to the PCB.

FIG. 33C illustrates a front view of h-shaped antenna 3206, with the risers having been unfolded. The antenna can have an unfolded width of about 9.33 millimeters, as shown in FIG. 33C. In other non-limiting embodiments, the unfolded width can range from about 1 to 20 mm, 5 to 15 mm, or 7.5 to 12.5 mm. The antenna can also have an unfolded length of about 12.04 millimeters. In other non-limiting embodiments, the unfolded length can range from about 1 to 22 mm, 7 to 17 mm, or 10 to 14 mm. An unfolded width or length, for example, can be the width or length of the antenna in which the folded risers, which can be a part of the antenna, are unfolded or straightened, as shown in FIG. 33C. In certain non-limiting embodiments, the antenna can have a mass of .024 grams. In other non-limiting embodiments, the antenna can have a mass ranging between .005 to 1.0 grams, .01 to .04 grams, or .02 to .03 grams. FIG. 33D illustrates an isometric view of h-shaped antenna 3206, with risers 3302, 3304, 3306, 3308, and cross-bar 3310.

Additional details of suitable devices, systems, methods, components and the operation thereof along with related features are set forth in International Publication No. WO2018/136898 to Rao et. al., International Publication No. WO2019/236850 to Thomas et. al., International Publication No. WO2019/236859 to Thomas et. al., International Publication No. WO2019/236876 to Thomas et. al., and U.S. Patent Publication No. 2020/0196919, filed June 6, 2019. Further details regarding embodiments of applicators, their components, and variants thereof, are described in U.S. Patent Publication Nos. 2013/0150691, 2016/0331283, and 2018/0235520. Further details regarding embodiments of sharp modules, sharps, their components, and variants thereof, are described in U.S. Patent Publication No. 2014/0171771.

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise.

Thus, the foregoing description of specific embodiments of the disclosed subject matter has been presented for purposes of illustration and description. It is explicitly acknowledged that express recitation of every possible combination and substitution is overly burdensome, especially given that the permissibility of each and every such combination and substitution will be readily recognized by those of ordinary skill in the art.

While the embodiments are susceptible to various modifications and alternative forms, specific examples thereof have been shown in the drawings and are herein described in detail. It will be apparent to those skilled in the art that various modifications and variations can be made in the method and system of the disclosed subject matter without departing from the scope of the disclosed subject matter which is solely defined by the appended claims. Thus, it is intended that the disclosed subject matter include modifications and variations that are within the scope of the appended claims.

## Claims

1. An apparatus for use with an analyte sensor, the apparatus comprising:
a printed circuit board (3102; 3202) configured to monitor an analyte level;
a battery (3104; 3204) connected to the printed circuit board and configured to power the printed circuit board;
a connector connected to the printed circuit board and configured to establish an electrical connection between the analyte sensor and the printed circuit board;
a processor (3112) connected to the printed circuit board and configured to process data associated with the monitored analyte level;
a plurality of risers (3214, 3216, 3218, 3220; 3302, 3304, 3306, 3308); and
**characterized by** an antenna (3206; 3306) for transmitting the monitored analyte level resting on the plurality of risers, wherein the risers extend from a surface of the printed circuit board by a fixed distance, and wherein the antenna comprises a cross bar (3212; 3310) located between a first set of the plurality of risers and a second set of the plurality of risers.

2. The apparatus of claim 1, wherein the antenna is a Bluetooth low energy antenna.

3. The apparatus of any of claims 1 to 2, wherein the plurality of risers comprises four risers, and wherein two of the four risers are configured to electrically connect the antenna to the printed circuit board.

4. The apparatus of any of claims 1 to 3, wherein the antenna is curved around an outer circumference of the battery.

5. The apparatus of any of claims 1 to 4, wherein the antenna is configured as an inverted h-shape or a j-shape.

6. The apparatus of any of claims 1 to 5, wherein the antenna comprises two or more ends forming a y-shape.

7. The apparatus of any of claims 1 to 6, wherein the antenna includes a free end (3312) that extends from the surface of the printed circuit board by the fixed distance.

8. The apparatus of any of claims 1 to 7, wherein one or more of the plurality of risers comprise a folded portion of the antenna.

9. The apparatus of any of claims 1 to 8, wherein a first set of the plurality of risers is located proximate to the connector, and wherein a second set of the plurality of risers is located proximate to the battery.

10. The apparatus of claim 9, wherein the second set or the first set of the plurality of risers is configured to electrically connect the antenna to the printed circuit board.

11. The apparatus of any of claims 1 to 10, wherein at least part of the plurality of risers are pre-plated tin over nickel.

12. The apparatus of any of claims 1 to 11, further comprising a near field communication antenna embedded within and around a circumference of the printed circuit board.

13. The apparatus of any of claims 1 to 12, wherein the fixed distance is greater than 1.5 millimeters.

14. The apparatus of any of claims 1 to 13, wherein the antenna has an unfolded width of about 9.33 millimeters and/or an unfolded length of about 12.04 millimeters.

15. The apparatus of any of claims 1 to 14, wherein the antenna has a mass of 0.024 grams.

16. The apparatus of any of claims 1 to 15, wherein the printed circuit board comprises FR4 material.

17. The apparatus of any of claims 1 to 16, wherein the analyte level comprises a glucose level and a portion of the analyte sensor is configured to be positioned in contact with fluid under a skin layer to monitor the analyte level in the fluid.

## Patentansprüche

1. Vorrichtung zur Verwendung mit einem Analytsensor, wobei die Vorrichtung umfasst:
eine Leiterplatte (3102; 3202), die dazu konfiguriert ist, eine Analytkonzentration zu überwachen;
eine Batterie (3104; 3204), die mit der Leiterplatte verbunden ist und dazu konfiguriert ist, die Leiterplatte mit Strom zu versorgen;
einen Verbinder, der mit der Leiterplatte verbunden ist und dazu konfiguriert ist, eine elektrische Verbindung zwischen dem Analytsensor und der Leiterplatte herzustellen;
einen Prozessor (3112), der mit der Leiterplatte verbunden ist und dazu konfiguriert ist, Daten zu verarbeiten, die mit der überwachten Analytkonzentration assoziiert sind;
eine Vielzahl von Risern (3214, 3216, 3218, 3220; 3302, 3304, 3306, 3308); und
**gekennzeichnet durch** eine Antenne (3206; 3306) zum Übertragen der überwachten Analytkonzentration, die auf der Vielzahl von Risern aufliegt, wobei sich die Riser um einen festgelegten Abstand von einer Oberfläche der Leiterplatte erstrecken, und wobei die Antenne eine Querschiene (3212; 3310) umfasst, die sich zwischen einem ersten Satz der Vielzahl von Risern und einem zweiten Satz der Vielzahl von Risern befindet.

2. Vorrichtung nach Anspruch 1, wobei die Antenne eine Bluetooth-Low-Energy-Antenne ist.

3. Vorrichtung nach einem der Ansprüche 1 bis 2, wobei die Vielzahl von Risern vier Riser umfasst, und wobei zwei der Riser dazu konfiguriert sind, die Antenne elektrisch mit der Leiterplatte zu verbinden.

4. Vorrichtung nach einem der Ansprüche 1 bis 3, wobei die Antenne um einen Außenumfang der Batterie gebogen ist.

5. Vorrichtung nach einem der Ansprüche 1 bis 4, wobei die Antenne als eine umgedrehte H-Form oder eine J-Form konfiguriert ist.

6. Vorrichtung nach einem der Ansprüche 1 bis 5, wobei die Antenne zwei oder mehr Enden umfasst, die eine Y-Form bilden.

7. Vorrichtung nach einem der Ansprüche 1 bis 6, wobei die Antenne ein freies Ende (3312) einschließt, das sich um einen festgelegten Abstand von der Oberfläche der Leiterplatte erstreckt.

8. Vorrichtung nach einem der Ansprüche 1 bis 7, wobei ein oder mehrere der Vielzahl von Risern einen gefalteten Abschnitt der Antenne umfassen.

9. Vorrichtung nach einem der Ansprüche 1 bis 8, wobei sich ein erster Satz der Vielzahl von Risern nahe dem Verbinder befindet, und wobei sich ein zweiter Satz der Vielzahl von Risern nahe der Batterie befindet.

10. Vorrichtung nach Anspruch 9, wobei der zweite Satz oder der erste Satz der Vielzahl von Risern dazu konfiguriert ist, die Antenne elektrisch mit der Leiterplatte zu verbinden.

11. Vorrichtung nach einem der Ansprüche 1 bis 10, wobei mindestens ein Teil der Vielzahl von Risern vorplattiertes Zinn-über-Nickel ist.

12. Vorrichtung nach einem der Ansprüche 1 bis 11, ferner umfassend eine Nahfeldkommunikationsantenne, die innerhalb eines oder um einen Umfang der Leiterplatte eingebettet ist.

13. Vorrichtung nach einem der Ansprüche 1 bis 12, wobei der festgelegte Abstand größer als 1,5 Millimeter ist.

14. Vorrichtung nach einem der Ansprüche 1 bis 13, wobei die Antenne eine entfaltete Breite von etwa 9,33 Millimeter und/oder eine entfaltete Länge von etwa 12,04 Millimeter aufweist.

15. Vorrichtung nach einem der Ansprüche 1 bis 14, wobei die Antenne ein Gewicht von 0,024 Gramm aufweist.

16. Vorrichtung nach einem der Ansprüche 1 bis 15, wobei die Leiterplatte FR4-Material umfasst.

17. Vorrichtung nach einem der Ansprüche 1 bis 16, wobei die Analytkonzentration eine Glukosekonzentration umfasst und ein Abschnitt des Analytsensors dazu konfiguriert ist, in Kontakt mit einem Fluid unter einer Hautschicht positioniert zu werden, um die Analytkonzentration in dem Fluid zu überwachen.

## Revendications

1. Appareil destiné à être utilisé avec un capteur d'analyte, l'appareil comprenant :
une carte de circuit imprimé (3102 ; 3202) configurée pour surveiller un niveau d'analyte ;
une batterie (3104 ; 3204) connectée à la carte de circuit imprimé et configurée pour alimenter la carte de circuit imprimé ;
un connecteur connecté à la carte de circuit imprimé et configuré pour établir une connexion électrique entre le capteur d'analyte et la carte de circuit imprimé ;
un processeur (3112) connecté à la carte de circuit imprimé et configuré pour traiter des données associées au niveau d'analyte surveillé ;
une pluralité de montants (3214, 3216, 3218, 3220 ; 3302, 3304, 3306, 3308) ; et
**caractérisé par** une antenne (3206 ; 3306) destinée à transmettre le niveau d'analyte surveillé reposant sur la pluralité de montants, dans lequel les montants s'étendent à partir d'une surface de la carte de circuit imprimé sur une distance fixe, et dans lequel l'antenne comprend une barre transversale (3212 ; 3310) située entre un premier ensemble de la pluralité de montants et un second ensemble de la pluralité de montants.

2. Appareil selon la revendication 1, dans lequel l'antenne est une antenne Bluetooth de faible énergie.

3. Appareil selon l'une quelconque des revendications 1 à 2, dans lequel la pluralité des montants comprend quatre montants, et dans lequel deux des quatre montants sont configurés pour connecter électriquement l'antenne à la carte de circuit imprimé.

4. Appareil selon l'une quelconque des revendications 1 à 3, dans lequel l'antenne est courbée autour d'une circonférence extérieure de la batterie.

5. Appareil selon l'une quelconque des revendications 1 à 4, dans lequel l'antenne est configurée en forme de h inversé ou en forme de j.

6. Appareil selon l'une quelconque des revendications 1 à 5, dans lequel l'antenne comprend deux extrémités ou plus formant une forme de y.

7. Appareil selon l'une quelconque des revendications 1 à 6, dans lequel l'antenne comporte une extrémité libre (3312) qui s'étend à partir de la surface de la carte de circuit imprimé sur la distance fixe.

8. Appareil selon l'une quelconque des revendications 1 à 7, dans lequel un ou plusieurs de la pluralité de montants comprennent une partie pliée de l'antenne.

9. Appareil selon l'une quelconque des revendications 1 à 8, dans lequel un premier ensemble de la pluralité de montants est situé à proximité du connecteur, et dans lequel un second ensemble de la pluralité de montants est situé à proximité de la batterie.

10. Appareil selon la revendication 9, dans lequel le second ensemble ou le premier ensemble de la pluralité de montants est configuré pour connecter électriquement l'antenne à la carte de circuit imprimé.

11. Appareil selon l'une quelconque des revendications 1 à 10, dans lequel au moins une partie de la pluralité des montants est de l'étain sur nickel pré-plaqué.

12. Appareil selon l'une quelconque des revendications 1 à 11, comprenant en outre une antenne de communication en champ proche noyée à l'intérieur et autour d'une circonférence de la carte de circuit imprimé.

13. Appareil selon l'une quelconque des revendications 1 à 12, dans lequel la distance fixe est supérieure à 1,5 millimètre.

14. Appareil selon l'une quelconque des revendications 1 à 13, dans lequel l'antenne a une largeur dépliée d'environ 9,33 millimètres et/ou une longueur dépliée d'environ 12,04 millimètres.

15. Appareil selon l'une quelconque des revendications 1 à 14, dans lequel l'antenne a une masse de 0,024 gramme.

16. Appareil selon l'une quelconque des revendications 1 à 15, dans lequel la carte de circuit imprimé comprend un matériau FR4.

17. Appareil selon l'une quelconque des revendications 1 à 16, dans lequel le niveau d'analyte comprend un niveau de glucose et une partie du capteur d'analyte est configurée pour être positionnée en contact avec un fluide sous une couche de peau afin de surveiller le niveau d'analyte dans le fluide.
